# EUROPEAN PATENT SPECIFICATION

(11) **EP 2 694 972 B1**
(45) Date of publication and mention of the grant of the patent: **21.06.2017**
(21) Application number: 12716858.1
(22) Date of filing: 04.04.2012
(51) Int. Cl.: G01N 33/574

(54) **METHODS FOR PREDICTING AND IMPROVING THE SURVIVAL OF GASTRIC CANCER PATIENTS**
VERFAHREN ZUR VORHERSAGE UND VERBESSERUNG DER ÜBERLEBENSZEIT VON MAGENKREBSPATIENTEN
PROCÉDÉS POUR PRÉDIRE ET AMÉLIORER LA SURVIE DE PATIENTS ATTEINTS DE CANCER GASTRIQUE

(30) Priority: 04.04.2011 US 201161471678 P; 25.01.2012 US 201261590787 P
(43) Date of publication of application: 12.02.2014
(73) Proprietor: DiaTech Holdings, Inc., Franklin, TN 37067 (US)
(72) Inventor: KIM, Phillip, Irvine, CA 92612 (US); SINGH, Sharat, Rancho Santa Fe, CA 92127 (US)
(74) Representative: Campbell, Patrick John Henry
(86) International application number: PCT/US2012/032206
(87) International publication number: WO 2012/138785

(56) References cited:
- WO-A1-2011/008990
- BACHLEITNER-HOFMANN T. ET AL.: "Stimulation of epidermal growth factor (EGFR) or HER-3 mediates resistance to MET tyrosine kinase inhibition in MET-amplified gastric cancer cells", PROC. AM. ASS. CANCER RES. ANN. MEETING, vol. 49, April 2008 (2008-04), page 1186, XP002678285, & 99TH ANNUAL MEETING OF THE AMERICAN-ASSOCIATION-FOR-CANCER-RESEARCH; SAN DIEGO, CA, USA; APRIL 12 -16, 2008
- BACHLEITNER-HOFMANN T. ET AL.: "Antitumor activity of SNX-2112, a synthetic heat shock protein-90 inhibitor, in MET-amplified tumor cells with or without resistance to selective MET inhibition", CLIN. CANCER RES., vol. 17, no. 1, 1 January 2011 (2011-01-01), pages 122-133, XP55030286,
- MUKOHARA T. ET AL.: "Foretinib (GSK1363089) inhibits growth of gastric cancer cell lines through blockade of inter-receptor tyrosine kinases networks", EUR. J. CANCER, vol. 8, no. 7, #109, November 2010 (2010-11), page 42, XP027497797,
- TABERNERO J. ET AL.: "Integration of targeted therapies", EUR. J. CANCER, vol. 7, no. 2, #305, September 2009 (2009-09), page 74, XP026689064,
- BACHLEITNER-HOFMANN T. ET AL.: "HER kinase activation confers resistance to MET tyrosine kinase inhibition in MET oncogene-addicted gastric cancer cells", MOL. CANCER THER., vol. 7, no. 11, November 2008 (2008-11), pages 3499-3508, XP55020686,
- CORSO S. ET AL.: "Activation of HER family members in gastric carcinoma cells mediates resistance to MET inhibition", MOL. CANCER, vol. 9, no. 1, 26 May 2010 (2010-05-26), page 121, XP021077869,
- PENTHEROUDAKIS G. ET AL.: "Incorporation of Targeted Agents in the Management of Patients with Advanced Gastric Cancer", CURR. MED. CHEM., vol. 18, no. 11, 1 April 2011 (2011-04-01) , pages 1629-1639, XP002678286,
- KIM P. ET AL.: "Highly sensitive proximity mediated immunoassay reveals HER2 status conversion in the circulating tumor cells of metastatic breast cancer patients", PROTEOME SCIENCE, vol. 9, no. 1, 15 December 2011 (2011-12-15), page 75, XP55030160,

## Description

### CROSS-REFERENCES TO RELATED APPLICATIONS

### BACKGROUND OF THE INVENTION

A wide variety of human malignancies exhibit sustained c-Met stimulation, overexpression, or mutation, including carcinomas of the breast, liver, lung, ovary, kidney, stomach (gastric) and thyroid. Notably, activating mutations in c-Met have been positively identified in patients with a particular hereditary form of papillary renal cancer, directly implicating c-Met in human tumorigenesis. Aberrant signaling of the c-Met signaling pathway due to dysregulation of the c-Met receptor or overexpression of its ligand, hepatocyte growth factor (HGF), has been associated with an aggressive phenotype. Extensive evidence that c-Met signaling is involved in the progression and spread of several cancers and an enhanced understanding of its role in disease have generated considerable interest in c-Met and HGF as major targets in cancer drug development.

This interest has led to the development of a variety of c-Met pathway antagonists with potential clinical applications. The three main approaches of pathway-selective anticancer drug development have included antagonism of ligand/receptor interaction, inhibition of the tyrosine kinase catalytic activity, and blockade of the receptor/effector interaction.

Bachleitner-Hofmann et al.: Proc. Am.Cancer Res. Ann. Meeting, vol. 49, April 2008, page 1186 describes that stimulation of EGFR or HER-3 with EGF or HRG is a mechanism by which MET-amplified tumor cells can be rescued from the growth-inhibitory effects of MET inhibition. Combined inhibition of MET, EGFR and HER-3 signaling prevents such tumor cell rescue, making it a promising approach for MET-amplified tumors coexpressing EGFR and/or HER-3.

Bachleitner-Hofmann et al.:Clin Cancer Res; 17(1); 122-33 provides evidence for the efficacy of HSP-90 inhibition in *MET*-amplified cancer cells, particularly when MET kinase inhibitor resistance has emerged. MUKOHARA T. ET AL.: EUR. J. CANCER, vol. 8, no. 7, #109, November 2010 (2010-11-01), pages 42, XP027497797 discloses that Foretinib (GSK1363089) inhibits growth of gastric cancer cell lines through blockade of inter-receptor tyrosine kinases networks.
TABERNERO J. ET AL.: EUR. J. CANCER, vol. 7, no. 2, #305, September 2009 (2009-09-01), pages 74, XP026689064 discloses Integration of targeted therapies and determination of overall survival of patients with Her2-positive gastric cancer following combination therapy.

c-Met is the cell surface receptor for hepatocyte growth factor (HGF), also known as scatter factor. HGF is a multidomain glycoprotein that is highly related to members of the plasminogen serine protease family. It is secreted as a single-chain, inactive polypeptide by mesenchymal cells and is cleaved to its active heterodimer by a number of proteases.

HGF binding induces c-Met receptor homodimerization and phosphorylation of two tyrosine residues (Y1234 and Y1235) within the catalytic site, regulating kinase activity. The carboxy-terminal tail includes tyrosines Y1349 and Y1356, which, when phosphorylated, serve as docking sites for intracellular adaptor proteins, leading to downstream signaling. The c-Met receptor is expressed in the epithelial cells of many organs during embryogenesis and in adulthood, including the liver, pancreas, prostate, kidney, muscle, and bone marrow.

One c-Met mediated cancer of interest is gastric cancer. Gastric cancer is the leading cause of cancer death worldwide with the incidence of 18.9/100,000 per year. The incidence of gastric cancer was estimated to be 934,000 cases, with 56% of the new cases occurring in East Asia. Gastric cancer accounts for 20.8% of all cancers in Korea according to the Central Tumor Registry data for 2002. Although gastrectomy is the only curative treatment in gastric cancer patients, a high recurrence rate ranging from 40-60% following curative surgery still accounts for poor overall survival.

Preliminary clinical results of several c-Met antagonists are now under clinical investigation. These agents, including monoclonal antibodies, disruptors of ligand/receptor interactions, and small-molecule tyrosine kinase inhibitors, have been encouraging. Several multi-targeted therapies have also been under investigation in the clinic and have demonstrated promise, particularly with regard to tyrosine kinase inhibition. In view of the role c-Met plays in a variety of cancers, there is need for the proper selection and strategies of therapies for an individual. This is especially true for c-Met mediated gastric cancers. The present invention satisfies these and other needs.

### BRIEF SUMMARY OF THE INVENTION

The present invention provides assays and methods for predicting the survival of a subject having an early stage (*e.g.*, stage I or II or transition in-between stages) gastric cancer after tumor surgery. In certain aspects, the present invention provides assays and methods for predicting therapeutic efficacy and/or response to combination therapy in a subject having an early stage gastric cancer.

In one aspect, the present invention relates to an in vitro method for predicting the survival of a subject having stage I gastric cancer after tumor surgery, the method comprising:
(a) determining the presence of activation or the level of activation of at least two analytes comprising cMET and HER1 in a cancer cell obtained from the subject, wherein cMET and HER1 are coactivated in the cancer cell; and
(b) predicting the survival of the subject based upon the presence of activation or the level of activation of the at least two analytes determined in step (a), wherein the subject is predicted to have worse survival compared to stage I gastric cancer subjects with cMET activation in the absence of HER1 activation.
In particular embodiments, the methods of the present invention rely on the detection of the activation state or level of a specific combination of signal transducer analytes in a cancer cell obtained from a subject having an early stage gastric cancer. Thus, the methods described herein are particularly useful for predicting the survival or prognosis of a subject having an early stage gastric cancer and for guiding treatment decisions both pre-tumor and post-tumor surgery by identifying subjects who would benefit from combination therapy as opposed to monotherapy in view of the activation state or level detected for the analytes.

In one embodiment, the present invention relates to the method predicts the disease-free survival or progression-free survival of the subject. The gastric cancer may be a diffuse-type gastric cancer.

In one embodiment, the present invention relates to the method as described in [0008a] wherein step (a) further comprises determining the presence or level of activation of at least one additional analyte comprising HER2 and/or HER3 in the cancer cell.

In one embodiment, the present invention relates to the said method in [0008b] wherein HER2 is not activated in the cancer cell, wherein optionally the subject is predicted to have worse survival compared to stage I gastric cancer subjects with cMET activation in the absence of HER1 and/or HER2 activation. In one embodiment, the present invention relates to the said method in [0008b] wherein cMET, HER1, and HER3 are coactivated but HER2 is not activated in the cancer cell, wherein optionally the subject is predicted to have worse survival compared to stage I gastric cancer subjects with cMET activation in the absence of HER1, HER2, and/or HER3 activation.
In one embodiment, the method as described in [0008a-0008c], wherein step (a) comprises determining the presence or level of activation of the analytes with a Collabotrative Enzyme Enhanced Reactive Immunoassay (CEER™).

In another aspect, the present invention relates to a composition comprising a cMET inhibitor and a HER1 inhibitor for use in the treatment of stage I gastric cancer in a subject, wherein cMET and HER1 are coactivated in the stage I gastric cancer, and wherein the subject has worse survival and/or poorer prognosis compared to stage I gastric cancer subjects with cMet activation in the absence of HER1 activation.
In one embodiment, the present invention relates to composition described in [0008d] for use in the treatment of stage I gastric cancer in a subject, wherein the composition is to be administered to the subject as primary therapy.
In another embodiment, the present invention relates to composition described in [0008d] for use in the treatment of stage I gastric cancer in a subject, wherein the composition is to be administered as adjuvant composition after tumor surgery.

In one embodiment, the present invention relates to composition described in [0008d] for use in the treatment of stage I gastric cancer in a subject, wherein HER2 is not activated in the stage I gastric cancer, wherein optionally the subject has worse survival and/or poorer prognosis compared to stage I gastric cancer subjects with cMet activation in the absence of HER1 and/or HER2 activation.

In another embodiment, the present invention relates to composition described in [0008d] for use in the treatment of stage I gastric cancer in a subject, wherein HER3 is activated in the stage I gastric cancer, wherein optionally the subject has worse survival and/or poorer prognosis compared to stage I gastric cancer subjects with cMET activation in the absence of HER1, HER2, and/or HER3 activation.
In one embodiment, the present invention relates to composition described in [0008d]for use in the treatment of stage I gastric cancer in a subject, wherein the cMET inhibitor is selected from the group consisting of AMG102, MetMAb, ARQ197, JNJ-38877605, PF- 2341066, PF-04217903, SGX523, GSK1363089/XL880, XL184, MGCD265, MK-2461, PHA-665752, and combinations thereof, or wherein the HER1 inhibitor is selected from the group consisting of erlotinib, lapatinib, gefitinib, BIBW-2992, and combinations thereof.

In one embodiment, the present invention relates to composition described in [0008d]for use in the treatment of stage I gastric cancer in a subject, wherein the presence of activation or the level of activation of cMET and HER1 is determined in a cancer cell obtained from the subject, wherein the cancer cell is selected from the group consisting of a primary tumor cell, a circulating tumor cell (CTC), an ascites tumor cell (ATC), and combinations thereof.

In one embodiment, the present invention relates to composition described in [0008g] for use in the treatment of stage I gastric cancer in a subject, wherein the presence of activation or the level of activation of cMET and HER1 is determined with a Collabotrative Enzyme Enhanced Reactive Immunoassay (CEER™).

The study set forth in Example 3 below describes the use of the multiplexed CEER™ platform to determine the levels of activated RTKs in fresh frozen gastric cancer tissues and the categorization of gastric cancer patients into potential subgroups (*e.g.*, HER1, HER2, truncated variants of HER2, p95HER2, HER3, cMET, PI3K, and IGF1R). Based on their pathway protein activation patterns, multiple signal protein activation was observed in a subgroup of tumors, and redundant pathway activation inputs led to residual downstream signaling, thus limiting the anti-tumor efficacy of monotherapy targeted against a single RTK. The anti-tumor effect of an HER1/2 inhibitor in combination with a cMET inhibitor in cMET- and HER-coactivated gastric cancer cell lines was determined. A powerful tool was developed which enables clinicians to monitor baseline and evolving alterations in RTK activation profile over treatment using circulating tumor cells and malignant cells isolated from peritoneal fluid (ascites). Taken together, this study provides evidence for concomitant RTK activation in gastric cancer human tissues and establishes a clinical tool to monitor RTK activation as cancer evolves during treatment and progression.

In some embodiments, the method predicts the disease-free survival or progression-free survival of a subject having stage I or II gastric cancer. In certain instances, the stage I gastric cancer is a stage IA or stage IB gastric cancer. In certain other instances, the stage II gastric cancer is a stage IIA or stage IIB gastric cancer.

In particular embodiments, cMET and HER1 are coactivated in the cancer cell. In such embodiments, the subject is predicted to have worse survival (*e.g.*, poorer prognosis) compared to stage I or II gastric cancer subjects with cMET activation in the absence of HER1 activation.

In some embodiments, the stage I or II gastric cancer corresponds to a histological subtype such as, for example, an intestinal-type, diffuse-type, or mixed-type gastric cancer. In particular embodiments, the stage I or II gastric cancer is a diffuse-type gastric cancer.

In other embodiments, step (a) further comprises determining the presence or level of activation of at least one additional analyte comprising HER2 and/or HER3 in the cancer cell. In certain instances, cMET and HER1 are coactivated but HER2 is not activated in the cancer cell. In such instances, the subject is predicted to have worse survival (*e.g.*, poorer prognosis) compared to stage I or II gastric cancer subjects with cMET activation in the absence of HER1 and/or HER2 activation. In certain other instances, cMET, HER1, and HER3 are coactivated but HER2 is not activated in the cancer cell. In such instances, the subject is predicted to have worse survival (*e.g.*, poorer prognosis) compared to stage I or II gastric cancer subjects with cMET activation in the absence of HER1, HER2, and/or HER3 activation.

In some embodiments, a cMET inhibitor is administered in combination with a HER1 inhibitor when cMET and HER1 are coactivated in the cancer cell, and optionally when HER2 is not activated and/or HER3 is activated in the cancer cell.

Total expression and activation (*e.g.*, phosphorylation) levels and/or status of signal transduction molecules can be determined using any of a variety of techniques. In certain embodiments, the expression and/or activation (*e.g.*, phosphorylation) level and/or status of signal transduction molecules in samples such as cellular extracts of cancer cells is detected with an immunoassay such as a single detection assay or a proximity dual detection assay (*e.g.,* a Collaborative Enzyme Enhanced Reactive Immunoassay (CEER™)) as described herein.

In particular embodiments, step (a) comprises determining the presence or level of activation of the analytes (*e.g.*, at least cMET and HER1 and optionally HER2 and/or HER3) with CEER™.

The CEER™ technology is described herein and in the following patent documents: PCT Patent Publication Nos. WO 2008/036802, WO 2009/012140, WO 2009/108637, WO 2010/132723, WO 2011/008990, WO 2011/050069 and WO 2012/088337.

In some embodiments, the method improves the prognosis (*e.g.*, increases disease-free survival or progression-free survival) of a subject having stage I or II gastric cancer. In certain instances, the stage I gastric cancer is a stage IA or stage IB gastric cancer. In certain other instances, the stage II gastric cancer is a stage IIA or stage IIB gastric cancer.

In other embodiments, the subject (*e.g.*, prior to starting the combination therapy) has worse survival and/or poorer prognosis compared to stage I or II gastric cancer subjects with cMet activation in the absence of HER1 activation.

In further embodiments, cMET and HER1 are coactivated but HER2 is not activated in the stage I or II gastric cancer. In such embodiments, the subject (*e.g.*, prior to starting the combination therapy) has worse survival and/or poorer prognosis compared to stage I or II gastric cancer subjects with cMet activation in the absence of HER1 and/or HER2 activation.

In yet other embodiments, cMET, HER1, and HER3 are coactivated in the stage I or II gastric cancer. In such embodiments, the subject (*e.g.*, prior to starting the combination therapy) has worse survival and/or poorer prognosis compared to stage I or II gastric cancer subjects with cMET activation in the absence of HER1, HER2, and/or HER3 activation.

In some embodiments, the combination therapy is administered to the subject as primary therapy (*e.g.,* as pre-operative treatment to shrink the size of a gastric tumor). In other embodiments, the combination therapy is administered to the subject as post-operative adjuvant therapy (*e.g.,* after tumor surgery). In particular embodiments, the combination therapy can be administered both pre-operatively as primary therapy and post-operatively as adjuvant therapy.

In certain embodiments, the cMET inhibitor is selected from the group consisting of foretinib (GSK1363089/XL-880), PHA-665752, AMG102, MetMAb, ARQ197, JNJ-38877605, PF-2341066, PF-04217903, SGX523, XL184, MGCD265, MK-2461, and combinations thereof. In certain other embodiments, the HER1 inhibitor is selected from the group consisting of erlotinib, lapatinib, gefitinib, BIBW-2992, and combinations thereof. In particular embodiments, the combination therapy comprises the cMET inhibitor(s) foretinib and/or PHA-665752 and the HER1 inhibitor lapatinib.

In particular embodiments, the presence or level of activation of analytes such as cMET and HER1 and optionally HER2 and/or HER3 (as well as other signal transduction biomarkers) is determined in a cancer cell obtained from the subject. In some instances, the cancer cell is selected from the group consisting of a primary tumor cell, a circulating tumor cell (CTC), an ascites tumor cell (ATC), and combinations thereof.

In certain embodiments, the method further comprises determining the presence or level of activation of analytes such as cMET and HER1 and optionally HER2 and/or HER3 (as well as other signal transduction biomarkers) in a cancer cell obtained from the subject prior to administering the combination therapy, *e.g.*, to determine whether cMET and HER1 are coactivated in the stage I or II gastric cancer. In particular embodiments, the presence or level of activation of such analytes is determined with CEER™.

Other objects, features, and advantages of the present invention will be apparent to one of skill in the art from the following detailed description and figures.

### BRIEF DESCRIPTION OF THE DRAWINGS

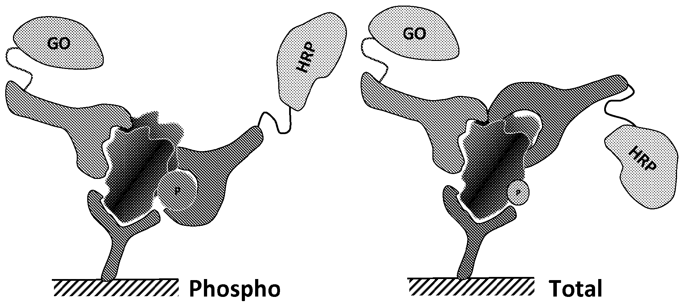

Figure 1 shows one embodiment of the proximity assay format of the present invention (*e.g.*, Collaborative Enzyme Enhanced Reactive-immunoassay (CEER™)), which is particularly useful in determining activated (*e.g.*, phosphorylated) and total analyte levels in a biological sample.

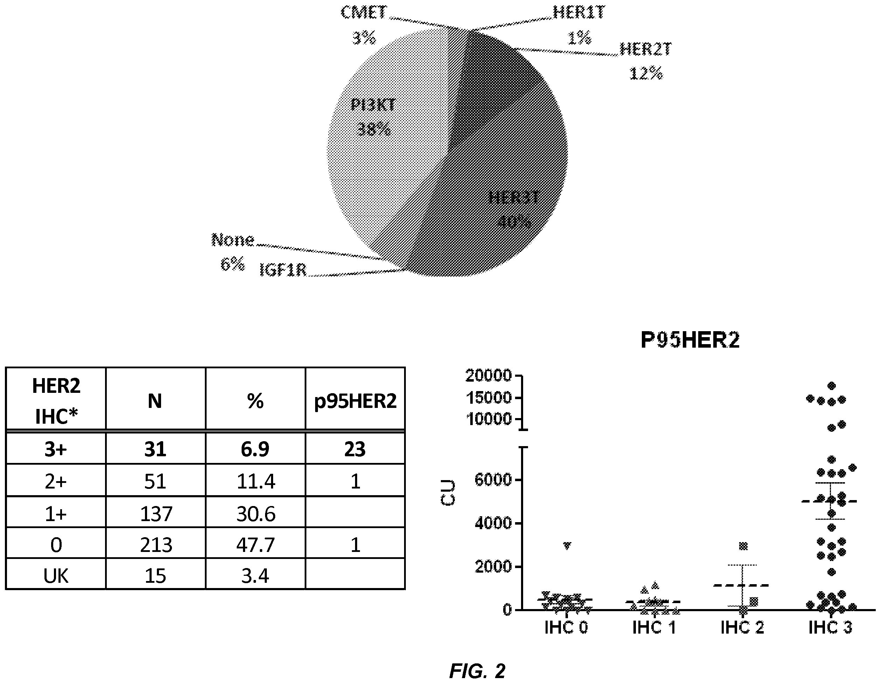

Figure 2 shows the expression of RTKs by CEER™ (top) and IHC (bottom left). Levels of signal proteins in each patient were ranked in order of "HER2, PI3K, HER3, cMET, HER1, IGF1R" before generating the diagram. The presence of p95HER2 in GCA patients (bottom left) and their levels in each HER2 with IHC subtype (bottom right) are also shown.

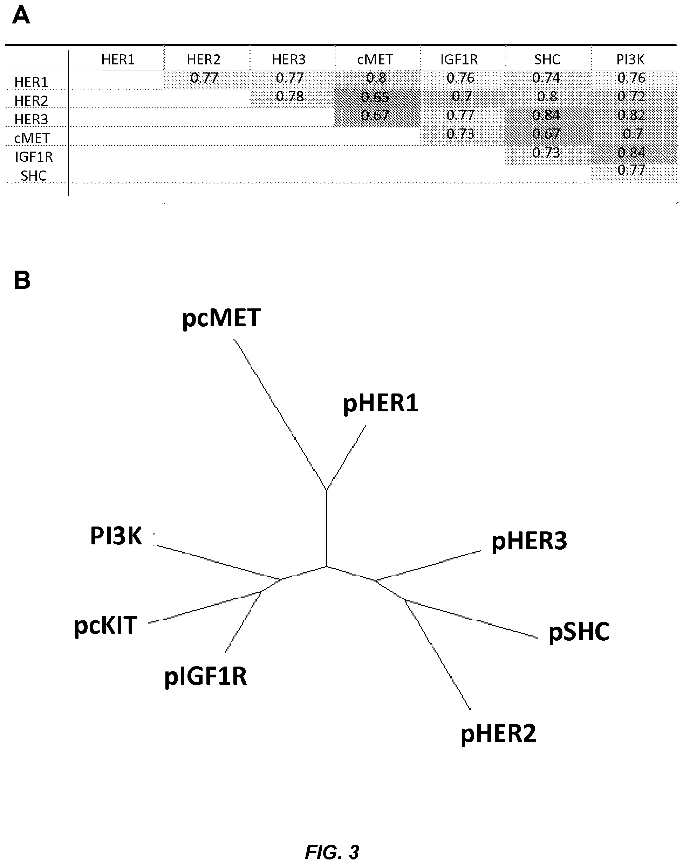

Figure 3 shows an exemplary co-activation correlation index generated by Multi Dimensional Scaling (A) and pattern (B).

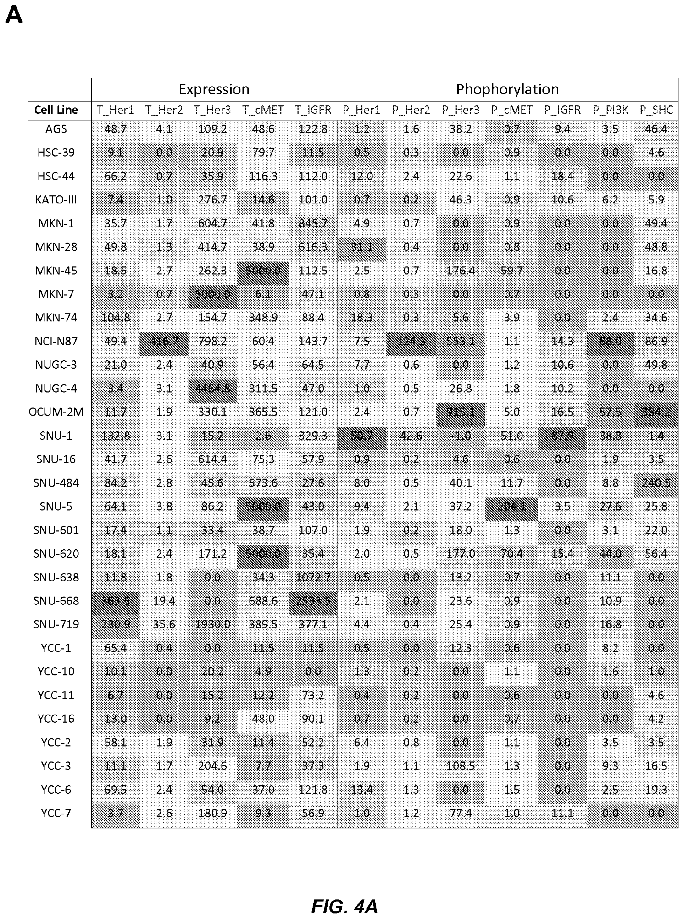

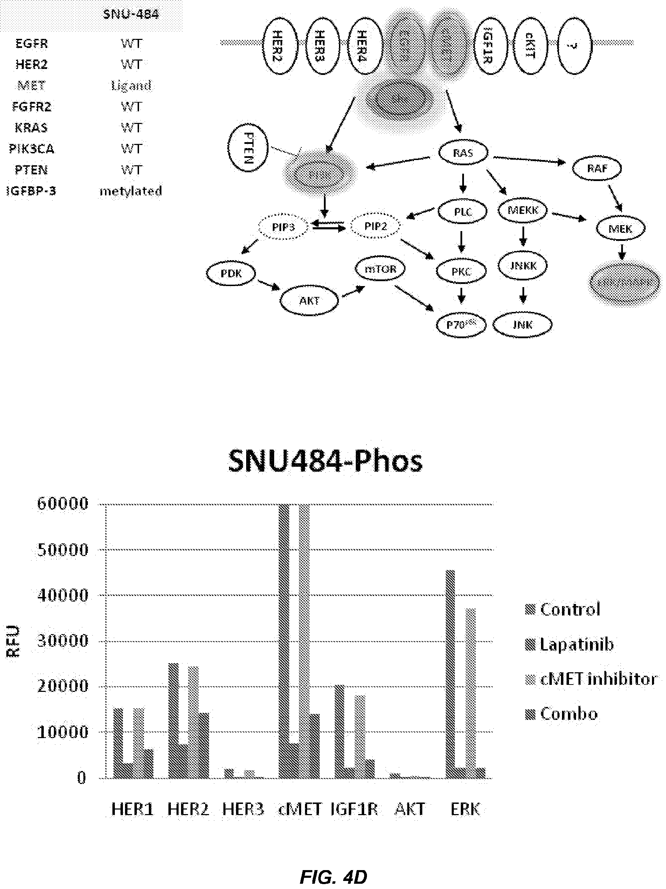

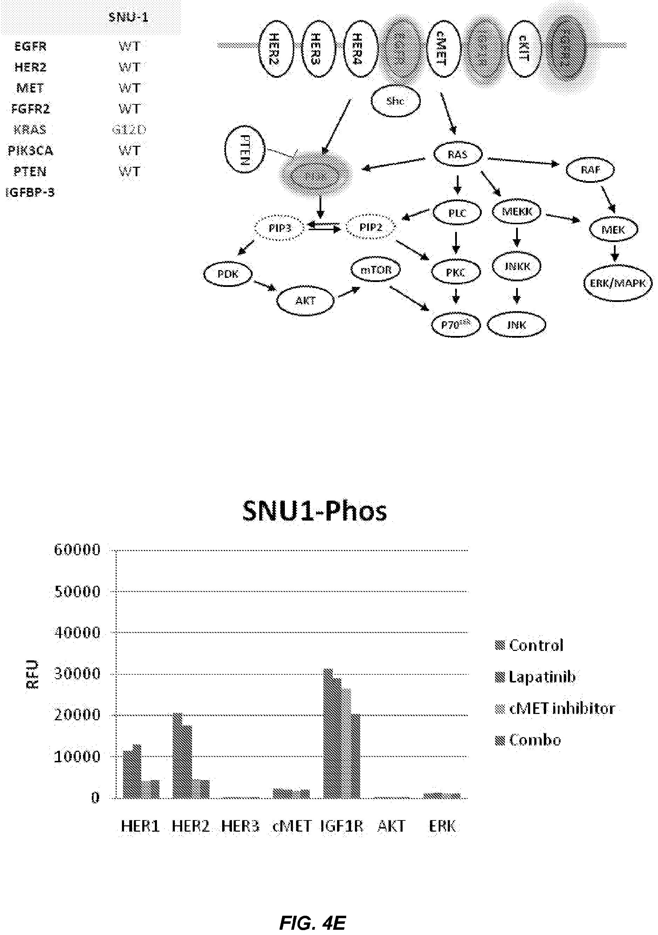

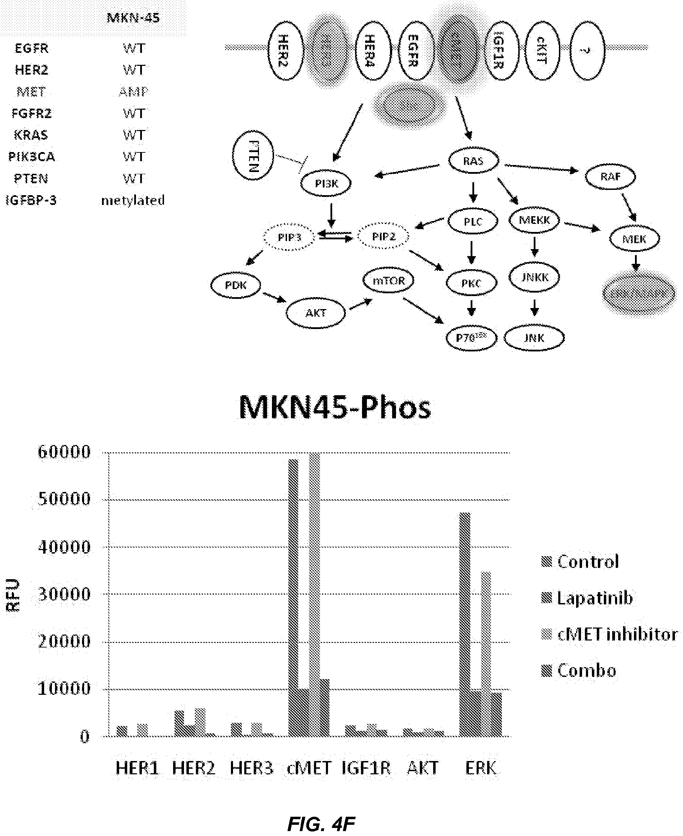

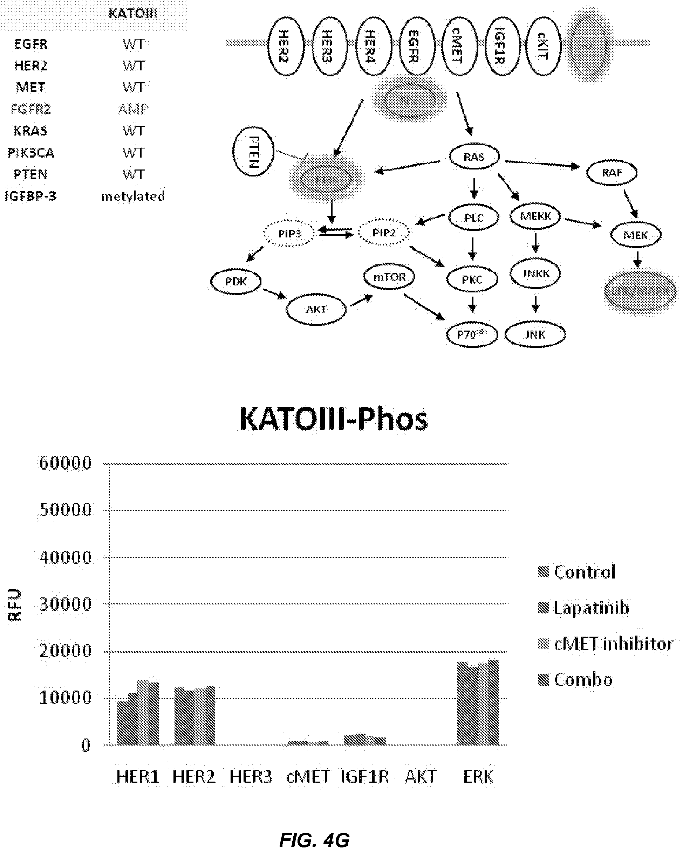

Figures 4A-G show the results of the comprehensive profiling of pathway proteins in a panel of gastric cancer cell lines described in Example 2.

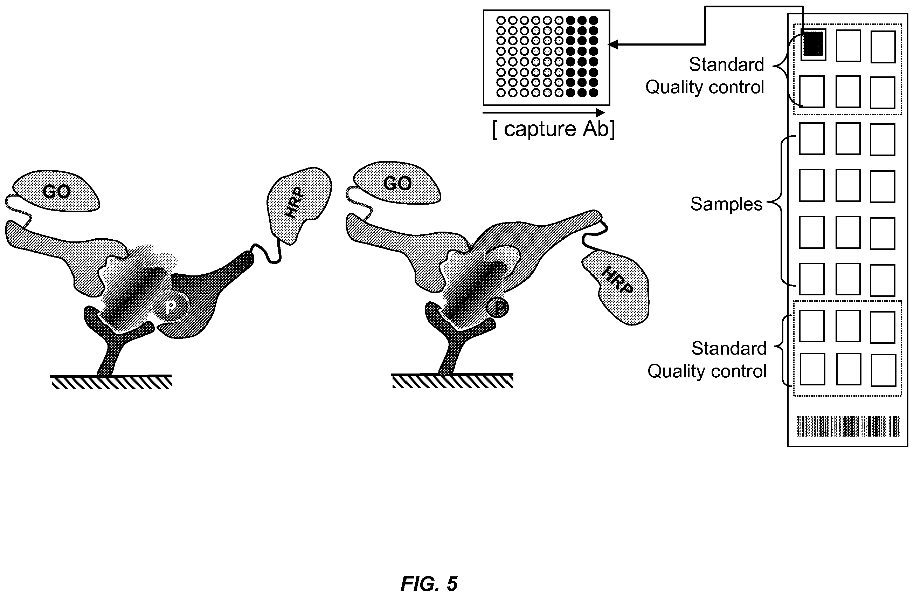

Figure 5 shows an exemplary schematic illustrating the principle of the CEER™ assay and array layout.

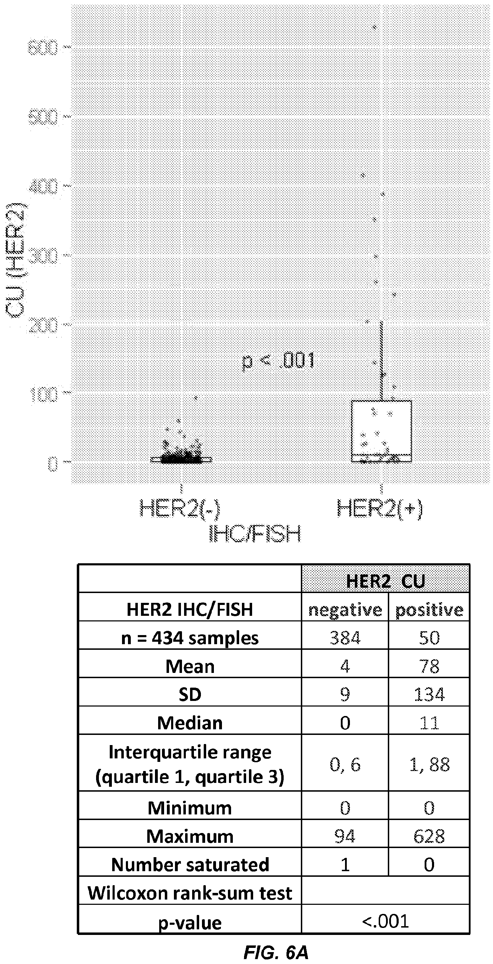

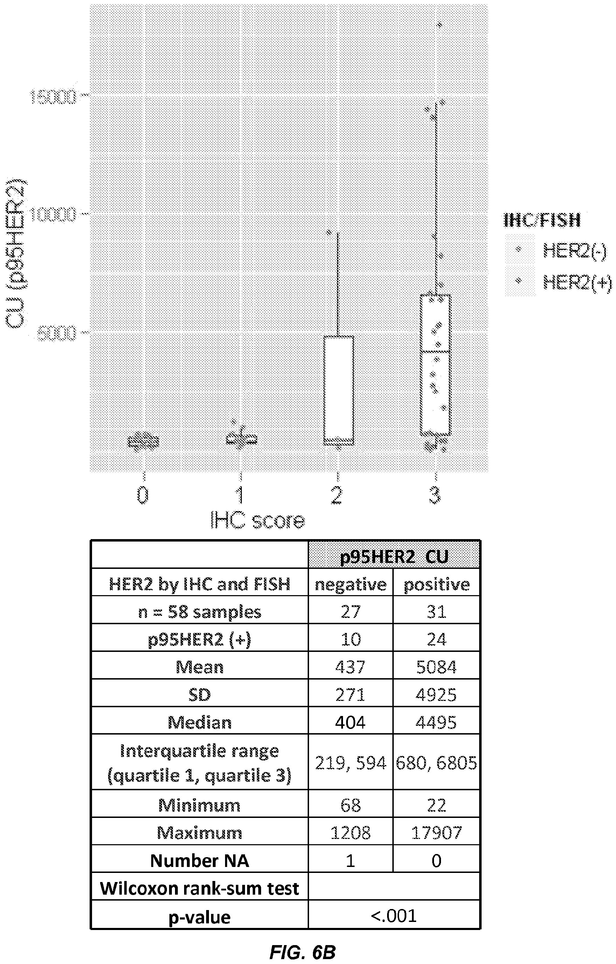

Figure 6 shows HER2 and p95HER2 expression in gastric cancers. (A) CU distribution for HER2 expression of patient samples at 0.25 µg lysate. The *x*-axis represents the IHC/FISH status, and the *y*-axis represents the CU values from CEER™ assay as determined from a BT474 standard curve. Separation is illustrated between the two groups with a median of 0 for the IHC/FISH negative population (384 of 434) compared to a median of 11 for the IHC/FISH positive population (50 of 434). One saturated sample, above the limit of quantitation, is not shown. Boxes represent the interquartile range, with the 75th percentile at the top and the 25th percentile at the bottom. The line in the middle of the box represents the median. Whiskers extend to the highest and lowest value within 1.5 times the interquartile range. *P* value <.001 was determined by Wilcoxon signed-rank test. (B) CU distribution for p95HER2 in a subset of the tumor samples (58 of 434) at 20 µg lysate. The *x-*axis represents the IHC status, and the *y*-axis represents the CU values from CEER™ assay for p95. Full-length HER2 was removed by immuno-depletion prior to the assay. The data points are colored based on the HER2 status by IHC and FISH. As shown, one data point with an IHC of 2 was determined to be positive by FISH analysis. In the bottom graph, CU values are shown for the samples with IHC of 3. Of the 34 samples determined to be positive for p95 by CEER™, 24 (71%) of them were HER2(+) by IHC/FISH.

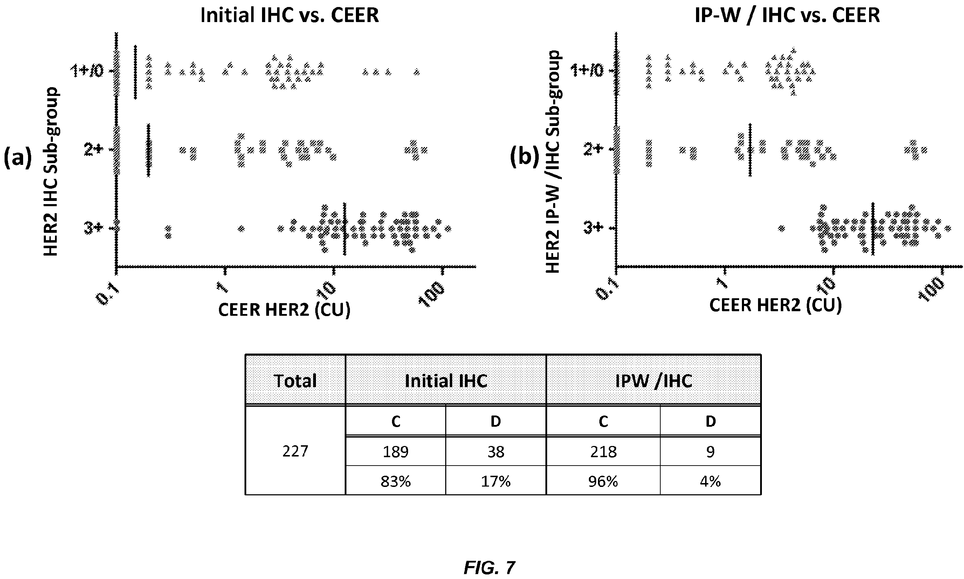

Figure 7 shows a comparison of HER2 expression in breast cancers via IHC and CEER™. The distribution of HER2 expression based on CEER™ in each IHC sub-group in breast cancer is shown in (A). There were 17% (38/227) discordant calls between IHC and CEER™ HER2 status (C -concordance, D - discordance). The outcome of IP-W analysis of 100 samples (including all with discordant calls) is summarized in (B). The concordance between CEER™ and IPW adjusted HER status is shown.

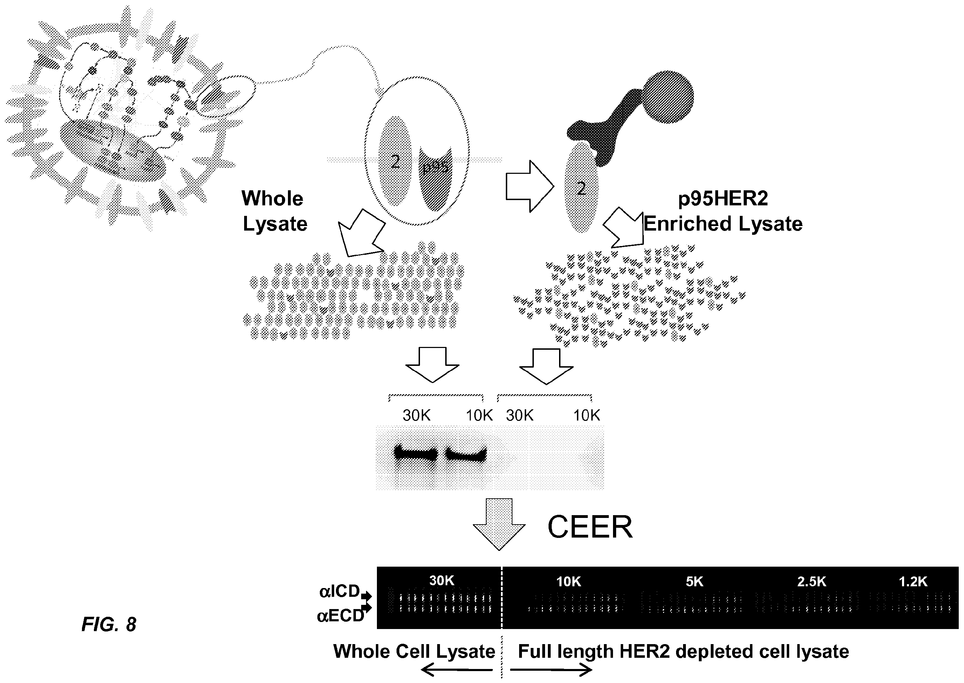

Figure 8 shows a non-limiting example of a strategy for determining full-length and truncated p95HER2 expression using the CEER™ assay.

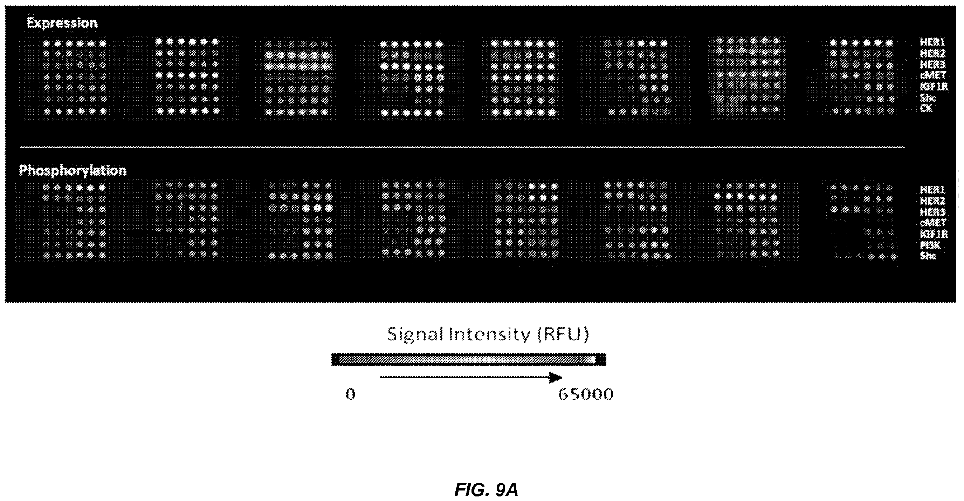

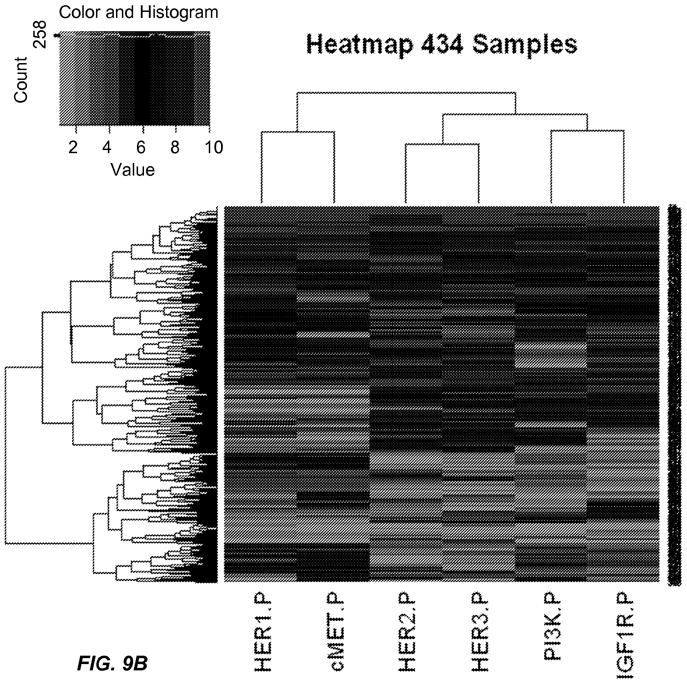

Figure 9 shows the profiling of phosphorylated markers in gastric cancers. (A) Representative immuno-array images are shown for pathway profiling of indicated signal transduction proteins. Array signal intensity ranges from black/dark blue (low) to red/white (high/saturation). (B) Heat map and hierarchical clustering of the 434 samples based on CU values from CEER™ assay for activated (phosphorylated) markers measured at 10 µg lysate concentration. Each column represents a marker and each row represents a patient sample. Relative levels of activation are depicted with a color scale where red represents the highest level of activation and green represents the lowest level. The CU values for each marker (column) were ranked by deciles. Jitter, between 0 and 0.1, was added to each biomarker CU value to create equally sized bins. Row and column dendrogram show the result of the hierarchical clustering calculation.

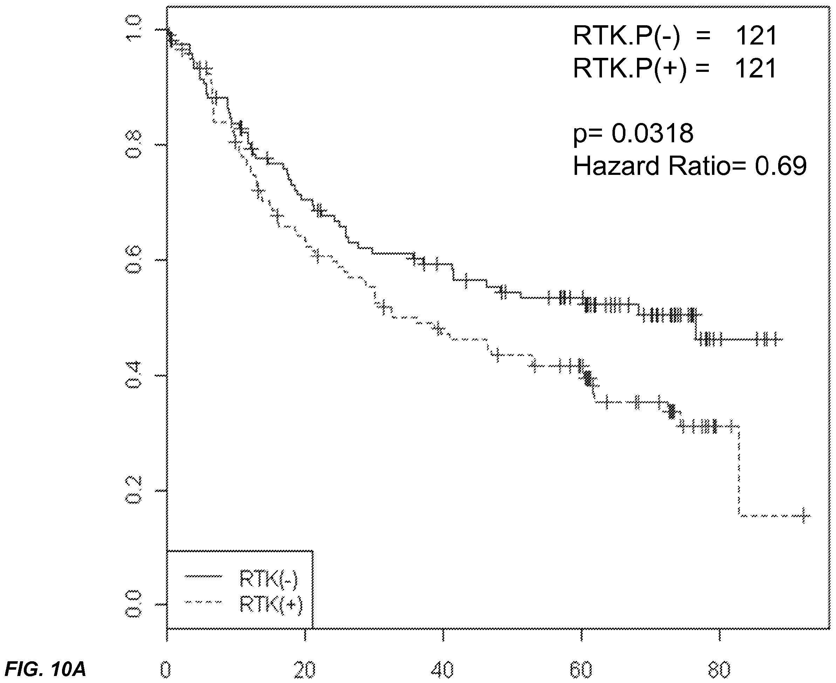

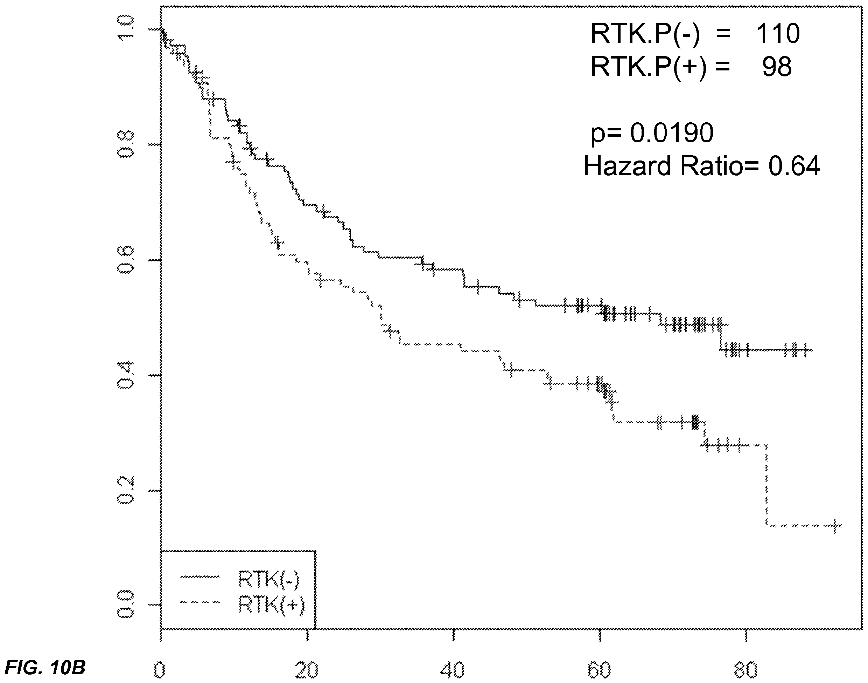

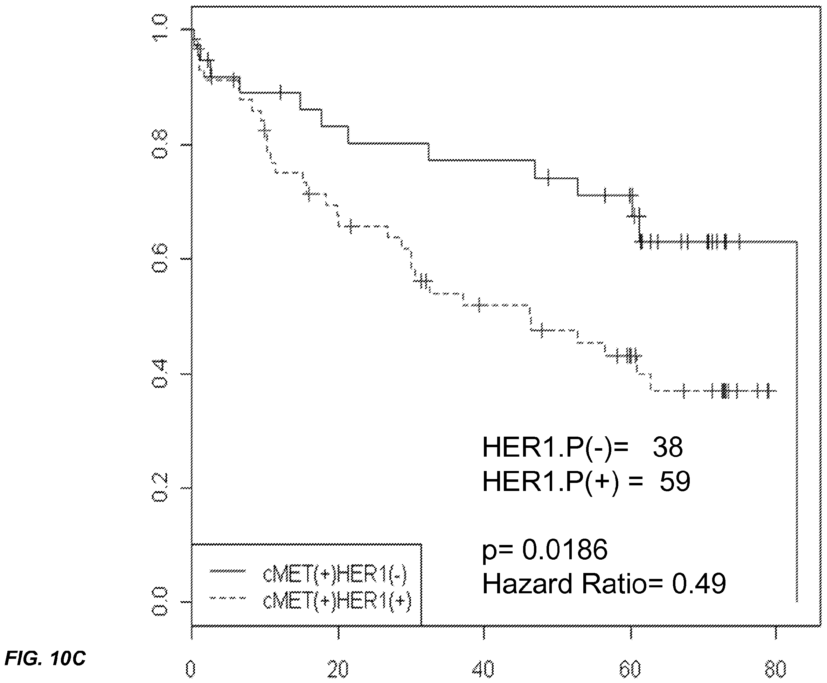

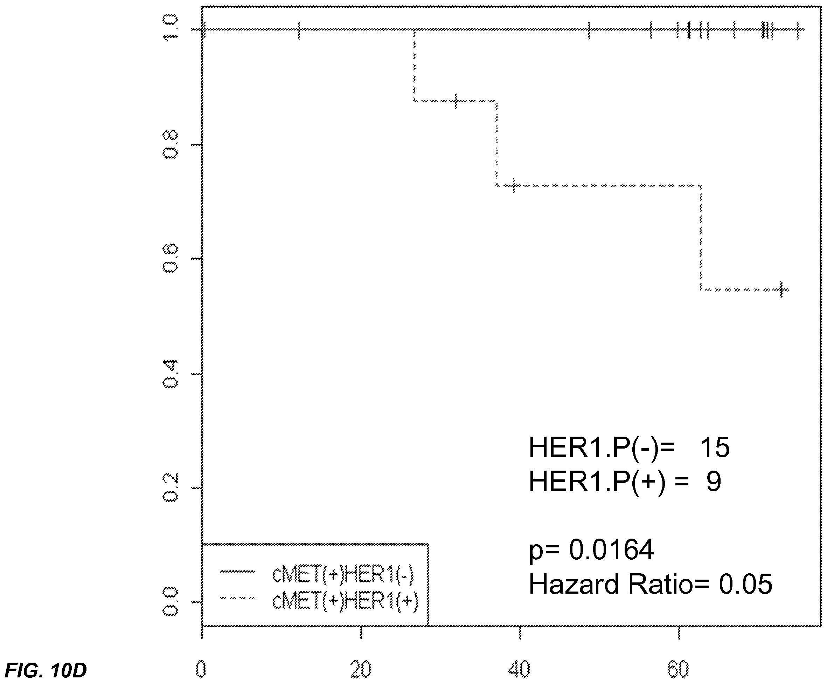

Figure 10 shows the disease-free survival differences in gastric cancers based on activated RTK profiling. (A & B) Disease free survival differences after curative surgery in gastric cancer samples of tumor stages II+III. The analysis compared the overall gastric cancer sample set (A) or only HER2(-) gastric cancer sample set (B) between cohorts with 0 RTK activation vs ≥1 RTK activation. Median survival of the two cohorts in all patients with stage II+III gastric cancer is 32.63 months (≥1 RTK activation) and 76.53 months (0 RTK activation) and in patients with HER2(-) stage II+III gastric cancer is 30.10 months (≥1 RTK activation) and 68.13 months (0 RTK activation). (C & D) Disease-free survival differences after curative surgery comparing c-MET(+) HER1(-) vs c-MET(+) HER1(+) cohorts in HER2(-) (C) or Stage I, HER2(-) (D) gastric cancer samples. Median survival of the two cohorts in HER2(-) patients is 46.17 months (c-MET(+) HER1(+)) and 82.80 months (c-MET(+) HER1(-)). Median survival times in patients with HER2(-) stage I gastric cancer are undefined for both cohorts. Sample numbers in each cohort, p-values and hazard ratios are indicated.

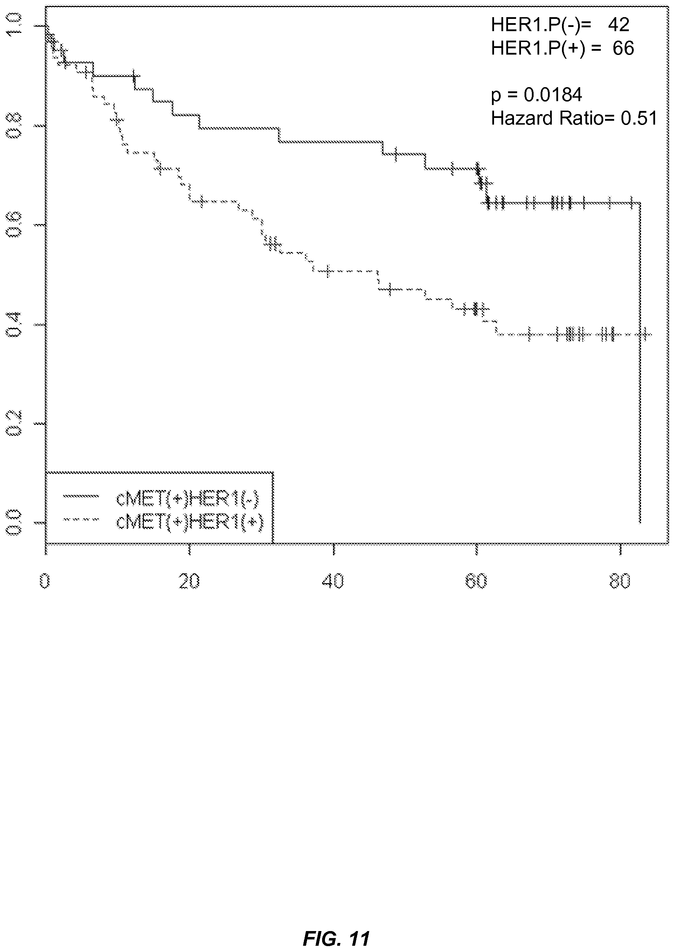

Figure 11 shows the disease-free survival differences between c-MET(+) HER1(-) vs. c-MET(+) HER1(+) gastric cancer cohorts. Disease-free survival differences after curative surgery in all (HER2(+) and HER2(-)) gastric cancer samples comparing the c-MET(+) HER1(-) vs c-MET(+) HER1(+) cohorts. Median survival of the two cohorts in gastric cancer patients is 46.17 months (c-MET(+) HER1(+)) and 82.80 months (c-MET(+) HER1(-)). Sample numbers in each cohort, p-values and hazard ratios are indicated.

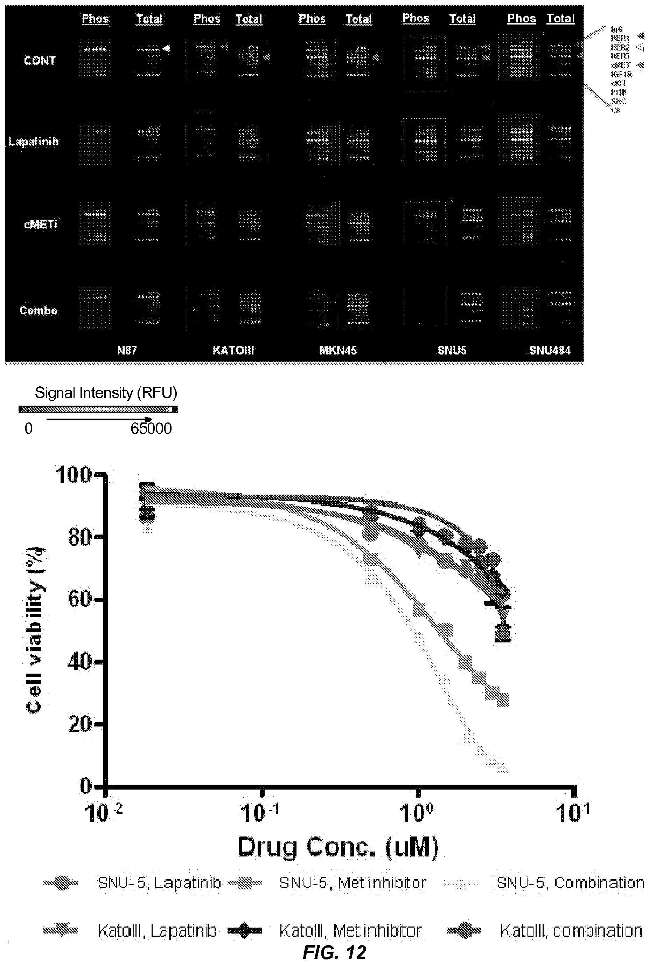

Figure 12 shows the inhibition of activated HER1/HER2 and MET in gastric cancer cells. Modulations of pathway proteins post lapatinib/cMET inhibitor treatments are shown along with the phenotypic effect of pathway inhibition on cancer cell growth inhibition with respective therapeutic treatments. HER1, HER2 and c-MET are indicated by red, yellow and green arrows, respectively.

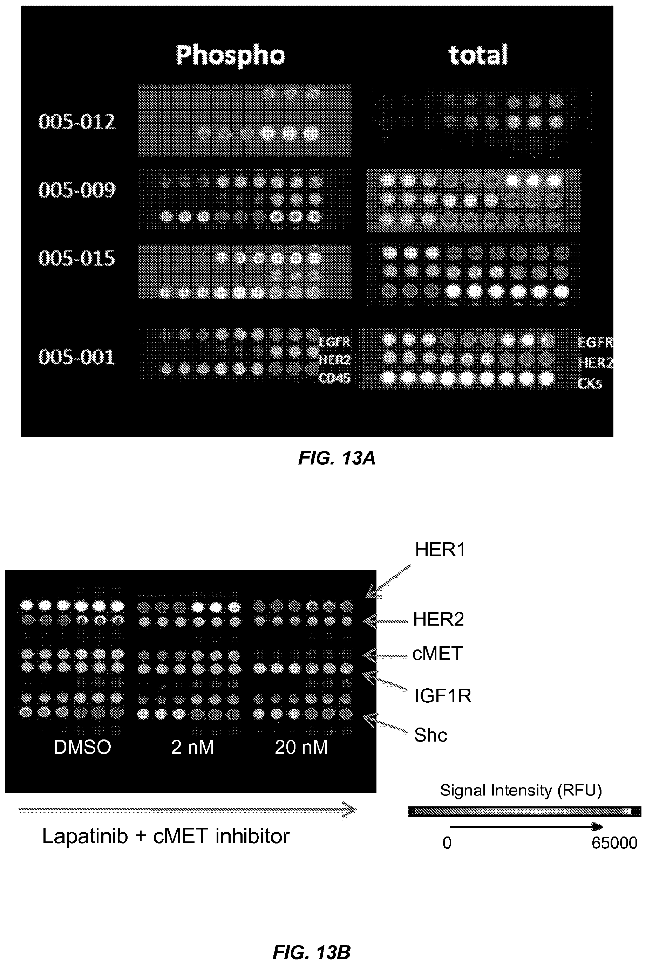

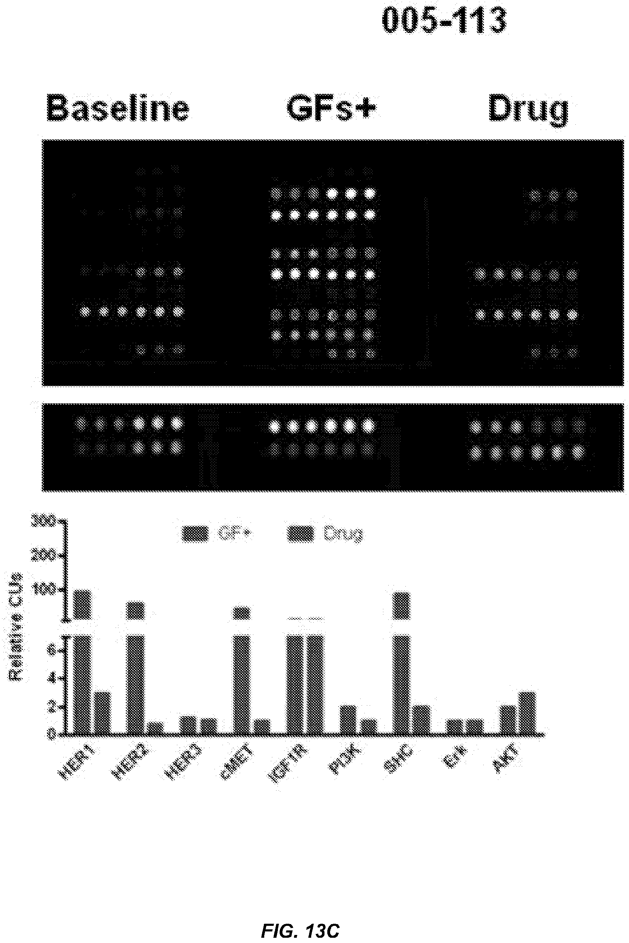

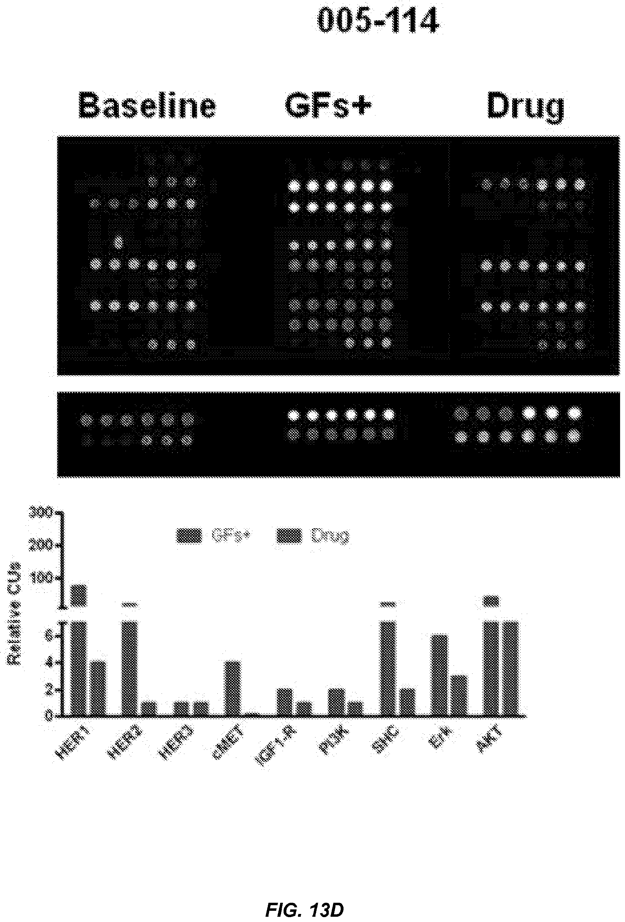

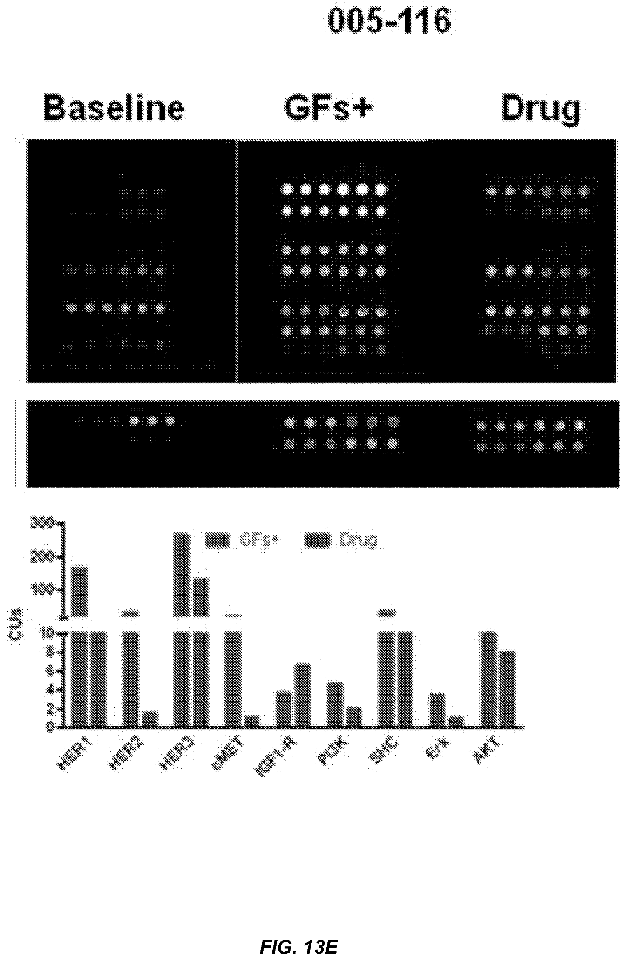

Figure 13 shows the profiling of phosphorylated markers in CTCs and ATCs from gastric cancer patients. (A) The expression and activation of HER1 and HER2 in CTCs isolated from gastric cancer patients are shown. Phospho-HER1, phospho-HER2, total HER1, total HER2 and CKs in CTC lysate were detected using CEER™. (B) Tumor cells isolated from ascites fluid obtained from gastric cancer patient 005-110 were treated with drugs (Lapatinib and cMET inhibitors) at indicated dosage for 4 hours. Modulations in phospho-proteins post-treatment are detected by CEER™. Clear reduction in the levels of pHER1, pHER2 and p-cMET were observed upon drug treatment in a dose dependent manner. While target-specific signal reduction was observed, an increased activation of non-targeted RTK, IGF1R, was also observed. (C-E) Tumor cells isolated from ascites fluid collected from gastric cancer patients were treated with a cocktail of growth factors (EGF, Heregulin, HGF and IGF) with or without 2 µM inhibitor cocktail (lapatinib and PHA-665,752) or DMSO for 4 hours at 37°C. Upon completion of growth factor/drug treatment, cells were lysed and profiled using CEER™. The relative CU was defined as the ratio of CUs over baseline (no drug treatment and no growth factor stimulation). The growth factor treatment induced activation of multiple signal transduction proteins. Drug treatment reduced activation of corresponding target proteins.

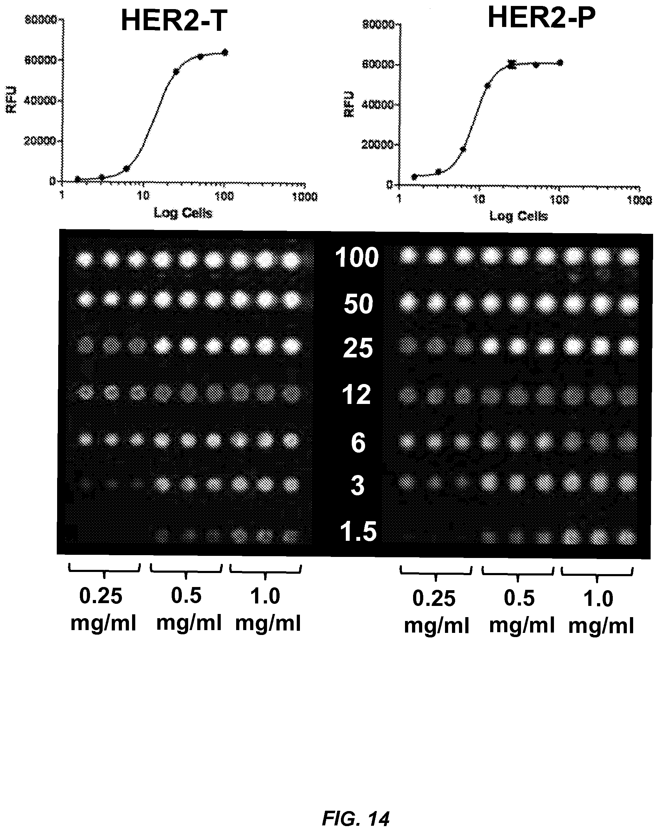

Figure 14 shows a standard curve for total HER2 and phosphorylated HER2. A standard curve of serially diluted cell lysates prepared from BT474 was used to normalize HER2 expression and the degree of phosphorylation in each sample. Each curve was plotted as a function of log signal intensity, measured as relative fluorescence unit (RFU) vs. log concentration of cell lysates and referenced to the standard cell lines. Image shown at single PMT setting, but multiple PMT scanning extends the dynamic range for the quantitation.

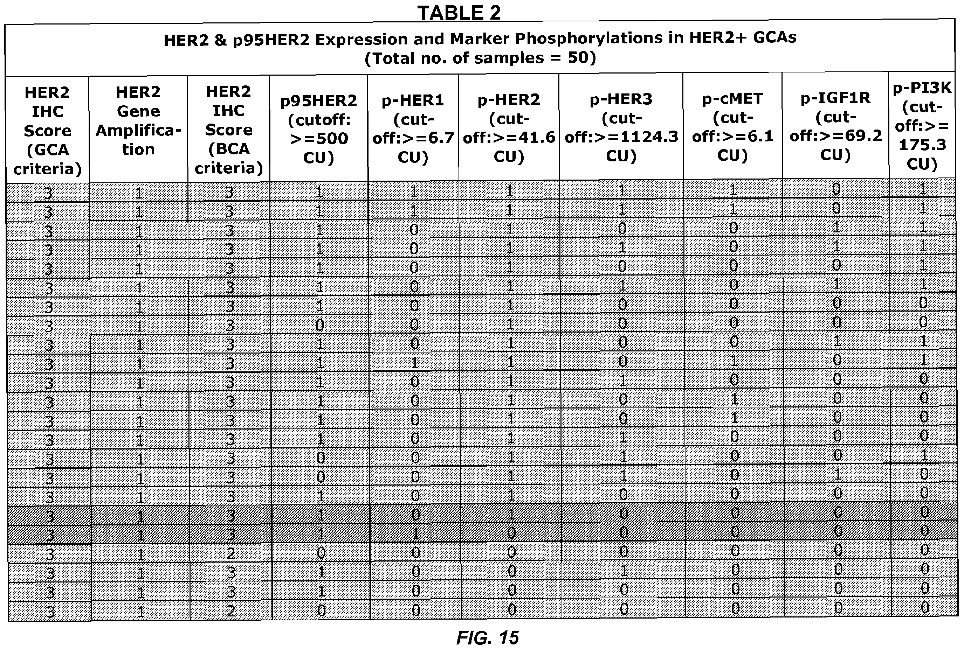

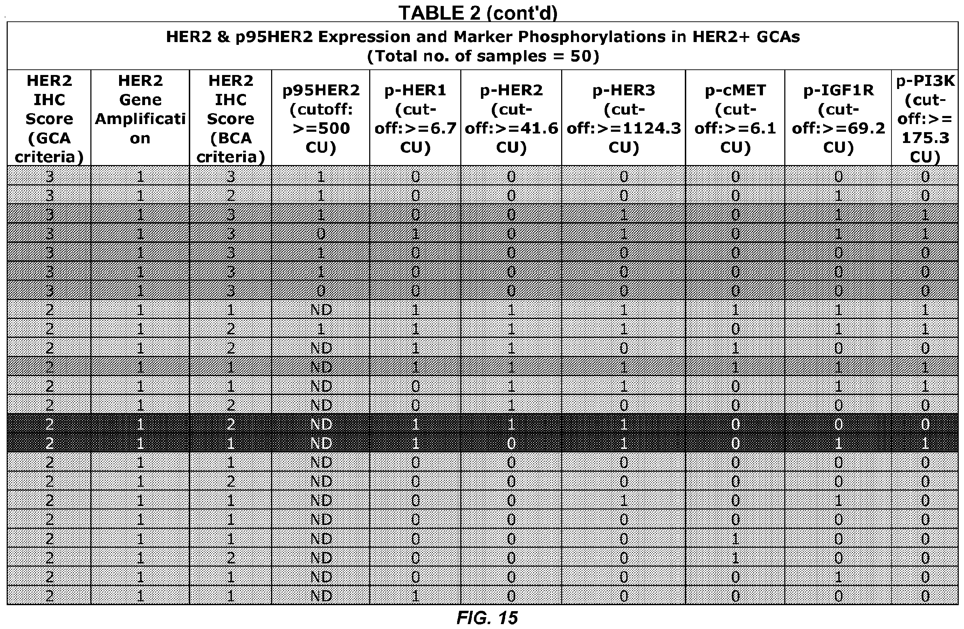

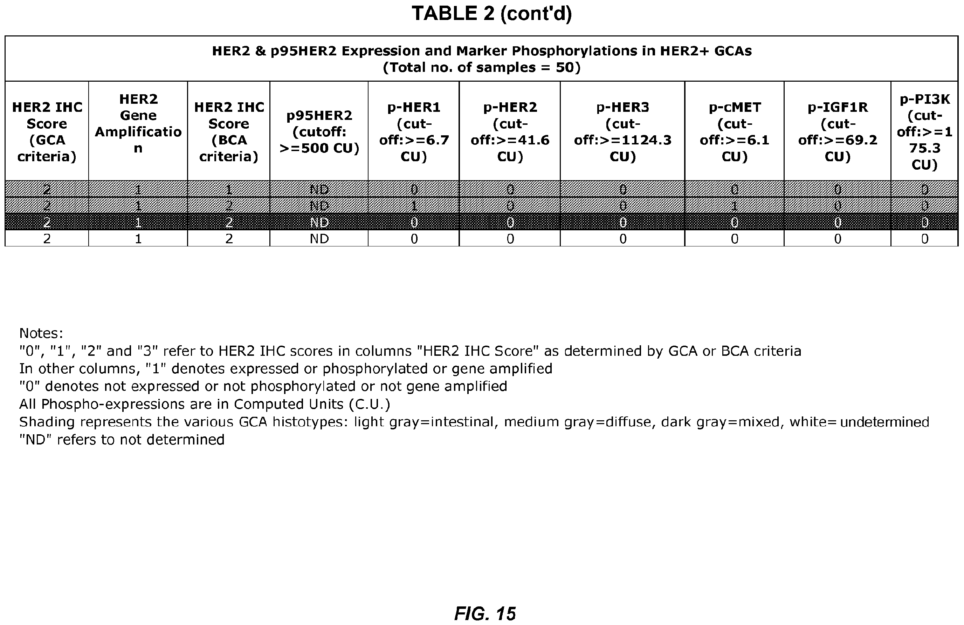

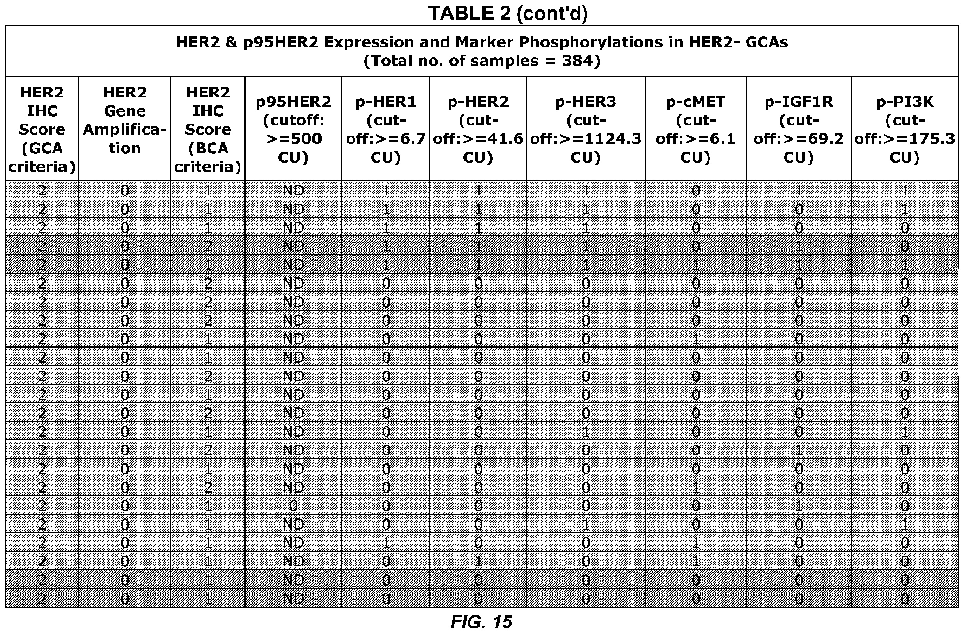

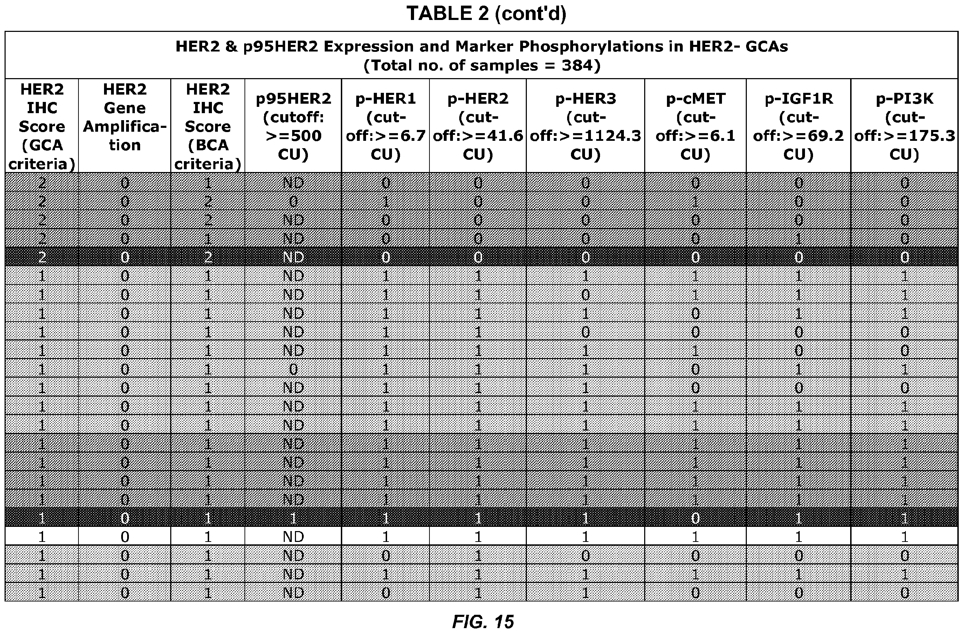

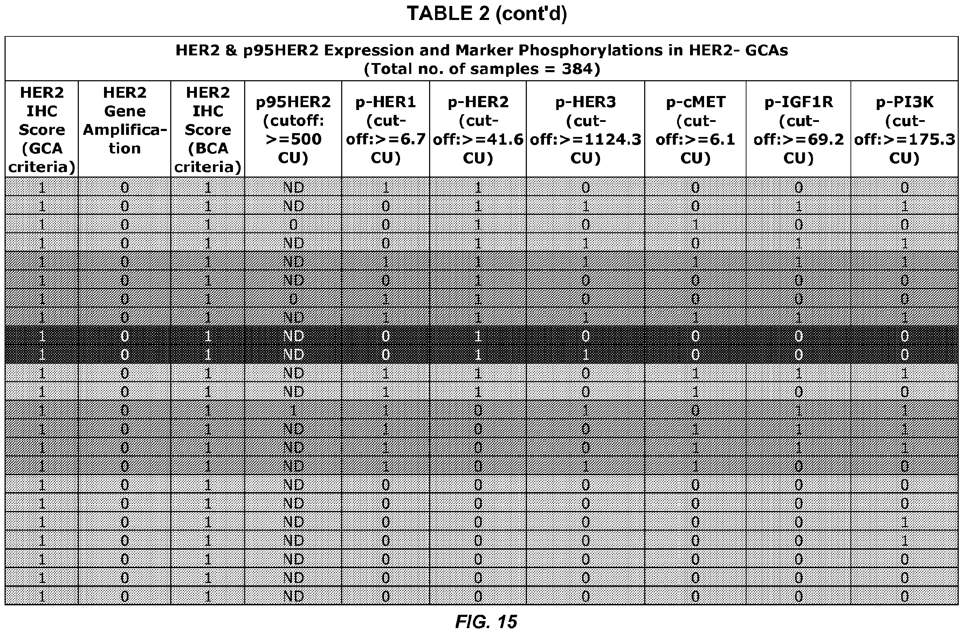

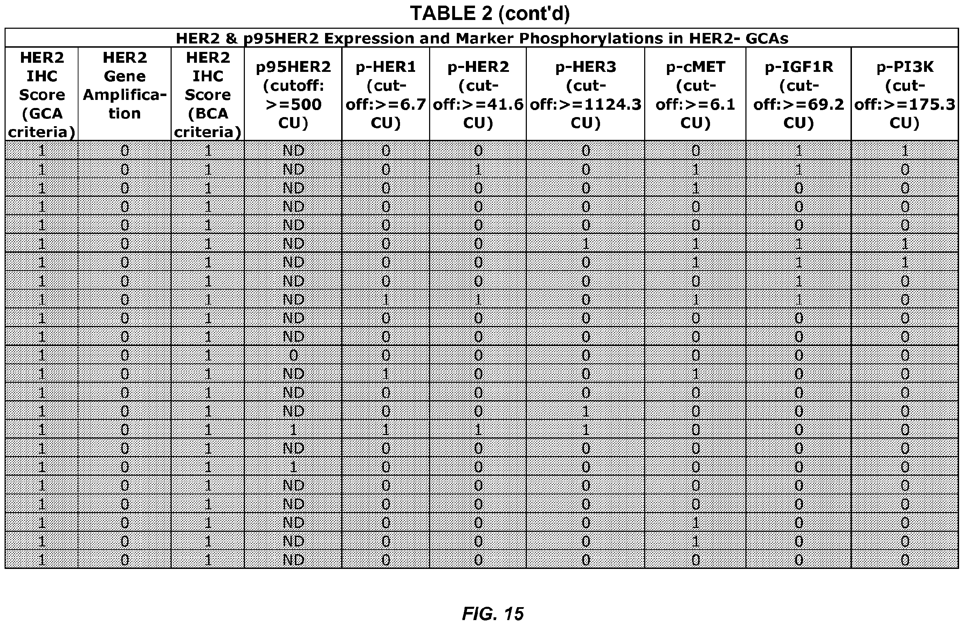

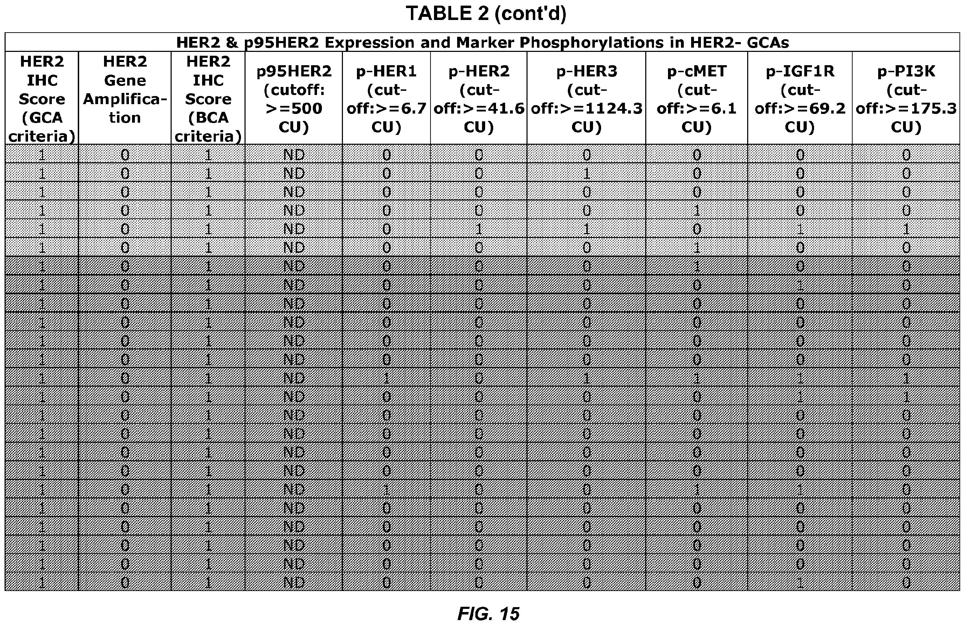

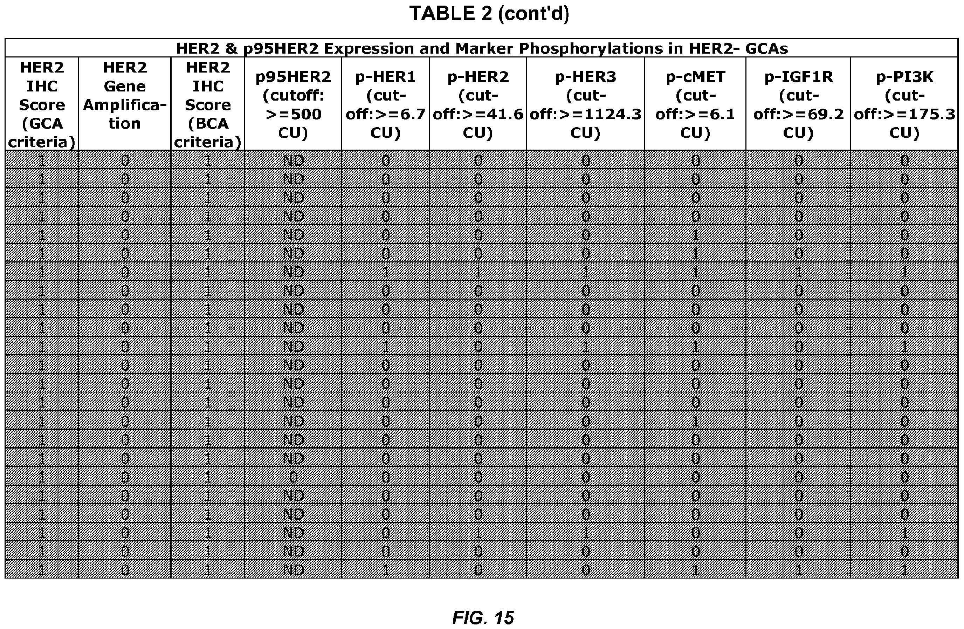

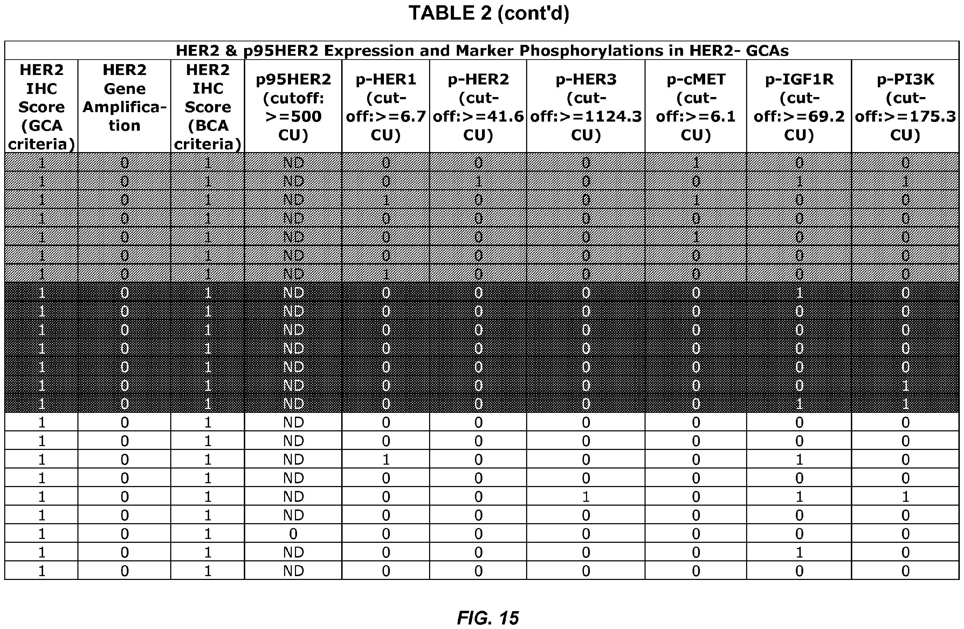

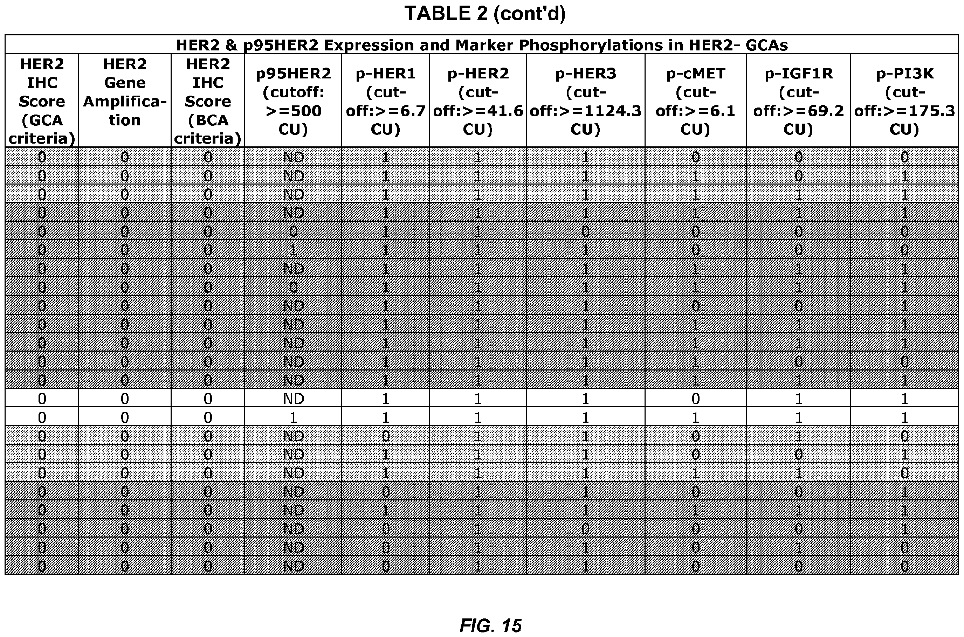

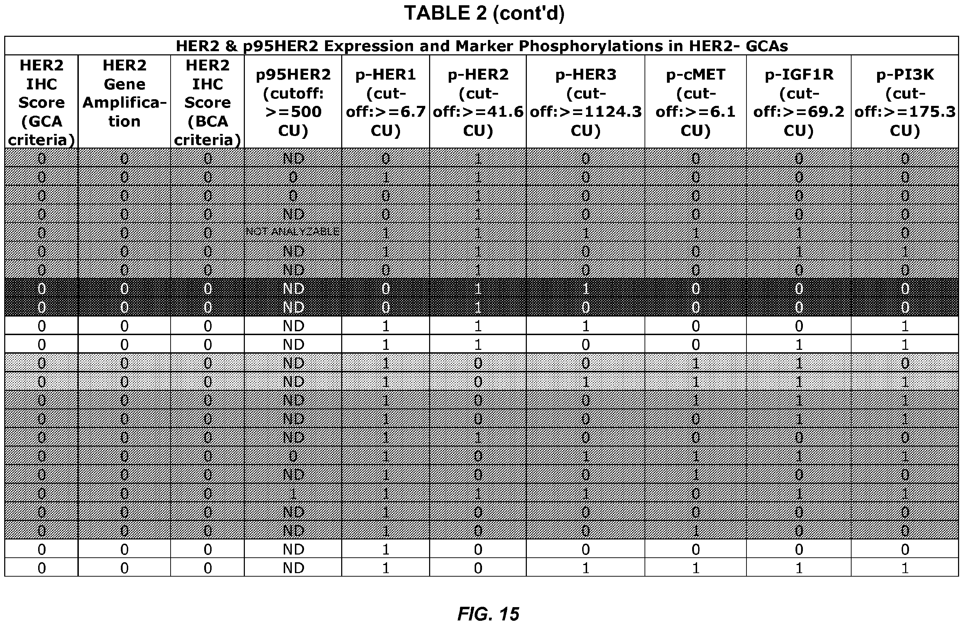

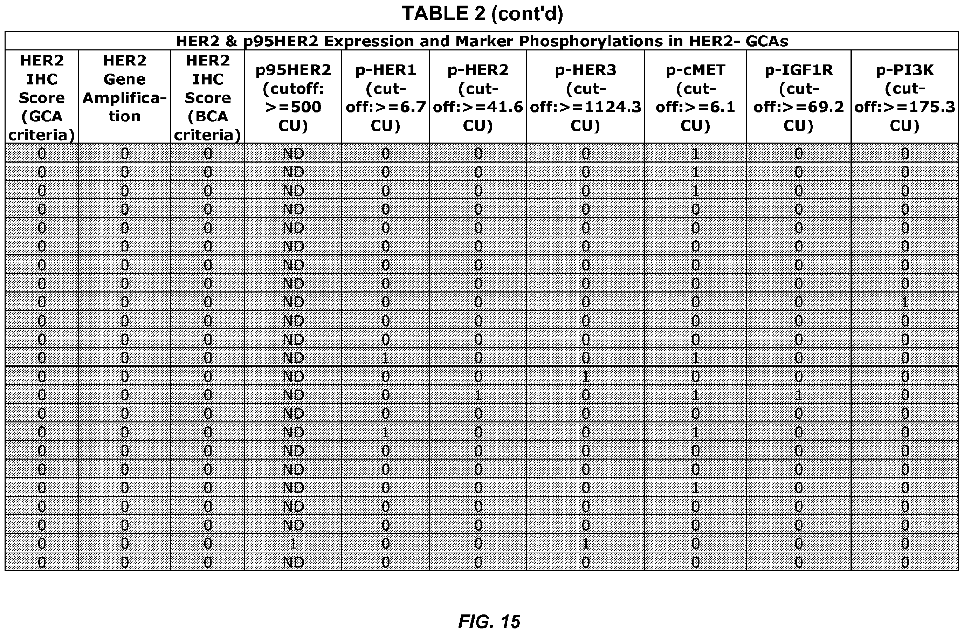

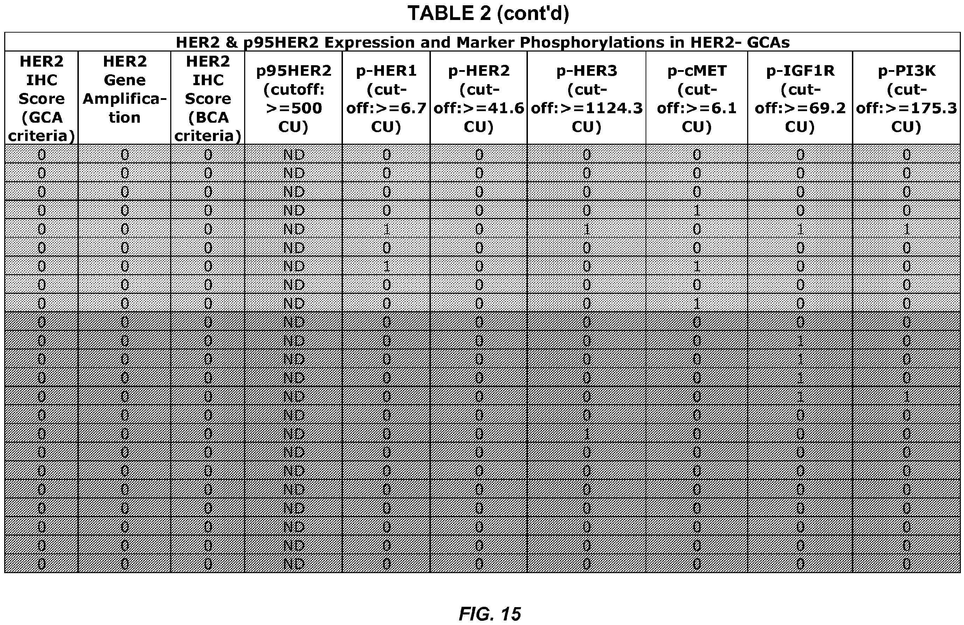

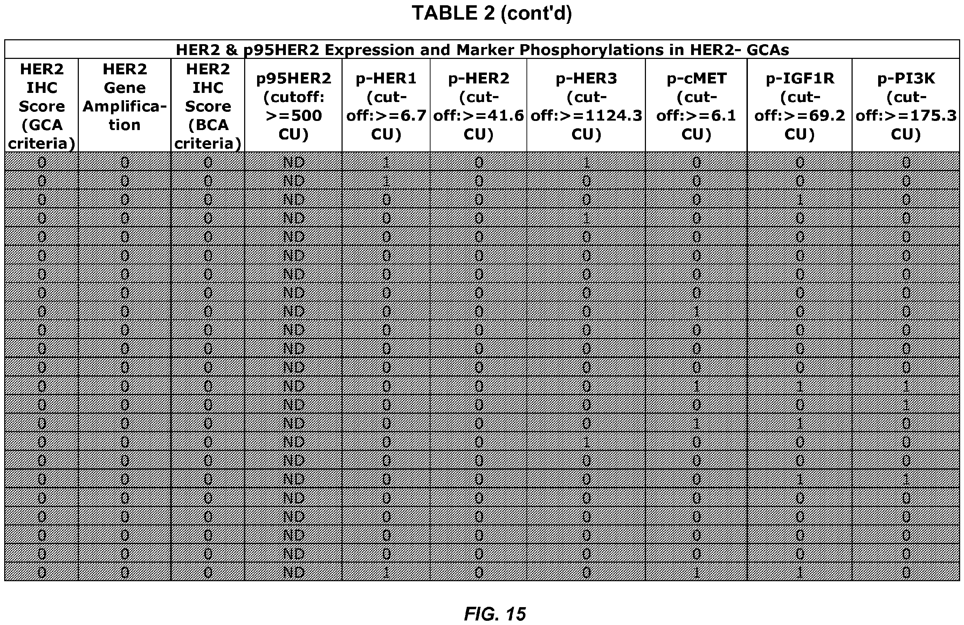

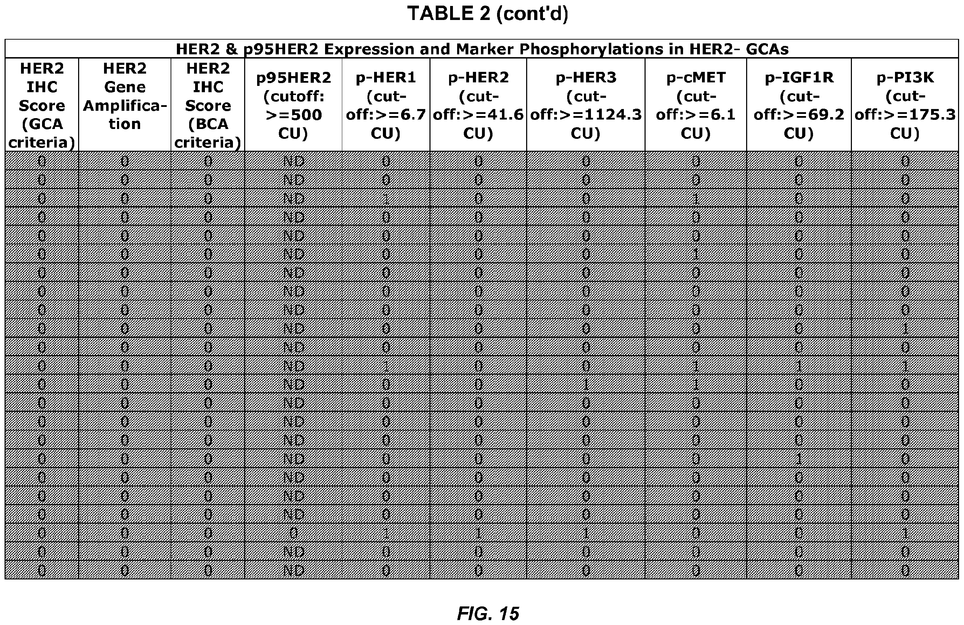

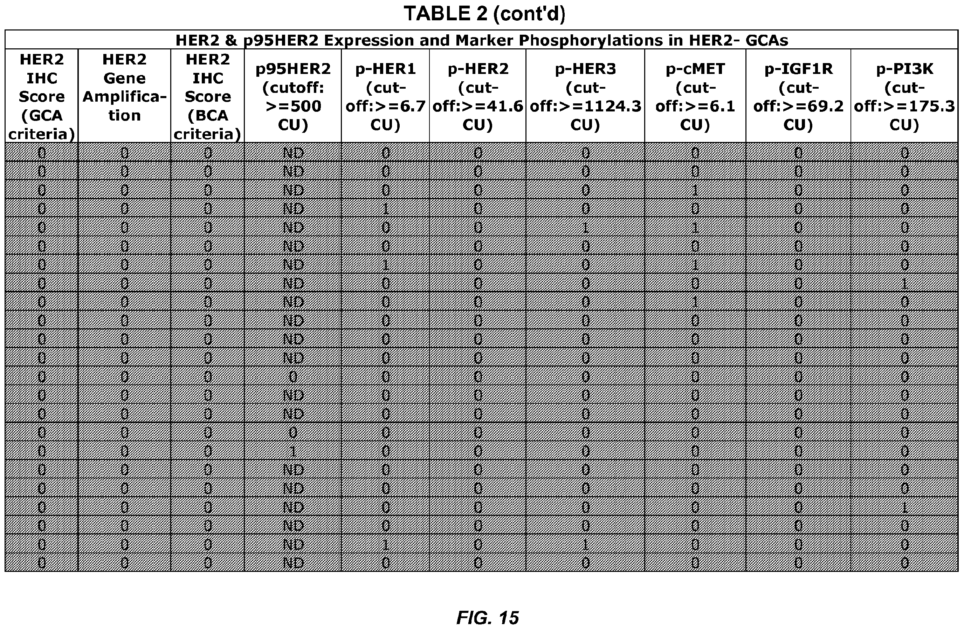

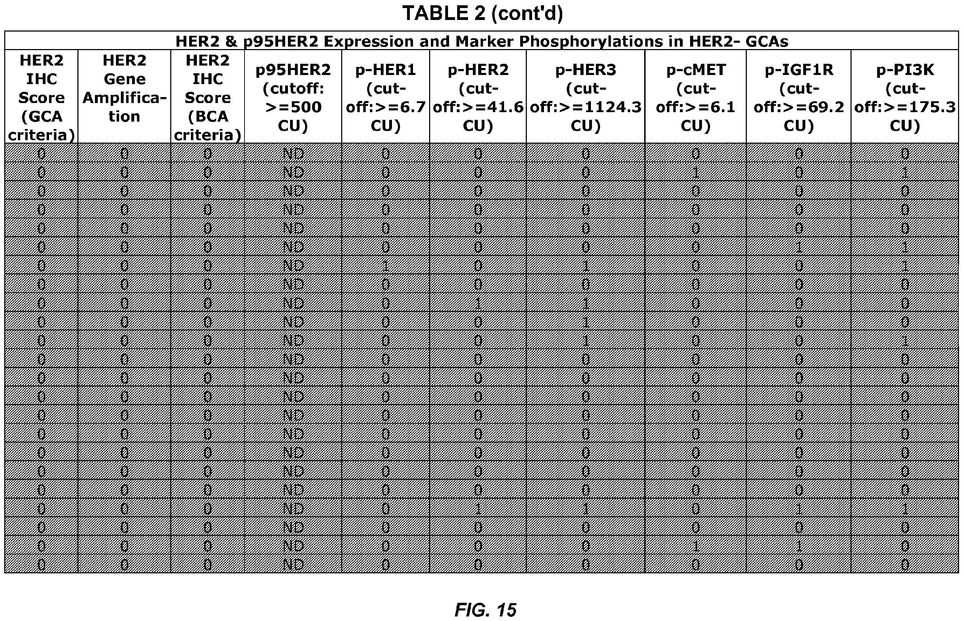

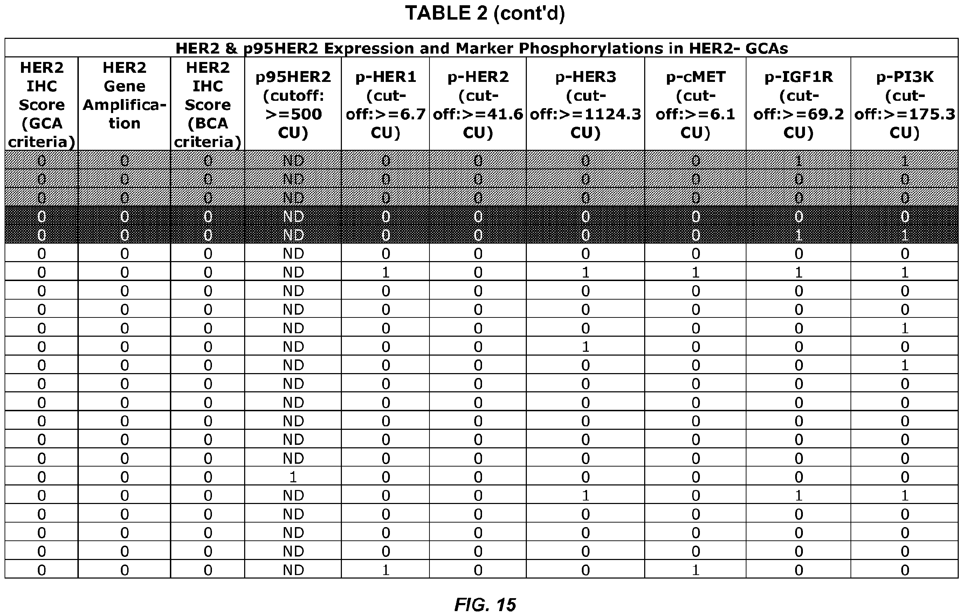

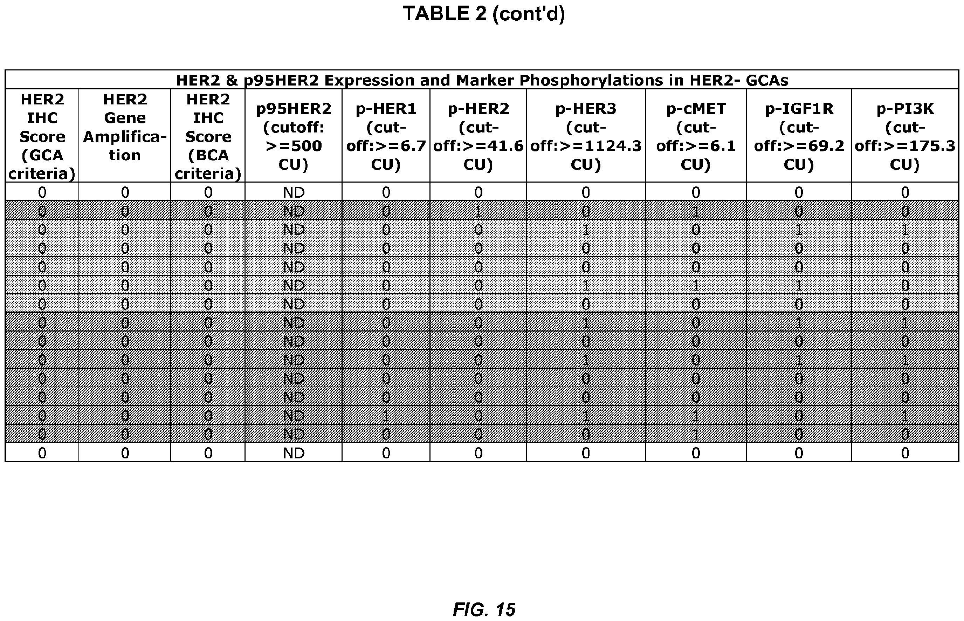

Figure 15 is Table 2. This table shows the HER2 gene amplification status ('1' is gene amplified and '0' is not gene amplified) and HER2 IHC scores (represented as '0', '1', '2' or '3') of HER2(+) and HER2(-) gastric cancer samples both based on gastric cancer (GCA) and breast cancer (BCA) scoring criteria. The histological subtypes of the gastric cancer samples are shaded: intestinal-type (light gray), diffuse-type (medium gray), and mixed-type (dark gray). The table also summarizes the p95HER2 expression status ('1' is p95(+), '0' is p95(-), 'ND' is not determined) and the phosphorylation status ('1' is phosphorylated and '0' is not phosphorylated) of HER1, HER2, HER3, c-MET, IGF1R and PI3K signaling molecules in each sample. Respective CU cut-offs for each marker are indicated.

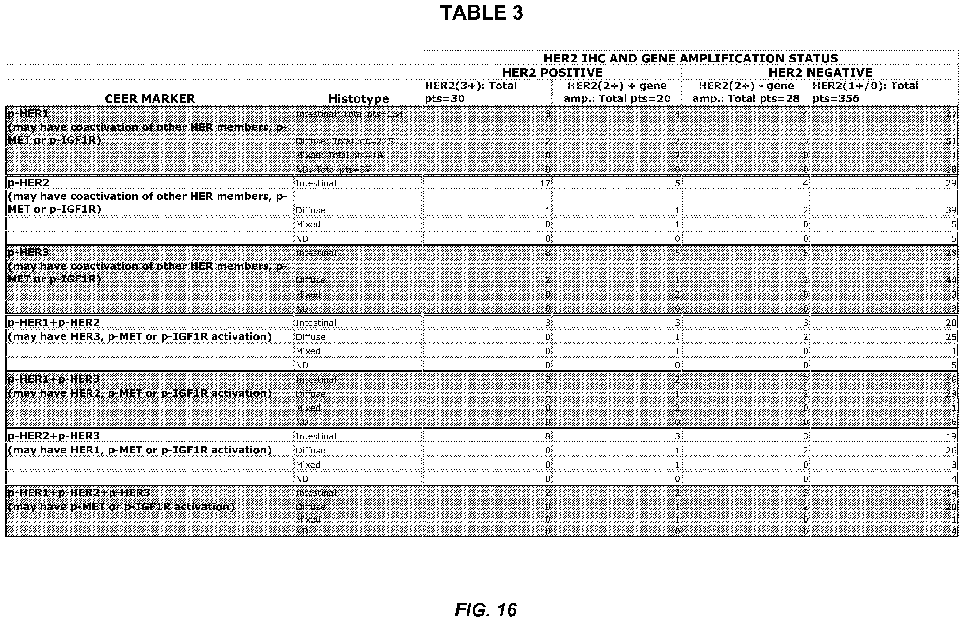

Figure 16 is Table 3. This table shows the distribution of sample numbers with respect to each activated HER kinase axis receptor member and its co-activations with other HER members. HER2 status and histological subtype of the samples are indicated.

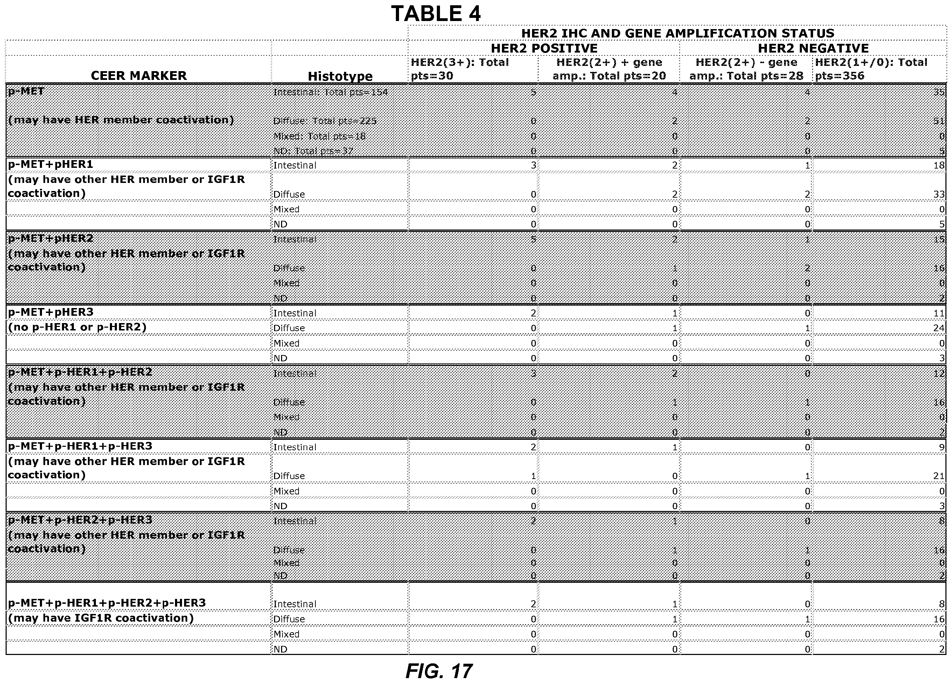

Figure 17 is Table 4. This table shows the distribution of sample numbers with respect to activated c-MET receptor and its co-activation patterns with other RTKs. HER2 status and histological subtype of the samples are indicated.

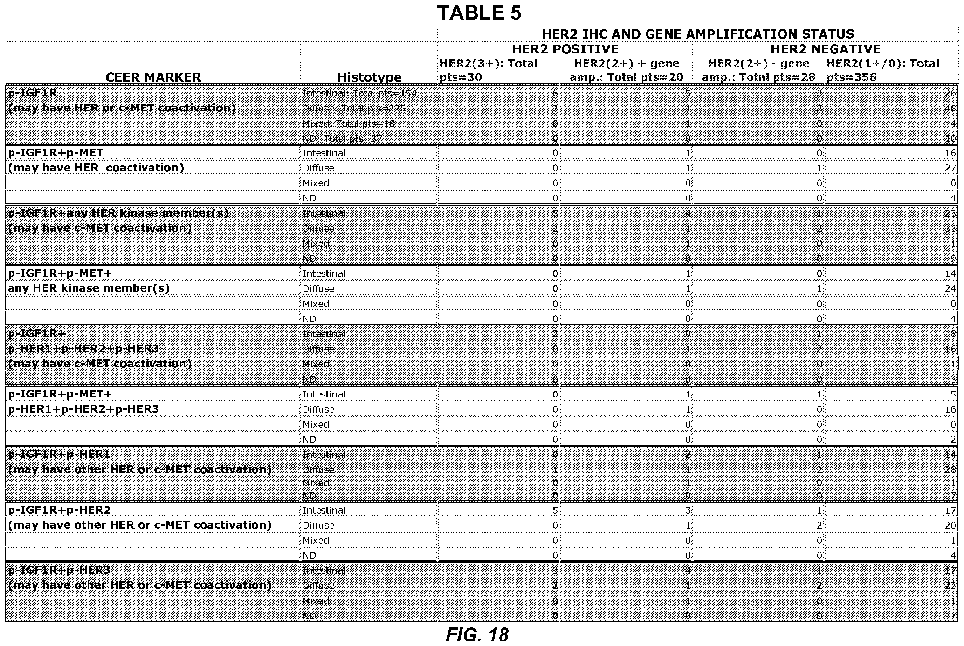

Figure 18 is Table 5. This table shows the distribution of sample numbers with respect to activated IGF1 receptor and its co-activation patterns with other RTKs. HER2 status and histological subtype of the samples are indicated.

### DETAILED DESCRIPTION OF THE INVENTION

### I. Introduction

With the advent of the molecularly targeted therapy era in the field of oncology, many oncologists have experienced an unexpected dramatic response to molecularly targeted agents in refractory, metastatic cancer patients who would otherwise have been on supportive care. In the clinic, however, even patients selected based on their target profile often don't respond to targeting agents or develop resistance after having a dramatic initial response. Major issues which hinder the enhancement of the therapeutic benefit with targeted agents include: (1) concomitant and redundant pathway activation in subgroups of patients; (2) the development of resistance by the activation of alternate signaling events which bypass the originally targeted protein(s) via pathway cross-talk; (3) an alteration in molecular and pathologic features as cancer metastasizes and progresses over the course of anti-cancer treatment; (4) limited availability of re-biopsy during or after treatments; and (5) limitation of companion diagnostics for targeted agents to identify potential drug resistant mechanisms to address "evolving" disease.

Gastric cancer is the leading cause of cancer death worldwide, with an incidence of 18.9/100,000 per year and a mortality rate of 14.7/100,000 per year (*see*, *e.g.,* Cunningham et al., Ann. Oncol., 16 Suppl 1, i22-23 (2005)), and is the most common malignancy in Korea. Metastatic gastric cancer remains a therapeutic challenge for medical oncologists due to poor prognosis. Currently, trastuzumab is the only active targeted agent which has been proven to be efficacious for gastric cancer in a randomized phase III trial (*see, e.g.,* Bang, Y. J. et al., Lancet, 376:687-697 (2010)). Other known activated pathway proteins in gastric cancer include the human epidermal growth factor receptor (HER) family, mesenchymal epithelial transition factor or hepatocyte growth factor (HGF) receptor (cMET), phosphatidylinositol 3-kinase (PI3K), and insulin-like growth factor 1 receptor (IGF1R).

The present invention demonstrates that gastric cancers can be segregated based on the phosphorylation profiles of key growth factor receptor signaling pathways that are active in this cancer type. In particular, the present invention identifies a level of heterogeneity in gastric cancers based on signaling pathway activation profiles that will directly affect the selection and outcome of targeted therapeutics in this cancer type. In particular aspects, a highly sensitive and novel immunoassay, CEER™, has been used on gastric cancer clinical samples that can be multiplexed to specifically analyze total and phosphorylated forms of several proteins. The study described in Example 3 demonstrates that >40% of gastric cancer patients have at least one signal transduction kinase activated among the HER1, HER2, HER3, c-MET, PI3K, and IGF1R molecules. Among the RTKs analyzed in this study, HER family members were the most frequently activated tyrosine kinases, with HER3 activated in 28% of gastric cancers, followed by HER2 (21%) and HER1 (15.7%). In addition, the study described in Example 3 shows that HER2 positive gastric cancers primarily display activated HER2 and HER3, whereas HER2 negative cancers typically harbor active HER1, HER3 and c-MET pathways, and that HER2 activated gastric cancers are a distinct subgroup from the c-MET activated gastric cancers. Furthermore, the study described in Example 3 illustrates that the majority of gastric cancers (>75% of HER2 positive and ∼85% of HER2 negative) do not have any single active RTK, but rather that they are driven by networks of concomitantly activated RTKs.

Notably, the study described in Example 3 demonstrates that the overall signaling characteristics in p-HER1:p-cMET co-expressing gastric cancer samples are different from samples that express p-cMET without activated HER1. A significant difference in median survival times was observed for p-HER1(-):p-cMET(+) vs. p-HER1(+):p-cMET(+) sample sets. In fact, this study demonstrates that coactivation of cMET and HER1 showed worse disease-free survival when compared to stage I patients with cMET activation alone in a HER2-negative sample set. As such, this study demonstrates that coactivation of cMET and HER1 (and optionally HER3) is closely associated with HER2-negative, diffuse-type, gastric cancers, and that such gastric cancer patients have significantly poorer survival. Thus, this study illustrates that combinatorial therapeutic inhibition of both HER1 and cMET is a more effective anti-tumor strategy in HER1 and cMET coactivated cancer cells as opposed to monotherapy against either target alone.

### II. Definitions

As used herein, the following terms have the meanings ascribed to them unless specified otherwise.

The term "cancer" is intended to include any member of a class of diseases characterized by the uncontrolled growth of aberrant cells. The term includes all known cancers and neoplastic conditions, whether characterized as malignant, benign, soft tissue, or solid, and cancers of all stages and grades including pre- and post-metastatic cancers. Examples of different types of cancer include, but are not limited to, digestive and gastrointestinal cancers such as gastric cancer (*e.g*., stomach cancer), colorectal cancer, gastrointestinal stromal tumors (GIST), gastrointestinal carcinoid tumors, colon cancer, rectal cancer, anal cancer, bile duct cancer, small intestine cancer, and esophageal cancer; breast cancer; lung cancer (*e.g.*, non-small cell lung cancer); gallbladder cancer; liver cancer; pancreatic cancer; appendix cancer; prostate cancer, ovarian cancer; renal cancer (*e.g.*, renal cell carcinoma); cancer of the central nervous system; skin cancer; lymphomas; gliomas; choriocarcinomas; head and neck cancers; osteogenic sarcomas; and blood cancers. As used herein, a "tumor" comprises one or more cancerous cells. In certain embodiments, the gastric cancer corresponds to a histological subtype such as, for example, an intestinal-type, diffuse-type, and/or mixed-type.

The term "analyte" includes any molecule of interest, typically a macromolecule such as a polypeptide, whose presence, amount (expression level), activation state or level, and/or identity is determined. In certain embodiments, the analyte is a signal transduction molecule such as, *e.g.*, a component of a HER (EGFR) or c-Met signaling pathway.

The term "signal transduction molecule" or "signal transducer" includes proteins and other molecules that carry out the process by which a cell converts an extracellular signal or stimulus into a response, typically involving ordered sequences of biochemical reactions inside the cell. Examples of signal transduction molecules include, but are not limited to, receptor tyrosine kinases such as EGFR (*e.g.*, EGFR/HER1/ErbB1, HER2/Neu/ErbB2, HER3/ErbB3, HER4/ErbB4), VEGFR1/FLT1, VEGFR2/FLK1/KDR, VEGFR3/FLT4, FLT3/FLK2, PDGFR (*e.g.*, PDGFRA, PDGFRB), c-KIT/SCFR, INSR (insulin receptor), IGF-IR, IGF-IIR, IRR (insulin receptor-related receptor), CSF-1R, FGFR 1-4, HGFR 1-2, CCK4, TRK A-C, c-MET, RON, EPHA 1-8, EPHB 1-6, AXL, MER, TYRO3, TIE 1-2, TEK, RYK, DDR 1-2, RET, c-ROS, V-cadherin, LTK (leukocyte tyrosine kinase), ALK (anaplastic lymphoma kinase), ROR 1-2, MUSK, AATYK 1-3, and RTK 106; truncated forms of receptor tyrosine kinases such as truncated HER2 receptors with missing amino-terminal extracellular domains (*e.g.,* p95ErbB2 (p95m), p110, p95c, p95n, *etc.*); receptor tyrosine kinase dimers (*e.g.*, p95HER2/HER3, p95HER2/HER2, HER2/HER2, HER2/HER3, HER1/HER2, HER2/HER3, HER2/HER4, *etc.*); non-receptor tyrosine kinases such as BCR-ABL, Src, Frk, Btk, Csk, Abl, Zap70, Fes/Fps, Fak, Jak, Ack, and LIMK; tyrosine kinase signaling cascade components such as AKT (*e.g.*, AKT1, AKT2, AKT3), MEK (MAP2K1), ERK2 (MAPK1), ERK1 (MAPK3), PI3K (*e.g.,* PIK3CA (p110), PIK3R1 (p85)), PDK1, PDK2, phosphatase and tensin homolog (PTEN), SGK3, 4E-BP1, P70S6K (*e.g.*, p70 S6 kinase splice variant alpha I), protein tyrosine phosphatases (*e.g.*, PTP1B, PTPN13, BDP1, *etc.*), RAF, PLA2, MEKK, JNKK, JNK, p38, Shc (p66), Ras (*e.g.,* K-Ras, N-Ras, H-Ras), Rho, Rac1, Cdc42, PLC, PKC, p53, cyclin D1, STAT1, STAT3, phosphatidylinositol 4,5-bisphosphate (PIP2), phosphatidylinositol 3,4,5-trisphosphate (PIP3), mTOR, BAD, p21, p27, ROCK, IP3, TSP-1, NOS, GSK-3β, RSK 1-3, JNK, c-Jun, Rb, CREB, Ki67, and paxillin; nuclear hormone receptors such as estrogen receptor (ER), progesterone receptor (PR), androgen receptor, glucocorticoid receptor, mineralocorticoid receptor, vitamin A receptor, vitamin D receptor, retinoid receptor, thyroid hormone receptor, and orphan receptors; nuclear receptor coactivators and repressors such as amplified in breast cancer-1 (AIB 1) and nuclear receptor corepressor 1 (NCOR), respectively; and combinations thereof.

The term "component of a HER signaling pathway" includes any one or more of an upstream ligand of a HER kinase receptor, binding partner of a HER kinase receptor, and/or downstream effector molecule that is modulated through a HER kinase receptor. Examples of HER signaling pathway components include, without limitation, heregulin, HER1/ErbB1, HER2/ErbB2, HER3/ErbB3, HER4/ErbB4, AKT (*e.g.*, AKT1, AKT2, AKT3, *etc.*), MEK (MAP2K1), ERK2 (MAPK1), ERK1 (MAPK3), PI3K (*e.g.*, PIK3CA (p110), PIK3R1 (p85), *etc.*), PDK1, PDK2, PTEN, SGK3, 4E-BP1, P70S6K (*e.g.*, splice variant alpha I), protein tyrosine phosphatases (*e.g.,* PTP1B, PTPN13, BDP1, *etc.*), HER dimers (*e.g.,* HER1/HER1, HER1/p95HER2, HER1/HER2, HER1/HER3, HER1/HER4, HER2/HER2, HER2/p95HER2, HER2/HER3, HER2/HER4, HER3/HER3, HER3/p95HER2, HER3/HER4, HER4/HER4, HER4/p95HER2, *etc.*), GSK-3β, PIP2, PIP3, p27, and combinations thereof.

The term "component of a c-Met signaling pathway" includes any one or more of an upstream ligand of c-Met, binding partner of c-Met, and/or downstream effector molecule that is modulated through c-Met. Examples of c-Met signaling pathway components include, but are not limited to, hepatocyte growth factor/scatter factor (HGF/SF), Plexin B1, CD44v6, AKT (*e.g.*, AKT1, AKT2, AKT3), MEK (MAP2K1), ERK2 (MAPK1), ERK1 (MAPK3), STAT (*e.g.*, STAT1, STAT3), PI3K (*e.g.*, PIK3CA (p110), PIK3R1 (p85)), GRB2, Shc (p66), Ras (*e.g.*, K-Ras, N-Ras, H-Ras), GAB1, SHP2, SRC, GRB2, CRKL, PLCγ, PKC (*e.g.*, PKCα, PKCβ, PKCδ), paxillin, FAK, adducin, RB, RB1, PYK2, and combinations thereof.

The term "activation state" refers to whether a particular signal transduction molecule such as a HER or c-Met signaling pathway component is activated. Similarly, the term "activation level" refers to what extent a particular signal transduction molecule such as a HER or c-Met signaling pathway component is activated. The activation state typically corresponds to the phosphorylation, ubiquitination, and/or complexation status of one or more signal transduction molecules. Non-limiting examples of activation states (listed in parentheses) include: HER1/EGFR (EGFRvIII, phosphorylated (p-) EGFR, EGFR:Shc, ubiquitinated (u-) EGFR, p-EGFRvIII); ErbB2 (p-ErbB2, p95HER2 (truncated ErbB2), p-p95HER2, ErbB2:Shc, ErbB2:PI3K, ErbB2:EGFR, ErbB2:ErbB3, ErbB2:ErbB4); ErbB3 (p-ErbB3, ErbB3:PI3K, p-ErbB3:PI3K, ErbB3:Shc); ErbB4 (p-ErbB4, ErbB4:Shc); c-MET (p-c-MET, c-Met:HGF complex); AKT1 (p-AKT1); AKT2 (p-AKT2); AKT3 (p-AKT3); PTEN (p-PTEN); P70S6K (p-P70S6K); MEK (p-MEK); ERK1 (p-ERK1); ERK2 (p-ERK2); PDK1 (p-PDK1); PDK2 (p-PDK2); SGK3 (p-SGK3); 4E-BP1 (p-4E-BP1); PIK3R1 (p-PIK3R1); c-KIT (p-c-KIT); ER (p-ER); IGF-1R (p-IGF-1R, IGF-1R:IRS, IRS:PI3K, p-IRS, IGF-1R:PI3K); INSR (p-INSR); FLT3 (p-FLT3); HGFR1 (p-HGFR1); HGFR2 (p-HGFR2); RET (p-RET); PDGFRA (p-PDGFRA); PDGFRB (p-PDGFRB); VEGFR1 (p-VEGFR1, VEGFR1:PLCγ, VEGFR1:Src); VEGFR2 (p-VEGFR2, VEGFR2:PLCγ, VEGFR2:Src, VEGFR2:heparin sulphate, VEGFR2:VE-cadherin); VEGFR3 (p-VEGFR3); FGFR1 (p-FGFR1); FGFR2 (p-FGFR2); FGFR3 (p-FGFR3); FGFR4 (p-FGFR4); TIE1 (p-TIEl); TIE2 (p-TIE2); EPHA (p-EPHA); EPHB (p-EPHB); GSK-3β (p-GSK-3β); NFKB (p-NFKB), IKB (p-IKB, p-P65:IKB); BAD (p-BAD, BAD:14-3-3); mTOR (p-mTOR); Rsk-1 (p-Rsk-1); Jnk (p-Jnk); P38 (p-P38); STAT1 (p-STAT1); STAT3 (p-STAT3); FAK (p-FAK); RB (p-RB); Ki67; p53 (p-p53); CREB (p-CREB); c-Jun (p-c-Jun); c-Src (p-c-Src); paxillin (p-paxillin); GRB2 (p-GRB2), Shc (p-Shc), Ras (p-Ras), GAB1 (p-GAB1), SHP2 (p-SHP2), GRB2 (p-GRB2), CRKL (p-CRKL), PLCγ (p-PLCγ), PKC (*e.g.*, p-PKCα, p-PKCβ, p-PKCδ), adducin (p-adducin), RB1 (p-RB1), and PYK2 (p-PYK2).

The term "sample" as used herein includes any biological specimen obtained from a patient. Samples include, without limitation, whole blood, plasma, serum, red blood cells, white blood cells (*e.g.*, peripheral blood mononuclear cells), ductal lavage fluid, ascites, pleural efflux, nipple aspirate, lymph (*e.g.*, disseminated tumor cells of the lymph node), bone marrow aspirate, saliva, urine, stool (*i.e.*, feces), sputum, bronchial lavage fluid, tears, fine needle aspirate (*e.g.*, harvested by random periareolar fine needle aspiration), any other bodily fluid, a tissue sample (*e.g.*, tumor tissue) such as a biopsy of a tumor (*e.g.*, needle biopsy) or a lymph node *(e.g.*, sentinel lymph node biopsy), a tissue sample (*e.g.*, tumor tissue) such as a surgical resection of a tumor, and cellular extracts thereof. In some embodiments, the sample is whole blood or a fractional component thereof such as plasma, serum, or a cell pellet. In other embodiments, the sample is obtained by isolating circulating cells of a solid tumor from whole blood or a cellular fraction thereof using any technique known in the art. In yet other embodiments, the sample is a formalin fixed paraffin embedded (FFPE) tumor tissue sample, *e.g.*, from a solid tumor such as gastric cancer. In particular embodiments, the sample is a tumor lysate or extract prepared from frozen tissue obtained from a subject having gastric cancer.

A "biopsy" refers to the process of removing a tissue sample for diagnostic or prognostic evaluation, and to the tissue specimen itself. Any biopsy technique known in the art can be applied to the methods and compositions of the present invention. The biopsy technique applied will generally depend on the tissue type to be evaluated and the size and type of the tumor (*i.e.*, solid or suspended (*i.e.*, blood or ascites)), among other factors. Representative biopsy techniques include excisional biopsy, incisional biopsy, needle biopsy (*e.g.,* core needle biopsy, fine-needle aspiration biopsy, *etc.*), surgical biopsy, and bone marrow biopsy. Biopsy techniques are discussed, for example, in Harrison's Principles of Internal Medicine, Kasper, et al., eds., 16th ed., 2005, Chapter 70, and throughout Part V. One skilled in the art will appreciate that biopsy techniques can be performed to identify cancerous and/or precancerous cells in a given tissue sample.

The term "subject" or "patient" or "individual" typically includes humans, but can also include other animals such as, *e.g.*, other primates, rodents, canines, felines, equines, ovines, porcines, and the like.

As used herein, the term "dilution series" is intended to include a series of descending concentrations of a particular sample (*e.g.*, cell lysate) or reagent (*e.g.*, antibody). A dilution series is typically produced by a process of mixing a measured amount of a starting concentration of a sample or reagent with a diluent (*e.g.*, dilution buffer) to create a lower concentration of the sample or reagent, and repeating the process enough times to obtain the desired number of serial dilutions. The sample or reagent can be serially diluted at least 2, 3, 4, 5, 6, 7, 8, 9, 10, 15, 20, 25, 30, 35, 40, 45, 50, 100, 500, or 1000-fold to produce a dilution series comprising at least 2, 3, 4, 5, 6, 7, 8, 9, 10, 11, 12, 13, 14, 15, 16, 17, 18, 19, 20, 25, 30, 35, 40, 45, or 50 descending concentrations of the sample or reagent. For example, a dilution series comprising a 2-fold serial dilution of a capture antibody reagent at a 1 mg/ml starting concentration can be produced by mixing an amount of the starting concentration of capture antibody with an equal amount of a dilution buffer to create a 0.5 mg/ml concentration of the capture antibody, and repeating the process to obtain capture antibody concentrations of 0.25 mg/ml, 0.125 mg/ml, 0.0625 mg/ml, 0.0325 mg/ml, *etc.*

The term "superior dynamic range" as used herein refers to the ability of an assay to detect a specific analyte in as few as one cell or in as many as thousands of cells. For example, the immunoassays described herein possess superior dynamic range because they advantageously detect a particular signal transduction molecule of interest in about 1-10,000 cells (*e.g.*, about 1, 5, 10, 25, 50, 75, 100, 250, 500, 750, 1000, 2500, 5000, 7500, or 10,000 cells) using a dilution series of capture antibody concentrations.

An "array" or "microarray" comprises a distinct set and/or dilution series of capture antibodies immobilized or restrained on a solid support such as, for example, glass (*e.g.*, a glass slide), plastic, chips, pins, filters, beads (*e.g.*, magnetic beads, polystyrene beads, *etc.*), paper, membrane (*e.g.*, nylon, nitrocellulose, polyvinylidene fluoride (PVDF), *etc.*), fiber bundles, or any other suitable substrate. The capture antibodies are generally immobilized or restrained on the solid support via covalent or noncovalent interactions (*e.g.*, ionic bonds, hydrophobic interactions, hydrogen bonds, Van der Waals forces, dipole-dipole bonds). In certain instances, the capture antibodies comprise capture tags which interact with capture agents bound to the solid support. The arrays used in the assays described herein typically comprise a plurality of different capture antibodies and/or capture antibody concentrations that are coupled to the surface of a solid support in different known/addressable locations.

The term "capture antibody" is intended to include an immobilized antibody which is specific for (*i.e.*, binds, is bound by, or forms a complex with) one or more analytes of interest in a sample such as a cellular extract. In particular embodiments, the capture antibody is restrained on a solid support in an array. Suitable capture antibodies for immobilizing any of a variety of signal transduction molecules on a solid support are available from Upstate (Temecula, CA), Biosource (Camarillo, CA), Cell Signaling Technologies (Danvers, MA), R&D Systems (Minneapolis, MN), Lab Vision (Fremont, CA), Santa Cruz Biotechnology (Santa Cruz, CA), Sigma (St. Louis, MO), and BD Biosciences (San Jose, CA).

The term "detection antibody" as used herein includes an antibody comprising a detectable label which is specific for (*i.e.*, binds, is bound by, or forms a complex with) one or more analytes of interest in a sample. The term also encompasses an antibody which is specific for one or more analytes of interest, wherein the antibody can be bound by another species that comprises a detectable label. Examples of detectable labels include, but are not limited to, biotin/streptavidin labels, nucleic acid (*e.g.*, oligonucleotide) labels, chemically reactive labels, fluorescent labels, enzyme labels, radioactive labels, and combinations thereof. Suitable detection antibodies for detecting the activation state and/or total amount of any of a variety of signal transduction molecules are available from Upstate (Temecula, CA), Biosource (Camarillo, CA), Cell Signaling Technologies (Danvers, MA), R&D Systems (Minneapolis, MN), Lab Vision (Fremont, CA), Santa Cruz Biotechnology (Santa Cruz, CA), Sigma (St. Louis, MO), and BD Biosciences (San Jose, CA). As a non-limiting example, phospho-specific antibodies against various phosphorylated forms of signal transduction molecules such as EGFR, c-KIT, c-Src, FLK-1, PDGFRA, PDGFRB, AKT, MAPK, PTEN, Raf, and MEK are available from Santa Cruz Biotechnology.

The term "activation state-dependent antibody" includes a detection antibody which is specific for (*i.e.*, binds, is bound by, or forms a complex with) a particular activation state of one or more analytes of interest in a sample. In preferred embodiments, the activation state-dependent antibody detects the phosphorylation, ubiquitination, and/or complexation state of one or more analytes such as one or more signal transduction molecules. In some embodiments, the phosphorylation of members of the EGFR family of receptor tyrosine kinases and/or the formation of heterodimeric complexes between EGFR family members is detected using activation state-dependent antibodies. In particular embodiments, activation state-dependent antibodies are useful for detecting one or more sites of phosphorylation in one or more of the following signal transduction molecules (phosphorylation sites correspond to the position of the amino acid in the human protein sequence): EGFR/HER1/ErbB1 (*e.g.*, tyrosine (Y) 1068); ErbB2/HER2 (*e.g.*, Y1248); ErbB3/HER3 (*e.g.*, Y1289); ErbB4/HER4 (*e.g.*, Y1284); c-Met (*e.g.*, Y1003, Y1230, Y1234, Y1235, and/or Y1349); SGK3 (*e.g.*, threonine (T) 256 and/or serine (S) 422); 4E-BP1 (*e.g.,* T70); ERK1 (*e.g.*, T185, Y187, T202, and/or Y204); ERK2 (*e.g.*, T185, Y187, T202, and/or Y204); MEK (*e.g.*, S217 and/or S221); PIK3R1 (*e.g.*, Y688); PDK1 (*e.g.*, S241); P70S6K (*e.g.*, T229, T389, and/or S421); PTEN (*e.g.*, S380); AKT1 (*e.g.*, S473 and/or T308); AKT2 (*e.g.*, S474 and/or T309); AKT3 (*e.g.*, S472 and/or T305); GSK-3β (*e.g.*, S9); NFKB (*e.g.*, S536); IKB (*e.g.*, S32); BAD (*e.g.*, S112 and/or S136); mTOR (*e.g.*, S2448); Rsk-1 (*e.g.*, T357 and/or S363); Jnk (*e.g.*, T183 and/or Y185); P38 (*e.g.*, T180 and/or Y182); STAT3 (*e.g.*, Y705 and/or S727); FAK (*e.g.*, Y397, Y576, S722, Y861, and/or S910); RB (*e.g.*, S249, T252, S612, and/or S780); RB1 (*e.g.*, S780); adducin (*e.g.*, S662 and/or S724); PYK2 (*e.g.*, Y402 and/or Y881); PKCα (*e.g.*, S657); PKCα/β (*e.g.*, T368 and/or T641); PKCδ (*e.g.*, T505); p53 (*e.g.*, S392 and/or S20); CREB (*e.g.*, S133); c-Jun (*e.g.*, S63); c-Src (*e.g.*, Y416); and paxillin (*e.g.*, Y31 and/or Y118).

The term "activation state-independent antibody" includes a detection antibody which is specific for (*i. e.*, binds, is bound by, or forms a complex with) one or more analytes of interest in a sample irrespective of their activation state. For example, the activation state-independent antibody can detect both phosphorylated and unphosphorylated forms of one or more analytes such as one or more signal transduction molecules.

The term "incubating" is used synonymously with "contacting" and "exposing" and does not imply any specific time or temperature requirements unless otherwise indicated.

"Receptor tyrosine kinases" or "RTKs" include a family of fifty-six (56) proteins characterized by a transmembrane domain and a tyrosine kinase motif. RTKs function in cell signaling and transmit signals regulating growth, differentiation, adhesion, migration, and apoptosis. The mutational activation and/or overexpression of receptor tyrosine kinases transforms cells and often plays a crucial role in the development and propagation of cancers. RTKs have become targets of various molecularly targeted agents such as trastuzumab, cetuximab, gefitinib, erlotinib, sunitinib, imatinib, nilotinib, and the like. One well-characterized signal transduction pathway is the MAP kinase pathway, which is responsible for transducing the signal from epidermal growth factor (EGF) to the promotion of cell proliferation in cells.

The term "disease-free survival" includes the length of time after treatment for a specific disease (*e.g.*, cancer) during which a patient survives with no sign of the disease (*e.g.*, without known recurrence). In certain embodiments, disease-free survival is a clinical parameter used to evaluate the efficacy of a particular therapy, which is usually measured in units of 1 or 5 years.

The term "progression-free survival" includes the length of time during and after treatment for a specific disease (*e.g.*, cancer) in which a patient is living with the disease without additional symptoms of the disease.

The term "overall survival" includes the clinical endpoint describing patients who are alive for a defined period of time after being diagnosed with or treated for a disease, such as cancer.

### III. Description of the Embodiments

The present invention provides assays and methods for predicting the survival of a subject having an early stage (*e.g.*, stage I or II or transition in-between stages) gastric cancer after tumor surgery. In certain aspects, the present invention provides assays and methods for predicting therapeutic efficacy and/or response to combination therapy in a subject having an early stage gastric cancer. The present invention further provides methods for treating a subject having an early stage gastric cancer by administering a combination therapy tailored to the signal transduction biomarkers that are activated in the gastric cancer. In particular embodiments, the methods of the present invention rely on the detection of the activation state or level of a specific combination of signal transducer analytes in a cancer cell obtained from a subject having an early stage gastric cancer. Thus, the methods described herein are particularly useful for predicting the survival or prognosis of a subject having an early stage gastric cancer and for guiding treatment decisions both pre-tumor and post-tumor surgery by identifying subjects who would benefit from combination therapy as opposed to monotherapy in view of the activation state or level detected for the analytes.

In certain aspects, the present invention provides a method for predicting the survival of a subject having stage I or II gastric cancer after tumor surgery, the method comprising:
(a) determining the presence or level of activation of at least two analytes comprising cMET and HER1 in a cancer cell obtained from the subject; and
(b) predicting the survival of the subject based upon the presence or level of activation of the at least two analytes determined in step (a).

In some embodiments, the method predicts the disease-free survival or progression-free survival of a subject having stage I or II gastric cancer. In certain instances, the stage I gastric cancer is a stage IA or stage IB gastric cancer. In certain other instances, the stage II gastric cancer is a stage IIA or stage IIB gastric cancer. In one alternative embodiment, the subject has a stage 0 gastric cancer. In another alternative embodiment, the subject has a late stage *(e.g.,* stage III such as stage IIIA, IIIB, or IIIC, or stage IV) gastric cancer. *See, e.g.,* Washington, Ann. Surg. Oncol., 17:3077-9 (2010) for a description of the 7th Edition of the AJCC gastric cancer staging system.

In particular embodiments, cMET and HER1 are coactivated in the cancer cell. In such embodiments, the subject is predicted to have worse survival (*e.g.*, poorer prognosis) compared to stage I or II gastric cancer subjects with cMET activation in the absence of HER1 activation. Non-limiting examples of worse survival outcomes include a shorter life span, a shorter time to cancer recurrence, a shorter time to developing symptoms of cancer, a higher risk of relapse, and combinations thereof.

In some embodiments, the stage I or II gastric cancer corresponds to a histological subtype such as, for example, an intestinal-type, diffuse-type, or mixed-type gastric cancer. In particular embodiments, the stage I or II gastric cancer is a diffuse-type gastric cancer. In other embodiments, cMET and HER1 are coactivated in the diffuse-type gastric cancer. In such embodiments, the subject is predicted to have worse survival (*e.g.*, poorer prognosis) compared to stage I or II gastric cancer subjects with cMET activation but without HER1 activation and/or a diffuse-type gastric cancer.

In other embodiments, step (a) further comprises determining the presence or level of activation of at least one additional analyte comprising HER2 and/or HER3 in the cancer cell. In certain instances, cMET and HER1 are coactivated but HER2 is not activated in the cancer cell. In such instances, the subject is predicted to have worse survival (*e.g.*, poorer prognosis) compared to stage I or II gastric cancer subjects with cMET activation in the absence of HER1 and/or HER2 activation. In certain other instances, cMET, HER1, and HER3 are coactivated but HER2 is not activated in the cancer cell. In such instances, the subject is predicted to have worse survival (*e.g.*, poorer prognosis) compared to stage I or II gastric cancer subjects with cMET activation in the absence of HER1, HER2, and/or HER3 activation.

In some embodiments, a worse survival outcome for the subject having stage I or II gastric cancer includes a life span, disease-free survival, or progression-free survival that is shorter by days, weeks, months, or years (*e.g.*, at least 1, 2, 3, 4, 5, 6, 7, 8, 9, 10, 11, 12, 13, 14, 15, 16, 17, 18, 19, 20, 25, 30, 35, 40, 45, or 50 days, weeks, months, or years) compared to a control subject (*e.g.*, a stage I or II gastric cancer subject with cMET activation but not HER1 activation).

In certain embodiments, the cancer cell is isolated from a tumor tissue sample such as a primary gastric tumor sample, *e.g.*, a stage I or II gastric tumor. In some instances, the cancer cell is selected from the group consisting of a primary tumor cell, a circulating tumor cell (CTC), an ascites tumor cell (ATC), and combinations thereof. In particular instances, a cellular extract is produced from the isolated cancer cell.

In certain embodiments, the method further comprises:
(c) administering a cMET inhibitor alone or a cMET inhibitor in combination with a HER1 inhibitor to the subject based upon the presence or level of activation of the at least cMET and HER1 determined in step (a).

In some embodiments, a cMET inhibitor is administered in combination with a HER1 inhibitor when cMET and HER1 are coactivated in the cancer cell, and optionally when HER2 is not activated and/or HER3 is activated in the cancer cell. Non-limiting examples of cMET inhibitors and HER1 inhibitors are described herein. In particular embodiments, the cMET inhibitor(s) foretinib and/or PHA-665752 is administered in combination with the HER1 inhibitor lapatinib. In further embodiments, one or more additional anticancer drugs are administered to the subject. Non-limiting examples of suitable additional anticancer drugs are described below.

In particular embodiments, step (a) comprises determining the presence or level of activation of the analytes (*e.g.*, at least cMET and HER1 and optionally HER2 and/or HER3 as well as other signal transduction biomarkers) with CEER™. In other embodiments, step (a) further comprises determining the presence or level of expression of one or more analytes (*e.g.*, at least cMET and HER1 and optionally HER2 and/or HER3 as well as other signal transduction biomarkers). In particular embodiments, the presence or level of expression of such analytes is determined with CEER™. In certain embodiments, the expression level and/or activation level of the analytes is calibrated against a standard curve generated for each of the analytes.

In certain instances, the methods of the present invention may further comprise a step of providing the result of the prediction in step (b) to a user (*e.g.*, a clinician such as an oncologist or a general practitioner) in a readable format. In some instances, the method may further comprise sending or reporting the result of the prediction in step (b) to a clinician, *e.g.*, an oncologist or a general practitioner. In other instances, the method may further comprise recording or storing the result of the prediction in step (b) in a computer database or other suitable machine or device for storing information, *e.g.*, at a laboratory.

In further aspects, the present invention provides a method for treating a subject having stage I or II gastric cancer, the method comprising:
administering a combination therapy comprising a cMET inhibitor and a HER1 inhibitor to the subject, wherein cMET and HER1 are coactivated in the stage I or II gastric cancer.

In some embodiments, the method improves the prognosis (*e.g.*, increases disease-free survival or progression-free survival) of a subject having stage I or II gastric cancer. In certain instances, the stage I gastric cancer is a stage IA or stage IB gastric cancer. In certain other instances, the stage II gastric cancer is a stage IIA or stage IIB gastric cancer. In one alternative embodiment, the subject has a stage 0 gastric cancer. In another alternative embodiment, the subject has a late stage (*e.g.*, stage III such as stage IIIA, IIIB, or IIIC, or stage IV) gastric cancer. *See, e.g.,* Washington, Ann. Surg. Oncol., 17:3077-9 (2010) for a description of the 7th Edition of the AJCC gastric cancer staging system.

In certain embodiments, the method increases the survival (*e.g.*, increases disease-free survival or progression-free survival) of the subject having stage I or II gastric cancer by days, weeks, months, or years (*e.g.*, at least 1, 2, 3, 4, 5, 6, 7, 8, 9, 10, 11, 12, 13, 14, 15, 16, 17, 18, 19, 20, 25, 30, 35, 40, 45, or 50 days, weeks, months, or years) compared to a control subject having stage I or II gastric cancer with cMET and HER1 coactivation not receiving the combination therapy.

In other embodiments, the subject (*e.g.*, prior to starting the combination therapy) has worse survival and/or poorer prognosis compared to stage I or II gastric cancer subjects with cMet activation in the absence of HER1 activation. Non-limiting examples of worse survival outcomes include a shorter life span, a shorter time to cancer recurrence, a shorter time to developing symptoms of cancer, a higher risk of relapse, and combinations thereof.

In further embodiments, cMET and HER1 are coactivated but HER2 is not activated in the stage I or II gastric cancer. In such embodiments, the subject (*e.g.*, prior to starting the combination therapy) has worse survival and/or poorer prognosis compared to stage I or II gastric cancer subjects with cMet activation in the absence of HER1 and/or HER2 activation.

In yet other embodiments, cMET, HER1, and HER3 are coactivated in the stage I or II gastric cancer. In such embodiments, the subject (*e.g.*, prior to starting the combination therapy) has worse survival and/or poorer prognosis compared to stage I or II gastric cancer subjects with cMET activation in the absence of HER1, HER2, and/or HER3 activation.

In some embodiments, a worse survival outcome for the subject having stage I or II gastric cancer includes a life span, disease-free survival, or progression-free survival that is shorter by days, weeks, months, or years (*e.g.*, at least 1, 2, 3, 4, 5, 6, 7, 8, 9, 10, 11, 12, 13, 14, 15, 16, 17, 18, 19, 20, 25, 30, 35, 40, 45, or 50 days, weeks, months, or years) compared to a control subject (*e.g.*, a stage I or II gastric cancer subject with cMET activation but not HER1 activation).

In other embodiments, the combination therapy is administered to the subject as primary therapy (*e.g.*, as pre-operative treatment to shrink the size of a gastric tumor). In other embodiments, the combination therapy is administered to the subject as post-operative adjuvant therapy (*e.g.*, after tumor surgery). In particular embodiments, the combination therapy can be administered both pre-operatively as primary therapy and post-operatively as adjuvant therapy. In certain instances, therapeutically effective amounts of a cMET inhibitor and a HER1 inhibitor are administered to the subject.

In certain embodiments, the cMET inhibitor is selected from the group consisting of foretinib (GSK1363089/XL-880), PHA-665752, AMG102, MetMab™, ARQ197, JNJ-38877605, PF-2341066, PF-04217903, SGX523, XL184, MGCD265, MK-2461, and combinations thereof. In certain other embodiments, the HER1 inhibitor is selected from the group consisting of erlotinib, lapatinib, gefitinib, BIBW-2992, and combinations thereof. In particular embodiments, the combination therapy comprises the cMET inhibitor(s) foretinib and/or PHA-665752 and the HER1 inhibitor lapatinib.

In other embodiments, the methods of the present invention further comprise the administration of one or more additional anticancer drugs to the subject. In particular embodiments, the anticancer drug comprises an agent that interferes with the function of activated signal transduction pathway components in cancer cells including, but not limited to, anti-signaling agents (*i.e.*, a cytostatic drugs) such as a monoclonal antibody or a tyrosine kinase inhibitor; anti-proliferative agents; chemotherapeutic agents (*i.e.*, a cytotoxic drugs); hormonal therapeutic agents; radiotherapeutic agents; vaccines; and/or any other compound with the ability to reduce or abrogate the uncontrolled growth of aberrant cells such as cancerous cells. In preferred embodiments, one or more of the additional anticancer drugs are selected from the group consisting of pan-HER inhibitors, HER3 inhibitors, HER1/2 inhibitors, HER1/2/4 inhibitors, IGF-1R inhibitors, MEK inhibitors, PI3K inhibitors, mTOR inhibitors, and combinations thereof.

Examples of anti-signaling agents suitable for use in the present invention include, without limitation, monoclonal antibodies such as trastuzumab (Herceptin^{®}), pertuzumab (2C4), alemtuzumab (Campath^{®}), bevacizumab (Avastin^{®}), cetuximab (Erbitux^{®}), gemtuzumab (Mylotarg^{®}), panitumumab (Vectibix™), rituximab (Rituxan^{®}), and tositumomab (BEXXAR^{®}); tyrosine kinase inhibitors such as gefitinib (Iressa^{®}), sunitinib (Sutent^{®}), erlotinib (Tarceva^{®}), lapatinib (GW-572016; Tykerb^{®}), canertinib (CI 1033), semaxinib (SU5416), vatalanib (PTK787/ZK222584), sorafenib (BAY 43-9006; Nexavar^{®}), imatinib mesylate (Gleevec^{®}), leflunomide (SU101), vandetanib (ZACTIMA™; ZD6474), pelitinib (EKB-569), CP-654577, CP-724714, HKI-272, PKI-166, AEE788, BMS-599626, HKI-357, BIBW-2992, ARRY-334543, JNJ-26483327, and combinations thereof.

Exemplary anti-proliferative agents include mTOR inhibitors such as sirolimus (rapamycin), temsirolimus (CCI-779), everolimus (RAD001), BEZ-235, and XL765; AKT inhibitors such as 1L6-hydroxymethyl-chiro-inositol-2-(R)-2-O-methyl-3-O-octadecyl-*sn-*glycerocarbonate, 9-methoxy-2-methylellipticinium acetate, 1,3-dihydro-1-(1-((4-(6-phenyl-1H-imidazo[4,5-g]quinoxalin-7-yl)phenyl)methyl)-4-piperidinyl)-2H-benzimidazol-2-one, 10-(4'-(N-diethylamino)butyl)-2-chlorophenoxazine, 3-formylchromone thiosemicarbazone (Cu(II)Cl₂ complex), API-2, a 15-mer peptide derived from amino acids 10-24 of the proto-oncogene TCL1 (Hiromura et al., J. Biol. Chem., 279:53407-53418 (2004), KP372-1, and the compounds described in Kozikowski et al., J. Am. Chem. Soc., 125:1144-1145 (2003) and Kau et al., Cancer Cell, 4:463-476 (2003); PI3K inhibitors such as PX-866, wortmannin, LY 294002, quercetin, tetrodotoxin citrate, thioperamide maleate, GDC-0941 (957054-30-7), IC87114, PI-103, PIK93, BEZ-235 (NVP-BEZ235), TGX-115, ZSTK474, (-)-deguelin, NU 7026, myricetin, tandutinib, GDC-0941 bismesylate, GSK690693, KU-55933, MK-2206, OSU-03012, perifosine, triciribine, XL-147, PIK75, TGX-221, NU 7441, PI 828, XL-765, and WHI-P 154; MEK inhibitors such as PD98059, ARRY-162, RDEA119, U0126, GDC-0973, PD184161, AZD6244, AZD8330, PD-0325901, and ARRY-142886; IGF-1R inhibitors such as BMS-536924; and combinations thereof.

Non-limiting examples of pan-HER inhibitors include PF-00299804, neratinib (HKI-272), AC480 (BMS-599626), BMS-690154, PF-02341066, HM781-36B, CI-1033, BIBW-2992, and combinations thereof.

Non-limiting examples of chemotherapeutic agents include platinum-based drugs (*e.g.*, oxaliplatin, cisplatin, carboplatin, spiroplatin, iproplatin, satraplatin, *etc.),* alkylating agents (*e.g.*, cyclophosphamide, ifosfamide, chlorambucil, busulfan, melphalan, mechlorethamine, uramustine, thiotepa, nitrosoureas, *etc.*), anti-metabolites (*e.g.,* 5-fluorouracil, 5-fluorocytosine, azathioprine, 6-mercaptopurine, methotrexate, leucovorin, capecitabine, cytarabine, floxuridine, fludarabine, gemcitabine (Gemzar^{®}), pemetrexed (ALIMTA^{®}), raltitrexed, *etc.),* plant alkaloids (*e.g.*, vincristine, vinblastine, vinorelbine, vindesine, podophyllotoxin, paclitaxel (Taxol^{®}), docetaxel (Taxotere^{®}), *etc.*), topoisomerase inhibitors (e.g., irinotecan, topotecan, amsacrine, etoposide (VP16), etoposide phosphate, teniposide, *etc.*), antitumor antibiotics (*e.g.*, doxorubicin, adriamycin, daunorubicin, epirubicin, actinomycin, bleomycin, mitomycin, mitoxantrone, plicamycin, *etc.*), pharmaceutically acceptable salts thereof, stereoisomers thereof, derivatives thereof, analogs thereof, and combinations thereof.

Examples of hormonal therapeutic agents include, without limitation, aromatase inhibitors (*e.g.*, aminoglutethimide, anastrozole (Arimidex^{®}), letrozole (Femara^{®}), vorozole, exemestane (Aromasin^{®}), 4-androstene-3,6,17-trione (6-OXO), 1,4,6-androstatrien-3,17-dione (ATD), formestane (Lentaron^{®}), *etc.*), selective estrogen receptor modulators (*e.g.*, bazedoxifene, clomifene, fulvestrant, lasofoxifene, raloxifene, tamoxifen, toremifene, *etc.*), steroids (*e.g.*, dexamethasone), finasteride, and gonadotropin-releasing hormone agonists (GnRH) such as goserelin, pharmaceutically acceptable salts thereof, stereoisomers thereof, derivatives thereof, analogs thereof, and combinations thereof.

Non-limiting examples of cancer vaccines useful in the present invention include ANYARA from Active Biotech, DCVax-LB from Northwest Biotherapeutics, EP-2101 from IDM Pharma, GV1001 from Pharmexa, IO-2055 from Idera Pharmaceuticals, INGN 225 from Introgen Therapeutics and Stimuvax from Biomira/Merck.

Examples of radiotherapeutic agents include, but are not limited to, radionuclides such as ⁴⁷Sc, ⁶⁴Cu, ⁶⁷Cu, ⁸⁹Sr, ⁸⁶Y, ⁸⁷Y, ⁹⁰Y, ¹⁰⁵Rh, ¹¹¹Ag, ¹¹¹In, ^{117m}Sn, ¹⁴⁹Pm, ¹⁵³Sm, ¹⁶⁶Ho, ¹⁷⁷Lu, ¹⁸⁶Re, ¹⁸⁸Re, ²¹¹At, and ²¹²Bi, optionally conjugated to antibodies directed against tumor antigens.

In certain embodiments, one or more additional anticancer drugs include lapatinib for HER1/2, gefitinib for HER1/2/4, BIBW-2992 for HER1/2, BMS-536924 for IGF-1R, PD-325901 for MEK, BEZ-235 for PI3K and mTOR, and combinations thereof.

In particular embodiments, the presence or level of activation of analytes such as cMET and HER1 and optionally HER2 and/or HER3 (as well as other signal transduction biomarkers) is determined in a cancer cell obtained from the subject. In some instances, the cancer cell is selected from the group consisting of a primary tumor cell, a circulating tumor cell (CTC), an ascites tumor cell (ATC), and combinations thereof.

In certain embodiments, the method further comprises determining the presence or level of activation of analytes such as cMET and HER1 and optionally HER2 and/or HER3 (as well as other signal transduction biomarkers) in a cancer cell obtained from the subject prior to administering the combination therapy, *e.g.*, to determine whether cMET and HER1 are coactivated in the stage I or II gastric cancer. In particular embodiments, the presence or level of activation of such analytes (*e.g.*, at least cMET and HER1, optionally HER2 and/or HER3) is determined with CEER™.

In certain embodiments, the method further comprises determining the presence or level of expression of analytes such as cMET and HER1 and optionally HER2 and/or HER3 (as well as other signal transduction biomarkers) in a cancer cell obtained from the subject prior to administering the combination therapy, *e.g.,* to determine whether cMET and HER1 are coexpressed in the stage I or II gastric cancer. In particular embodiments, the presence or level of expression of such analytes (*e.g.*, at least cMET and HER1, optionally HER2 and/or HER3) is determined with CEER™. In certain other embodiments, the expression level and/or activation level of the analytes is calibrated against a standard curve generated for each of the analytes.

In some embodiments, the expression level and/or activation level of a particular analyte of interest is expressed as a relative fluorescence unit (RFU) value that corresponds to the signal intensity for the analyte that is determined using, *e.g.*, a proximity assay such as CEER™. In other embodiments, the expression level and/or activation level of a particular analyte of interest is expressed as "-", "±", "+", "++", "+++", or "++++" that corresponds to increasing signal intensity for the analyte that is determined using, *e.g.*, a proximity assay such as CEER™. In some instances, an undetectable or minimally detectable level of expression and/or activation of a particular analyte of interest that is determined using, *e.g.*, a proximity assay such as CEER™, may be expressed as "-" or "±". In other instances, a low level of expression and/or activation of a particular analyte of interest that is determined using, *e.g.*, a proximity assay such as CEER™, may be expressed as "+". In still yet other instances, a moderate level of expression and/or activation of a particular analyte of interest that is determined using, *e.g.*, a proximity assay such as CEER™, may be expressed as "++". In further instances, a high level of expression and/or activation of a particular analyte of interest that is determined using, *e.g.*, a proximity assay such as CEER™, may be expressed as "+++". In other instances, a very high level of expression and/or activation of a particular analyte of interest that is determined using, *e.g.*, a proximity assay such as CEER™, may be expressed as "++++".

In other embodiments, the expression level and/or activation level of a particular analyte of interest is quantitated by calibrating or normalizing the RFU value determined using, *e.g.*, a proximity assay such as CEER™, against a standard curve generated for the analyte. In certain instances, a computed units (CU) value can be calculated based upon the standard curve. In certain other instances, the CU value can be expressed as "-", "±", "+", "++", "+++", or "++++" in accordance with the description above for the signal intensity of a particular analyte of interest. Example 3 ofUS 20120231965 provides a non-limiting example of data analysis for the quantitation of signal transduction pathway proteins in cells such as cancer cells.

In particular embodiments, the CU value for a particular analyte of interest can be calculated and compared to a cut-off value to determine whether a cell or sample is positive or negative for that analyte. In certain instances, the cut-off value for an analyte of interest, when expressed as a CU value, can be at least about 1, 2, 3, 4, 5, 6, 7, 8, 9, 10, 15, 20, 25, 30, 35, 40, 45, 50, 55, 60, 65, 70, 75, 80, 85, 90, 95, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, 10000, 15000, 20000, 25000, 30000, 35000, 40000, 45000, 50000, 55000, 60000, 65000, 70000, 75000, 80000, 85000, 90000, 95000, 100000, 500000, or more CU. In particular embodiments, 1 CU represents the expression of about 1-2×10⁶ RTKs and/or about 1-2×10⁵ activated (*e.g.*, phosphorylated) RTKs (*see,* Example 3 below).

In particular embodiments, a CU value for an analyte of interest that is equal to or greater than the cut-off value indicates that a cell or sample (*e.g.*, cellular extract) is positive for that analyte, *e.g.,* the analyte is overexpressed and/or activated (*e.g.*, phosphorylated) in the cell or sample. As a non-limiting example, a cut-off value of about 500 CU can be used to score for p95HER2 positivity. As another non-limiting example, a cut-off value of about 6 or 7 (*e.g.,* 6.7 CU) can be used to score for the presence of phosphorylated HER1 (p-HER1). As yet another non-limiting example, a cut-off value of about 41 or 42 (*e.g.*, 41.6 CU) can be used to score for the presence of phosphorylated HER2 (p-HER2). As another non-limiting example, a cut-off value of about 1124 or 1125 (*e.g.*, 1124.3 CU) can be used to score for the presence of phosphorylated HER3 (p-HER3). As another non-limiting example, a cut-off value of about 6 or 7 (*e.g.*, 6.1 CU) can be used to score for the presence of phosphorylated cMET (p-cMET). As yet another non-limiting example, a cut-off value of about 69 or 70 (*e.g.,* 69.2 CU) can be used to score for the presence of phosphorylated IGF1R (p-IGF1R). As another non-limiting example, a cut-off value of about 175 or 176 CU (*e.g.*, 175.3 CU) can be used to score for the presence of phosphorylated PI3K (p-PI3K). Figure 15 (Table 2) shows the use of the exemplary cut-off values described herein for determining whether a particular sample is positive for p95HER2 expression and/or HER1, HER2, HER3, cMET, IGF1R and/or PI3K phosphorylation.

In certain embodiments, the cut-off value is relative to a specific amount of sample analyzed, *e.g.*, particular cut-off values can be determined based upon the quantity of sample analyzed by a proximity assay such as CEER™ (*e.g.*, at least about 1, 5, 10, 15, 20, 25 30, 35, 40, 45, 50, 100, or more µg of tissue analyzed by CEER™). As a non-limiting example, a cut-off value of about 500 CU can be used to score for p95HER2 positivity per a specific amount (*e.g.*, 20 µg) of tissue analyzed (*see*, Example 3 below).

To preserve the *in situ* activation states, signal transduction proteins are typically extracted shortly after the cells are isolated, preferably within 96, 72, 48, 24, 6, or 1 hr, more preferably within 30, 15, or 5 minutes. The isolated cells may also be incubated with growth factors usually at nanomolar to micromolar concentrations for about 1-30 minutes to resuscitate or stimulate signal transducer activation (*see*, *e.g.*, Irish et al., Cell, 118:217-228 (2004)). Stimulatory growth factors include epidermal growth factor (EGF), heregulin (HRG), TGF-α, PIGF, angiopoietin (Ang), NRG1, PGF, TNF-α, VEGF, PDGF, IGF, FGF, HGF, cytokines, and the like. To evaluate potential anticancer therapies for an individual patient, the isolated cells can be incubated with one or more anticancer drugs of varying doses prior to, during, and/or after growth factor stimulation. Growth factor stimulation can be performed for a few minutes or hours (*e.g.*, about 1-5 minutes to about 1-6 hours). After isolation, treatment with the anticancer drug, and/or growth factor stimulation, the cells are lysed to extract the signal transduction proteins using any technique known in the art. Preferably, the cell lysis is initiated between about 1-360 minutes after growth factor stimulation, and more preferably at two different time intervals: (1) at about 1-5 minutes after growth factor stimulation; and (2) between about 30-180 minutes after growth factor stimulation. Alternatively, the lysate can be stored at -80°C until use.

In certain embodiments, the present invention further comprises determining the expression level (*e.g.*, total amount) and/or activation level (*e.g.*, level of phosphorylation or complex formation) of one or more (*e.g.*, at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, or more) additional analytes in a cancer cell.

Non-limiting examples of additional analytes such as signal transduction molecules that can be interrogated in a sample such as a cellular extract from a cancer cell include, without limitation, receptor tyrosine kinases, non-receptor tyrosine kinases, tyrosine kinase signaling cascade components, nuclear hormone receptors, nuclear receptor coactivators, nuclear receptor repressors, and combinations thereof. In certain instances, the presence or level of expression and/or activation of one or more of the following additional analytes can be determined in a sample such as a cellular extract from a cancer cell: p95HER2, HER4 (ErbB4), PI3K, SHC, Raf, SRC, MEK, NFkB-IkB, mTOR, PI3K (*e.g.,* PIK3CA and/or PIK3R1), VEGF, VEGFR1, VEGFR2, VEGFR3, EPH-A, EPH-B, EPH-C, EPH-D, FGFR, c-KIT, FLT-3, TIE-1, TIE-2, c-FMS, PDGFRA, PDGFRB, Abl, FTL 3, RET, HGFR, FGFR1, FGFR2, FGFR3, FGFR4, IGF-1R, ER, PR, NCOR, AIB1, AKT, ERK2 (MAPK1), ERK1 (MAPK3), PDK1, PDK2, PTEN, SGK3, 4E-BP1, P70S6K, protein tyrosine phosphatases (*e.g.*, PTP1B, PTPN13, BDP1, *etc.*), receptor dimers, GSK-3β, PIP2, PIP3, p27, and combinations thereof.

In one particular embodiment, the present invention comprises determining the expression level (*e.g.,* total amount) and/or activation level (*e.g.,* level of phosphorylation or complex formation) of at least one, two, three, four, five, six, seven, eight, nine, ten, or more markers such as the following analytes: HER1, HER2, HER3, p95HER2, cMET, IGF-1R, cKIT, PI3K (*e.g.*, PIK3CA and/or PIK3R1), SHC, and/or VEGFR (*e.g.*, VEGFR1, 2, and/or 3).

In certain preferred embodiments, the present invention comprises (i) determining the expression level of at least one or more of HER1, HER2, HER3, cMET, IGF-1R, PI3K, and/or SHC and/or (ii) determining the activation level of at least one or more of HER1, HER2, HER3, cMET, IGF-1R, PI3K, and/or SHC. In some embodiments, the activation level corresponds to a level of phosphorylation of HER1, HER2, HER3, cMET, IGF-1R, and/or SHC. In certain other embodiments, the activation level corresponds to a level of a PI3K complex. Examples of PI3K complexes include, without limitation, one or more complexes comprising a dimerized receptor tyrosine kinase pair, a PI3K p85 subunit (*e.g.*, PIK3R1), and a PI3K p110 subunit (*e.g.*, an α or β subunit such as PIK3CA or PIK3CB); *see, e.g.*, US2015-0017659.

In some embodiments, determining the expression level of the one or more analytes comprises detecting the total amount of each of the one or more analytes in the cellular extract with one or more antibodies specific for the corresponding analyte. In particular embodiments, the antibodies bind to the analyte irrespective of the activation state of the analyte to be detected, *i.e.*, the antibodies detect both the non-activated and activated forms of the analyte.

Total expression level and/or status can be determined using any of a variety of techniques. In certain embodiments, the total expression level and/or status of one or more analytes of interest such as signal transduction molecules in a sample such as a cellular extract from a cell line (*e.g.*, gastric cancer cell line) or tumor tissue (*e.g.*, gastric tumor tissue) is detected with an immunoassay such as a single detection assay or a proximity dual detection assay (*e.g.*, a Collaborative Enzyme Enhanced Reactive Immunoassay (CEER™)) as described herein.

In certain embodiments, determining the expression (*e.g.*, total) levels of one or more analytes comprises:
(i) incubating (*e.g.*, contacting) a cellular extract produced from a cell with one or a plurality of dilution series of capture antibodies (*e.g.*, capture antibodies specific for one or more analytes) to form a plurality of captured analytes, wherein the capture antibodies are restrained on a solid support (*e.g.*, to transform the analytes present in the cellular extract into complexes of captured analytes comprising the analytes and capture antibodies);
(ii) incubating (*e.g.*, contacting) the plurality of captured analytes with detection antibodies comprising one or a plurality of first and second activation state-independent antibodies specific for the corresponding analytes (*e.g.*, first and second activation state-independent antibodies specific for the one or more analytes) to form a plurality of detectable captured analytes (*e.g.*, to transform the complexes of captured analytes into complexes of detectable captured analytes comprising the captured analytes and detection antibodies),
   wherein the first activation state-independent antibodies are labeled with a facilitating moiety, the second activation state-independent antibodies are labeled with a first member of a signal amplification pair, and the facilitating moiety generates an oxidizing agent which channels to and reacts with the first member of the signal amplification pair;
(iii) incubating (*e.g.*, contacting) the plurality of detectable captured analytes with a second member of the signal amplification pair to generate an amplified signal; and
(iv) detecting the amplified signal generated from the first and second members of the signal amplification pair.

In certain other embodiments, determining the expression (*e.g.*, total) levels of one or more analytes that are truncated receptors (*e.g.*, p95HER2) comprises:
(i) incubating (*e.g.*, contacting) a cellular extract produced from a cell with a plurality of beads specific for an extracellular domain (ECD) binding region of a full-length receptor (*e.g.*, full-length HER2);
(ii) removing the plurality of beads from the cellular extract, thereby removing the full-length receptor (*e.g.*, full-length HER2) to form a cellular extract devoid of the full-length receptor (*e.g.*, full-length HER2) (*e.g.*, to transform the cellular extract into a cellular extract devoid of a specific full-length receptor or family of full-length receptors);
(iii) incubating (*e.g.*, contacting) the cellular extract devoid of the full-length receptor (*e.g.*, full-length HER2) with one or a plurality of capture antibodies specific for an intracellular domain (ICD) binding region of the full-length receptor (*e.g.*, full-length HER2) to form a plurality of captured truncated receptors, wherein the capture antibodies are restrained on a solid support (*e.g.*, to transform the truncated receptors present in a full-length receptor-depleted cellular extract into complexes of truncated receptors and capture antibodies);
(iv) incubating the plurality of captured truncated receptors with detection antibodies comprising one or a plurality of first and second activation state-independent antibodies specific for an ICD binding region of the full-length receptor (*e.g.*, full-length HER2) to form a plurality of detectable captured truncated receptors (*e.g.,* to transform the complexes of captured truncated receptors into complexes of detectable captured truncated receptors comprising the captured truncated receptors and detection antibodies),
   wherein the first activation state-independent antibodies are labeled with a facilitating moiety, the second activation state-independent antibodies are labeled with a first member of a signal amplification pair, and the facilitating moiety generates an oxidizing agent which channels to and reacts with the first member of the signal amplification pair;
(v) incubating (*e.g.*, contacting) the plurality of detectable captured truncated receptors with a second member of the signal amplification pair to generate an amplified signal; and
(vi) detecting the amplified signal generated from the first and second members of the signal amplification pair.

The first activation state-independent antibodies may be directly labeled with the facilitating moiety or indirectly labeled with the facilitating moiety, *e.g.*, via hybridization between an oligonucleotide conjugated to the first activation state-independent antibodies and a complementary oligonucleotide conjugated to the facilitating moiety. Similarly, the second activation state-independent antibodies may be directly labeled with the first member of the signal amplification pair or indirectly labeled with the first member of the signal amplification pair, *e.g.,* via binding between a first member of a binding pair conjugated to the second activation state-independent antibodies and a second member of the binding pair conjugated to the first member of the signal amplification pair. In certain instances, the first member of the binding pair is biotin and the second member of the binding pair is an avidin such as streptavidin or neutravidin.

In some embodiments, the facilitating moiety may be, for example, glucose oxidase. In certain instances, the glucose oxidase and the first activation state-independent antibodies can be conjugated to a sulfhydryl-activated dextran molecule as described in, *e.g.*, Examples 16-17 of PCT Publication No. WO2009/108637. The sulfhydryl-activated dextran molecule typically has a molecular weight of about 500kDa (*e.g.*, about 250, 300, 350, 400, 450, 500, 550, 600, 650, 700, or 750kDa). In other embodiments, the oxidizing agent may be, for example, hydrogen peroxide (H₂O₂). In yet other embodiments, the first member of the signal amplification pair may be, for example, a peroxidase such as horseradish peroxidase (HRP). In further embodiments, the second member of the signal amplification pair may be, for example, a tyramide reagent (*e.g.*, biotin-tyramide). Preferably, the amplified signal is generated by peroxidase oxidization of biotin-tyramide to produce an activated tyramide (*e.g.,* to transform the biotin-tyramide into an activated tyramide). The activated tyramide may be directly detected or indirectly detected, *e.g.*, upon the addition of a signal-detecting reagent. Non-limiting examples of signal-detecting reagents include streptavidin-labeled fluorophores and combinations of streptavidin-labeled peroxidases and chromogenic reagents such as, *e.g.*, 3,3',5,5'-tetramethylbenzidine (TMB).

In certain instances, the horseradish peroxidase and the second activation state-independent antibodies can be conjugated to a sulfhydryl-activated dextran molecule. The sulfhydryl-activated dextran molecule typically has a molecular weight of about 70kDa (*e.g.*, about 40, 45, 50, 55, 60, 65, 70, 75, 80, 85, 90, 95, or 100kDa).

The truncated receptor is typically a fragment of the full-length receptor and shares an intracellular domain (ICD) binding region with the full-length receptor. In certain embodiments, the full-length receptor comprises an extracellular domain (ECD) binding region, a transmembrane domain, and an intracellular domain (ICD) binding region. Without being bound to any particular theory, the truncated receptor may arise through the proteolytic processing of the ECD of the full-length receptor or by alternative initiation of translation from methionine residues that are located before, within, or after the transmembrane domain, *e.g.,* to create a truncated receptor with a shortened ECD or a truncated receptor comprising a membrane-associated or cytosolic ICD fragment.

In certain preferred embodiments, the truncated receptor is p95HER2 and the corresponding full-length receptor is HER2. However, one skilled in the art will appreciate that the methods described herein for detecting truncated proteins can be applied to a number of different proteins including, but not limited to, the EGFR VIII mutant (implicated in glioblastoma, colorectal cancer, *etc.*), other truncated receptor tyrosine kinases, caspases, and the like. Example 12 of PCT Publication No. WO2009/108637 provides an exemplary embodiment of the assay methods of the present invention for detecting truncated receptors such as p95HER2 in cells using a multiplex, high-throughput, proximity dual detection microarray ELISA having superior dynamic range.

In some embodiments, the plurality of beads specific for an ECD binding region comprises a streptavidin-biotin pair, wherein the streptavidin is attached to the bead and the biotin is attached to an antibody. In certain instances, the antibody is specific for the ECD binding region of the full-length receptor (*e.g.*, full-length HER2).

In some embodiments, each dilution series of capture antibodies comprises a series of descending capture antibody concentrations. In certain instances, the capture antibodies are serially diluted at least 2-fold (*e.g.,* 2, 5, 10, 20, 50, 100, 500, or 1000-fold) to produce a dilution series comprising a set number (*e.g.*, 2, 3, 4, 5, 6, 7, 8, 9, 10, 15, 20, 25, or more) of descending capture antibody concentrations which are spotted onto an array. Preferably, at least 2, 3, 4, 5, or 6 replicates of each capture antibody dilution are spotted onto the array.

In other embodiments, the solid support comprises glass (*e.g.*, a glass slide), plastic, chips, pins, filters, beads, paper, membrane (*e.g.*, nylon, nitrocellulose, polyvinylidene fluoride (PVDF), *etc.*), fiber bundles, or any other suitable substrate. In a preferred embodiment, the capture antibodies are restrained (*e.g.*, via covalent or noncovalent interactions) on glass slides coated with a nitrocellulose polymer such as, for example, FAST^{®} Slides, which are commercially available from Whatman Inc. (Florham Park, NJ). Exemplary methods for constructing antibody arrays suitable for use in the invention are described, *e.g.*, in PCT Publication No. WO2009/108637.

In further embodiments, determining the activation levels of one or more analytes comprises detecting a phosphorylation level of the one or more analytes in the cellular extract with antibodies specific for the phosphorylated form of each of the analytes to be detected.

Phosphorylation levels and/or status can be determined using any of a variety of techniques. For example, it is well known in the art that phosphorylated proteins can be detected via immunoassays using antibodies that specifically recognize the phosphorylated form of the protein (*see, e.g.,* Lin et al., Br. J. Cancer, 93:1372-1381 (2005)). Immunoassays generally include immunoblotting (*e.g.*, Western blotting), RIA, and ELISA. More specific types of immunoassays include antigen capture/antigen competition, antibody capture/antigen competition, two-antibody sandwiches, antibody capture/antibody excess, and antibody capture/antigen excess. Methods of making antibodies are described herein and in Harlow and Lane, Antibodies: A Laboratory Manual, 1988, Cold Spring Harbor Laboratory Press, Cold Spring Harbor, NY, USA. Phospho-specifc antibodies can be made *de novo* or obtained from commercial or noncommercial sources. Phosphorylation levels and/or status can also be determined by metabolically labeling cells with radioactive phosphate in the form of [γ-³²P]ATP or [γ-³³P]ATP. Phosphorylated proteins become radioactive and hence traceable and quantifiable through scintillation counting, radiography, and the like (see, e.g., Wang et al., J. Biol. Chem., 253:7605-7608 (1978)). For example, metabolically labeled proteins can be extracted from cells, separated by gel electrophoresis, transferred to a membrane, probed with an antibody specific for a particular analyte and subjected to autoradiography to detect ³²P or ³³P. Alternatively, the gel can be subjected to autoradiography prior to membrane transference and antibody probing.

In particular embodiments, the activation (*e.g.*, phosphorylation) level and/or status of one or more analytes of interest in a sample such as a cellular extract from a cell line (*e.g.*, gastric cancer cell line) or tumor tissue (*e.g.*, gastric tumor tissue) is detected with an immunoassay such as a single detection assay or a proximity dual detection assay (*e.g.*, a Collaborative Enzyme Enhanced Reactive Immunoassay (CEER™)) as described herein.

In certain embodiments, determining the activation (*e.g.*, phosphorylation) level of one or more analytes comprises:
(i) incubating (*e.g.*, contacting) a cellular extract produced from a cell with a dilution series of capture antibodies (*e.g.*, capture antibodies specific for one or more analytes) to form a plurality of captured analytes, wherein the capture antibodies are restrained on a solid support (*e.g.*, to transform the analytes present in the cellular extract into complexes of captured analytes comprising the analytes and capture antibodies);
(ii) incubating (*e.g.*, contacting) the plurality of captured analytes with detection antibodies comprising activation state-independent antibodies specific for the corresponding analytes (*e.g.*, activation state-independent antibodies specific for the one or more analytes) and activation state-dependent antibodies specific for the corresponding analytes (*e.g.*, activation state-dependent antibodies specific for the one or more analytes) to form a plurality of detectable captured analytes (*e.g.*, to transform the complexes of captured analytes into complexes of detectable captured analytes comprising the captured analytes and detection antibodies),
   wherein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(iii) incubating (*e.g.*, contacting) the plurality of detectable captured analytes with a second member of the signal amplification pair to generate an amplified signal; and
(iv) detecting the amplified signal generated from the first and second members of the signal amplification pair.

In certain other embodiments, determining the activation (*e.g.*, phosphorylation) level of one or more analytes that are truncated receptors (*e.g.*, p95HER2) comprises:
(i) incubating (*e.g.*, contacting) a cellular extract produced from a cell with a plurality of beads specific for an extracellular domain (ECD) binding region of a full-length receptor (*e.g.*, full-length HER2);
(ii) removing the plurality of beads from the cellular extract, thereby removing the full-length receptor (*e.g.*, full-length HER2) to form a cellular extract devoid of the full-length receptor (*e.g.*, full-length HER2) (*e.g.*, to transform the cellular extract into a cellular extract devoid of a specific full-length receptor or family of full-length receptors);
(iii) incubating (*e.g.*, contacting) the cellular extract devoid of the full-length receptor (*e.g.*, full-length HER2) with a plurality of capture antibodies specific for an intracellular domain (ICD) binding region of the full-length receptor (*e.g.,* full-length HER2) to form a plurality of captured truncated receptors, wherein the capture antibodies are restrained on a solid support (*e.g.*, to transform the truncated receptors present in a full-length receptor-depleted cellular extract into complexes of truncated receptors and capture antibodies);
(iv) incubating (*e.g.*, contacting) the plurality of captured truncated receptors with detection antibodies comprising activation state-independent antibodies and activation state-dependent antibodies specific for an ICD binding region of the full-length receptor (*e.g.*, full-length HER2) to form a plurality of detectable captured truncated receptors (*e.g.,* to transform the complexes of captured truncated receptors into complexes of detectable captured truncated receptors comprising the captured truncated receptors and detection antibodies),
   wherein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(v) incubating (*e.g.*, contacting) the plurality of detectable captured truncated receptors with a second member of the signal amplification pair to generate an amplified signal; and
(vi) detecting the amplified signal generated from the first and second members of the signal amplification pair.

The activation state-independent antibodies may be directly labeled with the facilitating moiety or indirectly labeled with the facilitating moiety, *e.g.*, via hybridization between an oligonucleotide conjugated to the activation state-independent antibodies and a complementary oligonucleotide conjugated to the facilitating moiety. Similarly, the activation state-dependent antibodies may be directly labeled with the first member of the signal amplification pair or indirectly labeled with the first member of the signal amplification pair, *e.g.*, via binding between a first member of a binding pair conjugated to the activation state-dependent antibodies and a second member of the binding pair conjugated to the first member of the signal amplification pair. In certain instances, the first member of the binding pair is biotin and the second member of the binding pair is an avidin such as streptavidin or neutravidin.

In some embodiments, the facilitating moiety may be, for example, glucose oxidase. In certain instances, the glucose oxidase and the activation state-independent antibodies can be conjugated to a sulfhydryl-activated dextran molecule as described in, *e.g.*, Examples 16-17 of PCT Publication No. WO2009/108637. The sulfhydryl-activated dextran molecule typically has a molecular weight of about 500kDa (*e.g.*, about 250, 300, 350, 400, 450, 500, 550, 600, 650, 700, or 750kDa). In other embodiments, the oxidizing agent may be, for example, hydrogen peroxide (H₂O₂). In yet other embodiments, the first member of the signal amplification pair may be, for example, a peroxidase such as horseradish peroxidase (HRP). In further embodiments, the second member of the signal amplification pair may be, for example, a tyramide reagent (*e.g.*, biotin-tyramide). Preferably, the amplified signal is generated by peroxidase oxidization of biotin-tyramide to produce an activated tyramide (*e.g.*, to transform the biotin-tyramide into an activated tyramide). The activated tyramide may be directly detected or indirectly detected, *e.g.*, upon the addition of a signal-detecting reagent. Non-limiting examples of signal-detecting reagents include streptavidin-labeled fluorophores and combinations of streptavidin-labeled peroxidases and chromogenic reagents such as, *e.g.*, 3,3',5,5'-tetramethylbenzidine (TMB).

In certain instances, the horseradish peroxidase and the activation state-dependent antibodies can be conjugated to a sulfhydryl-activated dextran molecule. The sulfhydryl-activated dextran molecule typically has a molecular weight of about 70kDa (*e.g.*, about 40, 45, 50, 55, 60, 65, 70, 75, 80, 85, 90, 95, or 100kDa).

The truncated receptor is typically a fragment of the full-length receptor and shares an intracellular domain (ICD) binding region with the full-length receptor. In certain embodiments, the full-length receptor comprises an extracellular domain (ECD) binding region, a transmembrane domain, and an intracellular domain (ICD) binding region. Without being bound to any particular theory, the truncated receptor may arise through the proteolytic processing of the ECD of the full-length receptor or by alternative initiation of translation from methionine residues that are located before, within, or after the transmembrane domain, *e.g.*, to create a truncated receptor with a shortened ECD or a truncated receptor comprising a membrane-associated or cytosolic ICD fragment.

In certain preferred embodiments, the truncated receptor is p95HER2 and the corresponding full-length receptor is HER2. However, one skilled in the art will appreciate that the methods described herein for detecting truncated proteins can be applied to a number of different proteins including, but not limited to, the EGFR VIII mutant (implicated in glioblastoma, colorectal cancer, *etc.*), other truncated receptor tyrosine kinases, caspases, and the like. Example 12 of PCT Publication No. WO2009/108637provides an exemplary embodiment of the assay methods of the present invention for detecting truncated receptors such as p95HER2 in cells using a multiplex, high-throughput, proximity dual detection microarray ELISA having superior dynamic range.

In some embodiments, the plurality of beads specific for an ECD binding region comprises a streptavidin-biotin pair, wherein the streptavidin is attached to the bead and the biotin is attached to an antibody. In certain instances, the antibody is specific for the ECD binding region of the full-length receptor (*e.g.*, full-length HER2).

In some embodiments, each dilution series of capture antibodies comprises a series of descending capture antibody concentrations. In certain instances, the capture antibodies are serially diluted at least 2-fold (*e.g.*, 2, 5, 10, 20, 50, 100, 500, or 1000-fold) to produce a dilution series comprising a set number (*e.g.*, 2, 3, 4, 5, 6, 7, 8, 9, 10, 15, 20, 25, or more) of descending capture antibody concentrations which are spotted onto an array. Preferably, at least 2, 3, 4, 5, or 6 replicates of each capture antibody dilution are spotted onto the array.

In other embodiments, the solid support comprises glass (*e.g.*, a glass slide), plastic, chips, pins, filters, beads, paper, membrane (*e.g.*, nylon, nitrocellulose, polyvinylidene fluoride (PVDF), *etc.*), fiber bundles, or any other suitable substrate. In a preferred embodiment, the capture antibodies are restrained (*e.g.*, via covalent or noncovalent interactions) on glass slides coated with a nitrocellulose polymer such as, for example, FAST^{®} Slides, which are commercially available from Whatman Inc. (Florham Park, NJ). Exemplary methods for constructing antibody arrays suitable for use in the invention are described, *e.g.*, in PCT Publication No. WO2009/108637.

### IV. Single Detection Assays

In some embodiments, the assay for detecting the expression and/or activation level or status of one or more analytes (*e.g.*, one or more signal transduction molecules such as one or more components of the HER and/or c-Met signaling pathways) of interest in a cellular extract of cells such as tumor cells is a multiplex, high-throughput two-antibody assay having superior dynamic range. As a non-limiting example, the two antibodies used in the assay can comprise: (1) a capture antibody specific for a particular analyte of interest; and (2) a detection antibody specific for an activated form of the analyte (*i.e.*, activation state-dependent antibody). The activation state-dependent antibody is capable of detecting, for example, the phosphorylation, ubiquitination, and/or complexation state of the analyte. Alternatively, the detection antibody comprises an activation state-independent antibody, which detects the total amount of the analyte in the cellular extract. The activation state-independent antibody is generally capable of detecting both the activated and non-activated forms of the analyte.

In one particular embodiment, the two-antibody assay for detecting the expression or activation level of an analyte of interest comprises:
(i) incubating the cellular extract with one or a plurality of dilution series of capture antibodies to form a plurality of captured analytes;
(ii) incubating the plurality of captured analytes with detection antibodies specific for the corresponding analytes to form a plurality of detectable captured analytes, wherein the detection antibodies comprise activation state-dependent antibodies for detecting the activation (*e.g.*, phosphorylation) level of the analyte or activation state-independent antibodies for detecting the expression level (*e.g.*, total amount) of the analyte;
(iii) incubating the plurality of detectable captured analytes with first and second members of a signal amplification pair to generate an amplified signal; and
(iv) detecting the amplified signal generated from the first and second members of the signal amplification pair.

The two-antibody assays described herein are typically antibody-based arrays which comprise a plurality of different capture antibodies at a range of capture antibody concentrations that are coupled to the surface of a solid support in different addressable locations. Examples of suitable solid supports for use in the present invention are described above.

The capture antibodies and detection antibodies are preferably selected to minimize competition between them with respect to analyte binding (*i. e.*, both capture and detection antibodies can simultaneously bind their corresponding signal transduction molecules).

In one embodiment, the detection antibodies comprise a first member of a binding pair (*e.g.*, biotin) and the first member of the signal amplification pair comprises a second member of the binding pair (*e.g.*, streptavidin). The binding pair members can be coupled directly or indirectly to the detection antibodies or to the first member of the signal amplification pair using methods well-known in the art. In certain instances, the first member of the signal amplification pair is a peroxidase (*e.g.*, horseradish peroxidase (HRP), catalase, chloroperoxidase, cytochrome c peroxidase, eosinophil peroxidase, glutathione peroxidase, lactoperoxidase, myeloperoxidase, thyroid peroxidase, deiodinase, *etc.*), and the second member of the signal amplification pair is a tyramide reagent (*e.g.*, biotin-tyramide). In these instances, the amplified signal is generated by peroxidase oxidization of the tyramide reagent to produce an activated tyramide in the presence of hydrogen peroxide (H₂O₂).

The activated tyramide is either directly detected or detected upon the addition of a signal-detecting reagent such as, for example, a streptavidin-labeled fluorophore or a combination of a streptavidin-labeled peroxidase and a chromogenic reagent. Examples of fluorophores suitable for use in the present invention include, but are not limited to, an Alexa Fluor^{®} dye (*e.g.*, Alexa Fluor^{®} 555), fluorescein, fluorescein isothiocyanate (FITC), Oregon Green™; rhodamine, Texas red, tetrarhodamine isothiocynate (TRITC), a CyDye™ fluor (*e.g.,* Cy2, Cy3, Cy5), and the like. The streptavidin label can be coupled directly or indirectly to the fluorophore or peroxidase using methods well-known in the art. Non-limiting examples of chromogenic reagents suitable for use in the present invention include 3,3',5,5'-tetramethylbenzidine (TMB), 3,3'-diaminobenzidine (DAB), 2,2'-azino-bis(3-ethylbenzothiazoline-6-sulfonic acid) (ABTS), 4-chloro-1-napthol (4CN), and/or porphyrinogen.

An exemplary protocol for performing the two-antibody assays described herein is provided in Example 3 of PCT Publication No. WO2009/108637.

In another embodiment of a two-antibody approach, the present invention provides a method for detecting the expression or activation level of a truncated receptor, the method comprising:
(i) incubating the cellular extract with a plurality of beads specific for an extracellular domain (ECD) binding region of a full-length receptor;
(ii) removing the plurality of beads from the cellular extract, thereby removing the full-length receptor to form a cellular extract devoid of the full-length receptor;
(iii) incubating the cellular extract devoid of the full-length receptor with a dilution series of one or a plurality of capture antibodies specific for an intracellular domain (ICD) binding region of the full-length receptor to form a plurality of captured truncated receptors;
(iv) incubating the plurality of captured truncated receptors with detection antibodies specific for an ICD binding region of the full-length receptor to form a plurality of detectable captured truncated receptors, wherein the detection antibodies comprise activation state-dependent antibodies for detecting the activation (*e.g.*, phosphorylation) level of the truncated receptor or activation state-independent antibodies for detecting the expression level (*e.g.*, total amount) of the truncated receptor;
(v) incubating the plurality of detectable captured truncated receptors with first and second members of a signal amplification pair to generate an amplified signal; and
(vi) detecting an amplified signal generated from the first and second members of the signal amplification pair.

In certain embodiments, the truncated receptor is p95HER2 and the full-length receptor is HER2. In certain other embodiments, the plurality of beads specific for an extracellular domain (ECD) binding region comprises a streptavidin-biotin pair, wherein the biotin is attached to the bead and the biotin is attached to an antibody (*e.g.*, wherein the antibody is specific for the ECD binding region of the full-length receptor).

Figure 14A of PCT Publication No. WO2009/108637 shows that beads coated with an antibody directed to the extracellular domain (ECD) of a receptor of interest binds the full-length receptor *(e.g.,* HER2), but not the truncated receptor (*e.g.,* p95HER2) to remove any full-length receptor from the assay. Figure 14B of PCT Publication No. WO2009/108637 shows that the truncated receptor (*e.g.*, p95HER2), once bound to a capture antibody, may then be detected by a detection antibody that is specific for the intracellular domain (ICD) of the full-length receptor (*e.g.*, HER2). The detection antibody may be directly conjugated to horseradish peroxidase (HRP). Tyramide signal amplification (TSA) may then be performed to generate a signal to be detected. The expression level or activation state of the truncated receptor (*e.g.*, p95HER2) can be interrogated to determine, *e.g.*, its total concentration or its phosphorylation state, ubiquitination state, and/or complexation state.

In another embodiment, the present invention provides kits for performing the two-antibody assays described above comprising: (a) a dilution series of one or a plurality of capture antibodies restrained on a solid support; and (b) one or a plurality of detection antibodies (*e.g.*, activation state-independent antibodies and/or activation state-dependent antibodies). In some instances, the kits can further contain instructions for methods of using the kit to detect the expression levels and/or activation states of one or a plurality of signal transduction molecules of cells such as tumor cells. The kits may also contain any of the additional reagents described above with respect to performing the specific methods of the present invention such as, for example, first and second members of the signal amplification pair, tyramide signal amplification reagents, wash buffers, *etc.*

### V. Proximity Dual Detection Assays

In some embodiments, the assay for detecting the expression and/or activation level of one or more analytes (*e.g.*, one or more signal transduction molecules such as one or more components of the HER and/or c-Met signaling pathways) of interest in a cellular extract of cells such as tumor cells is a multiplex, high-throughput proximity (*i. e.,* three-antibody) assay having superior dynamic range. As a non-limiting example, the three antibodies used in the proximity assay can comprise: (1) a capture antibody specific for a particular analyte of interest; (2) a detection antibody specific for an activated form of the analyte (*i.e.*, activation state-dependent antibody); and (3) a detection antibody which detects the total amount of the analyte (*i.e.*, activation state-independent antibody). The activation state-dependent antibody is capable of detecting, *e.g.*, the phosphorylation, ubiquitination, and/or complexation state of the analyte, while the activation state-independent antibody is capable of detecting the total amount (*i.e.*, both the activated and non-activated forms) of the analyte.

In one particular embodiment, the proximity assay for detecting the activation level or status of an analyte of interest comprises:
(i) incubating the cellular extract with one or a plurality of dilution series of capture antibodies to form a plurality of captured analytes;
(ii) incubating the plurality of captured analytes with detection antibodies comprising one or a plurality of activation state-independent antibodies and one or a plurality of activation state-dependent antibodies specific for the corresponding analytes to form a plurality of detectable captured analytes,
   wherein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(iii) incubating the plurality of detectable captured analytes with a second member of the signal amplification pair to generate an amplified signal; and
(iv) detecting the amplified signal generated from the first and second members of the signal amplification pair.

In another particular embodiment, the proximity assay for detecting the activation level or status of an analyte of interest that is a truncated receptor comprises:
(i) incubating the cellular extract with a plurality of beads specific for an extracellular domain (ECD) binding region of a full-length receptor;
(ii) removing the plurality of beads from the cellular extract, thereby removing the full-length receptor to form a cellular extract devoid of the full-length receptor;
(iii) incubating the cellular extract devoid of the full-length receptor with one or a plurality of capture antibodies specific for an intracellular domain (ICD) binding region of the full-length receptor to form a plurality of captured truncated receptors;
(iv) incubating the plurality of captured truncated receptors with detection antibodies comprising one or a plurality of activation state-independent antibodies and one or a plurality of activation state-dependent antibodies specific for an ICD binding region of the full-length receptor to form a plurality of detectable captured truncated receptors,
   wherein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(v) incubating the plurality of detectable captured truncated receptors with a second member of the signal amplification pair to generate an amplified signal; and
(vi) detecting the amplified signal generated from the first and second members of the signal amplification pair.

In certain embodiments, the truncated receptor is p95HER2 and the full-length receptor is HER2. In certain other embodiments, the plurality of beads specific for an extracellular domain (ECD) binding region comprises a streptavidin-biotin pair, wherein the biotin is attached to the bead and the biotin is attached to an antibody (*e.g.*, wherein the antibody is specific for the ECD binding region of the full-length receptor).

In alternative embodiments, the activation state-dependent antibodies can be labeled with a facilitating moiety and the activation state-independent antibodies can be labeled with a first member of a signal amplification pair.

As another non-limiting example, the three antibodies used in the proximity assay can comprise: (1) a capture antibody specific for a particular analyte of interest; (2) a first detection antibody which detects the total amount of the analyte (*i. e.,* a first activation state-independent antibody); and (3) a second detection antibody which detects the total amount of the analyte (*i. e.,* a second activation state-independent antibody). In preferred embodiments, the first and second activation state-independent antibodies recognize different (*e.g.*, distinct) epitopes on the analyte.

In one particular embodiment, the proximity assay for detecting the expression level of an analyte of interest comprises:
(i) incubating the cellular extract with one or a plurality of dilution series of capture antibodies to form a plurality of captured analytes;
(ii) incubating the plurality of captured analytes with detection antibodies comprising one or a plurality of first and second activation state-independent antibodies specific for the corresponding analytes to form a plurality of detectable captured analytes,
   wherein the first activation state-independent antibodies are labeled with a facilitating moiety, the second activation state-independent antibodies are labeled with a first member of a signal amplification pair, and the facilitating moiety generates an oxidizing agent which channels to and reacts with the first member of the signal amplification pair;
(iii) incubating the plurality of detectable captured analytes with a second member of the signal amplification pair to generate an amplified signal; and
(iv) detecting the amplified signal generated from the first and second members of the signal amplification pair.

In another particular embodiment, the proximity assay for detecting the expression level of an analyte of interest that is a truncated receptor comprises:
(i) incubating the cellular extract with a plurality of beads specific for an extracellular domain (ECD) binding region of a full-length receptor;
(ii) removing the plurality of beads from the cellular extract, thereby removing the full-length receptor to form a cellular extract devoid of the full-length receptor;
(iii) incubating the cellular extract devoid of the full-length receptor with one or a plurality of capture antibodies specific for an intracellular domain (ICD) binding region of the full-length receptor to form a plurality of captured truncated receptors;
(iv) incubating the plurality of captured truncated receptors with detection antibodies comprising one or a plurality of first and second activation state-independent antibodies specific for an ICD binding region of the full-length receptor to form a plurality of detectable captured truncated receptors,
   wherein the first activation state-independent antibodies are labeled with a facilitating moiety, the second activation state-independent antibodies are labeled with a first member of a signal amplification pair, and the facilitating moiety generates an oxidizing agent which channels to and reacts with the first member of the signal amplification pair;
(v) incubating the plurality of detectable captured truncated receptors with a second member of the signal amplification pair to generate an amplified signal; and
(vi) detecting the amplified signal generated from the first and second members of the signal amplification pair.

In certain embodiments, the truncated receptor is p95HER2 and the full-length receptor is HER2. In certain other embodiments, the plurality of beads specific for an extracellular domain (ECD) binding region comprises a streptavidin-biotin pair, wherein the biotin is attached to the bead and the biotin is attached to an antibody (*e.g.*, wherein the antibody is specific for the ECD binding region of the full-length receptor).

In alternative embodiments, the first activation state-independent antibodies can be labeled with a first member of a signal amplification pair and the second activation state-independent antibodies can be labeled with a facilitating moiety.

The proximity assays described herein are typically antibody-based arrays which comprise one or a plurality of different capture antibodies at a range of capture antibody concentrations that are coupled to the surface of a solid support in different addressable locations. Examples of suitable solid supports for use in the present invention are described above.

The capture antibodies, activation state-independent antibodies, and activation state-dependent antibodies are preferably selected to minimize competition between them with respect to analyte binding *(i. e.,* all antibodies can simultaneously bind their corresponding signal transduction molecules).

In some embodiments, activation state-independent antibodies for detecting activation levels of one or more of the analytes or, alternatively, first activation state-independent antibodies for detecting expression levels of one or more of the analytes further comprise a detectable moiety. In such instances, the amount of the detectable moiety is correlative to the amount of one or more of the analytes in the cellular extract. Examples of detectable moieties include, but are not limited to, fluorescent labels, chemically reactive labels, enzyme labels, radioactive labels, and the like. Preferably, the detectable moiety is a fluorophore such as an Alexa Fluor^{®} dye (*e.g.,* Alexa Fluor^{®} 647), fluorescein, fluorescein isothiocyanate (FITC), Oregon Green™; rhodamine, Texas red, tetrarhodamine isothiocynate (TRITC), a CyDye™ fluor (*e.g.*, Cy2, Cy3, Cy5), and the like. The detectable moiety can be coupled directly or indirectly to the activation state-independent antibodies using methods well-known in the art.

In certain instances, activation state-independent antibodies for detecting activation levels of one or more of the analytes or, alternatively, first activation state-independent antibodies for detecting expression levels of one or more of the analytes are directly labeled with the facilitating moiety. The facilitating moiety can be coupled to activation state-independent antibodies using methods well-known in the art. A suitable facilitating moiety for use in the present invention includes any molecule capable of generating an oxidizing agent which channels to *(i. e.,* is directed to) and reacts with (*i. e.,* binds, is bound by, or forms a complex with) another molecule in proximity (*i. e.,* spatially near or close) to the facilitating moiety. Examples of facilitating moieties include, without limitation, enzymes such as glucose oxidase or any other enzyme that catalyzes an oxidation/reduction reaction involving molecular oxygen (O₂) as the electron acceptor, and photosensitizers such as methylene blue, rose bengal, porphyrins, squarate dyes, phthalocyanines, and the like. Non-limiting examples of oxidizing agents include hydrogen peroxide (H₂O₂), a singlet oxygen, and any other compound that transfers oxygen atoms or gains electrons in an oxidation/reduction reaction. Preferably, in the presence of a suitable substrate (*e.g.,* glucose, light, *etc.),* the facilitating moiety (*e.g.,* glucose oxidase, photosensitizer, *etc.)* generates an oxidizing agent (*e.g.,* hydrogen peroxide (H₂O₂), single oxygen, *etc.)* which channels to and reacts with the first member of the signal amplification pair (*e.g.*, horseradish peroxidase (HRP), hapten protected by a protecting group, an enzyme inactivated by thioether linkage to an enzyme inhibitor, *etc.)* when the two moieties are in proximity to each other.

In certain other instances, activation state-independent antibodies for detecting activation levels of one or more of the analytes or, alternatively, first activation state-independent antibodies for detecting expression levels of one or more of the analytes are indirectly labeled with the facilitating moiety via hybridization between an oligonucleotide linker conjugated to the activation state-independent antibodies and a complementary oligonucleotide linker conjugated to the facilitating moiety. The oligonucleotide linkers can be coupled to the facilitating moiety or to the activation state-independent antibodies using methods well-known in the art. In some embodiments, the oligonucleotide linker conjugated to the facilitating moiety has 100% complementarity to the oligonucleotide linker conjugated to the activation state-independent antibodies. In other embodiments, the oligonucleotide linker pair comprises at least one, two, three, four, five, six, or more mismatch regions, *e.g.*, upon hybridization under stringent hybridization conditions. One skilled in the art will appreciate that activation state-independent antibodies specific for different analytes can either be conjugated to the same oligonucleotide linker or to different oligonucleotide linkers.

The length of the oligonucleotide linkers that are conjugated to the facilitating moiety or to the activation state-independent antibodies can vary. In general, the linker sequence can be at least about 5, 10, 15, 20, 25, 30, 35, 40, 45, 50, 75, or 100 nucleotides in length. Typically, random nucleic acid sequences are generated for coupling. As a non-limiting example, a library of oligonucleotide linkers can be designed to have three distinct contiguous domains: a spacer domain; signature domain; and conjugation domain. Preferably, the oligonucleotide linkers are designed for efficient coupling without destroying the function of the facilitating moiety or activation state-independent antibodies to which they are conjugated.

The oligonucleotide linker sequences can be designed to prevent or minimize any secondary structure formation under a variety of assay conditions. Melting temperatures are typically carefully monitored for each segment within the linker to allow their participation in the overall assay procedures. Generally, the range of melting temperatures of the segment of the linker sequence is between 1-10°C. Computer algorithms (*e.g.*, OLIGO 6.0) for determining the melting temperature, secondary structure, and hairpin structure under defined ionic concentrations can be used to analyze each of the three different domains within each linker. The overall combined sequences can also be analyzed for their structural characterization and their comparability to other conjugated oligonucleotide linker sequences, *e.g.*, whether they will hybridize under stringent hybridization conditions to a complementary oligonucleotide linker.

The spacer region of the oligonucleotide linker provides adequate separation of the conjugation domain from the oligonucleotide crosslinking site. The conjugation domain functions to link molecules labeled with a complementary oligonucleotide linker sequence to the conjugation domain via nucleic acid hybridization. The nucleic acid-mediated hybridization can be performed either before or after antibody-analyte (*i. e.*, antigen) complex formation, providing a more flexible assay format. Unlike many direct antibody conjugation methods, linking relatively small oligonucleotides to antibodies or other molecules has minimal impact on the specific affinity of antibodies towards their target analyte or on the function of the conjugated molecules.

In some embodiments, the signature sequence domain of the oligonucleotide linker can be used in complex multiplexed protein assays. Multiple antibodies can be conjugated with oligonucleotide linkers with different signature sequences. In multiplex immunoassays, reporter oligonucleotide sequences labeled with appropriate probes can be used to detect cross-reactivity between antibodies and their antigens in the multiplex assay format.

Oligonucleotide linkers can be conjugated to antibodies or other molecules using several different methods. For example, oligonucleotide linkers can be synthesized with a thiol group on either the 5' or 3' end. The thiol group can be deprotected using reducing agents (*e.g.*, TCEP-HCl) and the resulting linkers can be purified by using a desalting spin column. The resulting deprotected oligonucleotide linkers can be conjugated to the primary amines of antibodies or other types of proteins using heterobifunctional cross linkers such as SMCC. Alternatively, 5'-phosphate groups on oligonucleotides can be treated with watersoluble carbodiimide EDC to form phosphate esters and subsequently coupled to amine-containing molecules. In certain instances, the diol on the 3'-ribose residue can be oxidized to aldehyde groups and then conjugated to the amine groups of antibodies or other types of proteins using reductive amination. In certain other instances, the oligonucleotide linker can be synthesized with a biotin modification on either the 3' or 5' end and conjugated to streptavidin-labeled molecules.

Oligonucleotide linkers can be synthesized using any of a variety of techniques known in the art, such as those described in Usman et al., J. Am. Chem. Soc., 109:7845 (1987); Scaringe et al., Nucl. Acids Res., 18:5433 (1990); Wincott et al., Nucl. Acids Res., 23:2677-2684 (1995); and Wincott et al., Methods Mol. Bio., 74:59 (1997). In general, the synthesis of oligonucleotides makes use of common nucleic acid protecting and coupling groups, such as dimethoxytrityl at the 5'-end and phosphoramidites at the 3'-end. Suitable reagents for oligonucleotide synthesis, methods for nucleic acid deprotection, and methods for nucleic acid purification are known to those of skill in the art.

In certain instances, activation state-dependent antibodies for detecting activation levels of one or more of the analytes or, alternatively, second activation state-independent antibodies for detecting expression levels of one or more of the analytes are directly labeled with the first member of the signal amplification pair. The signal amplification pair member can be coupled to activation state-dependent antibodies to detect activation levels or second activation state-independent antibodies to detect expression levels using methods well-known in the art. In certain other instances, activation state-dependent antibodies or second activation state-independent antibodies are indirectly labeled with the first member of the signal amplification pair via binding between a first member of a binding pair conjugated to the activation state-dependent antibodies or second activation state-independent antibodies and a second member of the binding pair conjugated to the first member of the signal amplification pair. The binding pair members (*e.g.*, biotin/streptavidin) can be coupled to the signal amplification pair member or to the activation state-dependent antibodies or second activation state-independent antibodies using methods well-known in the art. Examples of signal amplification pair members include, but are not limited to, peroxidases such horseradish peroxidase (HRP), catalase, chloroperoxidase, cytochrome c peroxidase, eosinophil peroxidase, glutathione peroxidase, lactoperoxidase, myeloperoxidase, thyroid peroxidase, deiodinase, and the like. Other examples of signal amplification pair members include haptens protected by a protecting group and enzymes inactivated by thioether linkage to an enzyme inhibitor.

In one example of proximity channeling, the facilitating moiety is glucose oxidase (GO) and the first member of the signal amplification pair is horseradish peroxidase (HRP). When the GO is contacted with a substrate such as glucose, it generates an oxidizing agent *(i. e.,* hydrogen peroxide (H₂O₂)). If the HRP is within channeling proximity to the GO, the H₂O₂ generated by the GO is channeled to and complexes with the HRP to form an HRP-H₂O₂ complex, which, in the presence of the second member of the signal amplification pair (*e.g.,* a chemiluminescent substrate such as luminol or isoluminol or a fluorogenic substrate such as tyramide (*e.g.*, biotin-tyramide), homovanillic acid, or 4-hydroxyphenyl acetic acid), generates an amplified signal. Methods of using GO and HRP in a proximity assay are described in, *e.g.,* Langry et al., U.S. Dept. of Energy Report No. UCRL-ID-136797 (1999). When biotin-tyramide is used as the second member of the signal amplification pair, the HRP-H₂O₂ complex oxidizes the tyramide to generate a reactive tyramide radical that covalently binds nearby nucleophilic residues. The activated tyramide is either directly detected or detected upon the addition of a signal-detecting reagent such as, for example, a streptavidin-labeled fluorophore or a combination of a streptavidin-labeled peroxidase and a chromogenic reagent. Examples of fluorophores suitable for use in the present invention include, but are not limited to, an Alexa Fluor^{®} dye (*e.g.*, Alexa Fluor^{®} 555), fluorescein, fluorescein isothiocyanate (FITC), Oregon Green™; rhodamine, Texas red, tetrarhodamine isothiocynate (TRITC), a CyDye™ fluor (*e.g.*, Cy2, Cy3, Cy5), and the like. The streptavidin label can be coupled directly or indirectly to the fluorophore or peroxidase using methods well-known in the art. Non-limiting examples of chromogenic reagents suitable for use in the present invention include 3,3',5,5'-tetramethylbenzidine (TMB), 3,3'-diaminobenzidine (DAB), 2,2'-azino-bis(3-ethylbenzothiazoline-6-sulfonic acid) (ABTS), 4-chloro-1-napthol (4CN), and/or porphyrinogen.

In another example of proximity channeling, the facilitating moiety is a photosensitizer and the first member of the signal amplification pair is a large molecule labeled with multiple haptens that are protected with protecting groups that prevent binding of the haptens to a specific binding partner (*e.g.,* ligand, antibody, *etc.*). For example, the signal amplification pair member can be a dextran molecule labeled with protected biotin, coumarin, and/or fluorescein molecules. Suitable protecting groups include, but are not limited to, phenoxy-, analino-, olefin-, thioether-, and selenoether-protecting groups. Additional photosensitizers and protected hapten molecules suitable for use in the proximity assays of the present invention are described in U.S. Patent No. 5,807,675. When the photosensitizer is excited with light, it generates an oxidizing agent *(i.e.,* singlet oxygen). If the hapten molecules are within channeling proximity to the photosensitizer, the singlet oxygen generated by the photosensitizer is channeled to and reacts with thioethers on the protecting groups of the haptens to yield carbonyl groups (ketones or aldehydes) and sulphinic acid, releasing the protecting groups from the haptens. The unprotected haptens are then available to specifically bind to the second member of the signal amplification pair *(e.g.,* a specific binding partner that can generate a detectable signal). For example, when the hapten is biotin, the specific binding partner can be an enzyme-labeled streptavidin. Exemplary enzymes include alkaline phosphatase, β-galactosidase, HRP, *etc.* After washing to remove unbound reagents, the detectable signal can be generated by adding a detectable *(e.g.,* fluorescent, chemiluminescent, chromogenic, *etc.)* substrate of the enzyme and detected using suitable methods and instrumentation known in the art. Alternatively, the detectable signal can be amplified using tyramide signal amplification and the activated tyramide either directly detected or detected upon the addition of a signal-detecting reagent as described above.

In yet another example of proximity channeling, the facilitating moiety is a photosensitizer and the first member of the signal amplification pair is an enzyme-inhibitor complex. The enzyme and inhibitor (*e.g.*, phosphonic acid-labeled dextran) are linked together by a cleavable linker (*e.g.*, thioether). When the photosensitizer is excited with light, it generates an oxidizing agent *(i.e.,* singlet oxygen). If the enzyme-inhibitor complex is within channeling proximity to the photosensitizer, the singlet oxygen generated by the photosensitizer is channeled to and reacts with the cleavable linker, releasing the inhibitor from the enzyme, thereby activating the enzyme. An enzyme substrate is added to generate a detectable signal, or alternatively, an amplification reagent is added to generate an amplified signal.

In a further example of proximity channeling, the facilitating moiety is HRP, the first member of the signal amplification pair is a protected hapten or an enzyme-inhibitor complex as described above, and the protecting groups comprise p-alkoxy phenol. The addition of phenylenediamine and H₂O₂ generates a reactive phenylene diimine which channels to the protected hapten or the enzyme-inhibitor complex and reacts with p-alkoxy phenol protecting groups to yield exposed haptens or a reactive enzyme. The amplified signal is generated and detected as described above *(see, e.g.,* U.S. Patent Nos. 5,532,138 and 5,445,944).

An exemplary protocol for performing the proximity assays described herein is provided in Example 4 of PCT Publication No. WO2009/108637.

In another embodiment, the present invention provides kits for performing the proximity assays described above comprising: (a) a dilution series of one or a plurality of capture antibodies restrained on a solid support; and (b) one or a plurality of detection antibodies (*e.g.*, a combination of activation state-independent antibodies and activation state-dependent antibodies for detecting activation levels and/or a combination of first and second activation state-independent antibodies for detecting expression levels). In some instances, the kits can further contain instructions for methods of using the kit to detect the expression and/or activation status of one or a plurality of signal transduction molecules of cells such as tumor cells. The kits may also contain any of the additional reagents described above with respect to performing the specific methods of the present invention such as, for example, first and second members of the signal amplification pair, tyramide signal amplification reagents, substrates for the facilitating moiety, wash buffers, *etc.*

### VI. Production of Antibodies

The generation and selection of antibodies not already commercially available for analyzing the levels of expression and activation of signal transduction molecules in tumor cells in accordance with the immunoassays of the present invention can be accomplished several ways. For example, one way is to express and/or purify a polypeptide of interest *(i.e.,* antigen) using protein expression and purification methods known in the art, while another way is to synthesize the polypeptide of interest using solid phase peptide synthesis methods known in the art. *See, e.g.,* Guide to Protein Purification, Murray P. Deutcher, ed., Meth. Enzymol., Vol. 182 (1990); Solid Phase Peptide Synthesis, Greg B. Fields, ed., Meth. Enzymol., Vol. 289 (1997); Kiso et al., Chem. Pharm. Bull., 38:1192-99 (1990); Mostafavi et al., Biomed. Pept. Proteins Nucleic Acids, 1:255-60, (1995); and Fujiwara et al., Chem. Pharm. Bull., 44:1326-31 (1996). The purified or synthesized polypeptide can then be injected, for example, into mice or rabbits, to generate polyclonal or monoclonal antibodies. One skilled in the art will recognize that many procedures are available for the production of antibodies, for example, as described in Antibodies, A Laboratory Manual, Harlow and Lane, Eds., Cold Spring Harbor Laboratory, Cold Spring Harbor, N.Y. (1988). One skilled in the art will also appreciate that binding fragments or Fab fragments which mimic (*e.g.,* retain the functional binding regions of) antibodies can also be prepared from genetic information by various procedures. *See, e.g.,* Antibody Engineering: A Practical Approach, Borrebaeck, Ed., Oxford University Press, Oxford (1995); and Huse et al., J. Immunol., 149:3914-3920 (1992).

Those skilled in the art will recognize that many approaches can be taken in producing antibodies or binding fragments and screening and selecting for affinity and specificity for the various polypeptides of interest, but these approaches do not change the scope of the present invention.

A more detailed description of polyclonal antibodies, monoclonal antibodies, humanized antibodies, human antibodies, bispecific antibodies, fragments thereof, and methods of purifying antibodies is found in PCT Publication No. WO 2010/132723.

One of skill in the art will appreciate that any binding molecule having a function similar to an antibody, *e.g.,* a binding molecule or binding partner which is specific for one or more analytes of interest in a sample, can also be used in the methods of the present invention. Examples of suitable antibody-like molecules include, but are not limited to, domain antibodies, unibodies, nanobodies, shark antigen reactive proteins, avimers, adnectins, anticalms, affinity ligands, phylomers, aptamers, affibodies, trinectins, and the like.

### VII. Methods of Administration

According to the methods of the present invention, the anticancer drugs described herein are administered to a subject by any convenient means known in the art. The methods of the present invention can be used to predict the therapeutic efficacy of an anticancer drug or a combination of anticancer drugs in a subject having a gastric tumor. The methods of the present invention can also be used to predict the response of a gastric tumor to treatment with an anticancer drug or a combination of anticancer drugs. The methods of the invention can also be used to select or identify a suitable anticancer drug or a combination of anticancer drugs for the treatment of a gastric tumor. The methods of the invention can also be used to treat a subject having stage I or II gastric cancer by administering a combination therapy of cMET and HER1 inhibitors to the subject. One skilled in the art will appreciate that one or more anticancer drugs described herein can be administered alone or as part of a combined therapeutic approach with conventional chemotherapy, radiotherapy, hormonal therapy, immunotherapy, and/or surgery.

In certain embodiments, the anticancer drug comprises an anti-signaling agent (*i.e.,* a cytostatic drug) such as a monoclonal antibody or a tyrosine kinase inhibitor; an anti-proliferative agent; a chemotherapeutic agent (*i.e.,* a cytotoxic drug); a hormonal therapeutic agent; a radiotherapeutic agent; a vaccine; and/or any other compound with the ability to reduce or abrogate the uncontrolled growth of aberrant cells such as cancerous cells. In some embodiments, the subject is treated with one or more anti-signaling agents, anti-proliferative agents, and/or hormonal therapeutic agents in combination with at least one chemotherapeutic agent. Exemplary monoclonal antibodies, tyrosine kinase inhibitors, anti-proliferative agents, chemotherapeutic agents, hormonal therapeutic agents, radiotherapeutic agents, and vaccines are described above.

In some embodiments, the anticancer drugs described herein can be co-administered with conventional immunotherapeutic agents including, but not limited to, immunostimulants *(e.g.,* Bacillus Calmette-Guérin (BCG), levamisole, interleukin-2, alpha-interferon, *etc.),* immunotoxins (*e.g.,* anti-CD33 monoclonal antibody-calicheamicin conjugate, anti-CD22 monoclonal antibody-pseudomonas exotoxin conjugate, *etc.),* and radioimmunotherapy (*e.g.,* anti-CD20 monoclonal antibody conjugated to ¹¹¹In, ⁹⁰Y, or ¹³¹I, *etc.*).

Anticancer drugs can be administered with a suitable pharmaceutical excipient as necessary and can be carried out via any of the accepted modes of administration. Thus, administration can be, for example, oral, buccal, sublingual, gingival, palatal, intravenous, topical, subcutaneous, transcutaneous, transdermal, intramuscular, intra-joint, parenteral, intra-arteriole, intradermal, intraventricular, intracranial, intraperitoneal, intravesical, intrathecal, intralesional, intranasal, rectal, vaginal, or by inhalation. By "co-administer" it is meant that an anticancer drug is administered at the same time, just prior to, or just after the administration of a second drug (*e.g.,* another anticancer drug, a drug useful for reducing the side-effects associated with anticancer drug therapy, a radiotherapeutic agent, a hormonal therapeutic agent, an immunotherapeutic agent, *etc.).*

A therapeutically effective amount of an anticancer drug may be administered repeatedly, *e.g.,* at least 2, 3, 4, 5, 6, 7, 8, or more times, or the dose may be administered by continuous infusion. The dose may take the form of solid, semi-solid, lyophilized powder, or liquid dosage forms, such as, for example, tablets, pills, pellets, capsules, powders, solutions, suspensions, emulsions, suppositories, retention enemas, creams, ointments, lotions, gels, aerosols, foams, or the like, preferably in unit dosage forms suitable for simple administration of precise dosages.

As used herein, the term "unit dosage form" refers to physically discrete units suitable as unitary dosages for human subjects and other mammals, each unit containing a predetermined quantity of an anticancer drug calculated to produce the desired onset, tolerability, and/or therapeutic effects, in association with a suitable pharmaceutical excipient *(e.g.,* an ampoule). In addition, more concentrated dosage forms may be prepared, from which the more dilute unit dosage forms may then be produced. The more concentrated dosage forms thus will contain substantially more than, *e.g.,* at least 1, 2, 3, 4, 5, 6, 7, 8, 9, 10, or more times the amount of the anticancer drug.

Methods for preparing such dosage forms are known to those skilled in the art *(see, e.g.,* REMINGTON'S PHARMACEUTICAL SCIENCES, 18TH ED., Mack Publishing Co., Easton, PA (1990)). The dosage forms typically include a conventional pharmaceutical carrier or excipient and may additionally include other medicinal agents, carriers, adjuvants, diluents, tissue permeation enhancers, solubilizers, and the like. Appropriate excipients can be tailored to the particular dosage form and route of administration by methods well known in the art (*see, e.g., REMINGTON'S PHARMACEUTICAL SCIENCES, supra*).

Examples of suitable excipients include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline, syrup, methylcellulose, ethylcellulose, hydroxypropylmethylcellulose, and polyacrylic acids such as Carbopols, *e.g.,* Carbopol 941, Carbopol 980, Carbopol 981, *etc.* The dosage forms can additionally include lubricating agents such as talc, magnesium stearate, and mineral oil; wetting agents; emulsifying agents; suspending agents; preserving agents such as methyl-, ethyl-, and propyl-hydroxy-benzoates (*i.e.,* the parabens); pH adjusting agents such as inorganic and organic acids and bases; sweetening agents; and flavoring agents. The dosage forms may also comprise biodegradable polymer beads, dextran, and cyclodextrin inclusion complexes.

For oral administration, the therapeutically effective dose can be in the form of tablets, capsules, emulsions, suspensions, solutions, syrups, sprays, lozenges, powders, and sustained-release formulations. Suitable excipients for oral administration include pharmaceutical grades of mannitol, lactose, starch, magnesium stearate, sodium saccharine, talcum, cellulose, glucose, gelatin, sucrose, magnesium carbonate, and the like.

In some embodiments, the therapeutically effective dose takes the form of a pill, tablet, or capsule, and thus, the dosage form can contain, along with an anticancer drug, any of the following: a diluent such as lactose, sucrose, dicalcium phosphate, and the like; a disintegrant such as starch or derivatives thereof; a lubricant such as magnesium stearate and the like; and a binder such a starch, gum acacia, polyvinylpyrrolidone, gelatin, cellulose and derivatives thereof. An anticancer drug can also be formulated into a suppository disposed, for example, in a polyethylene glycol (PEG) carrier.

Liquid dosage forms can be prepared by dissolving or dispersing an anticancer drug and optionally one or more pharmaceutically acceptable adjuvants in a carrier such as, for example, aqueous saline (*e.g.,* 0.9% w/v sodium chloride), aqueous dextrose, glycerol, ethanol, and the like, to form a solution or suspension, *e.g.,* for oral, topical, or intravenous administration. An anticancer drug can also be formulated into a retention enema.

For topical administration, the therapeutically effective dose can be in the form of emulsions, lotions, gels, foams, creams, jellies, solutions, suspensions, ointments, and transdermal patches. For administration by inhalation, an anticancer drug can be delivered as a dry powder or in liquid form via a nebulizer. For parenteral administration, the therapeutically effective dose can be in the form of sterile injectable solutions and sterile packaged powders. Preferably, injectable solutions are formulated at a pH of from about 4.5 to about 7.5.

The therapeutically effective dose can also be provided in a lyophilized form. Such dosage forms may include a buffer, e.g., bicarbonate, for reconstitution prior to administration, or the buffer may be included in the lyophilized dosage form for reconstitution with, *e.g.,* water. The lyophilized dosage form may further comprise a suitable vasoconstrictor, *e.g.,* epinephrine. The lyophilized dosage form can be provided in a syringe, optionally packaged in combination with the buffer for reconstitution, such that the reconstituted dosage form can be immediately administered to a subject.

A subject can also be monitored at periodic time intervals to assess the efficacy of a certain therapeutic regimen. For example, the activation states of certain signal transduction molecules may change based on the therapeutic effect of treatment with one or more of the anticancer drugs described herein. The subject can be monitored to assess response and understand the effects of certain drugs or treatments in an individualized approach. Additionally, subjects who initially respond to a specific anticancer drug or combination of anticancer drugs may become refractory to the drug or drug combination, indicating that these subjects have developed acquired drug resistance. These subjects can be discontinued on their current therapy and an alternative treatment prescribed in accordance with the methods of the present invention.

### VIII. Examples

The following examples are offered to illustrate, but not to limit, the claimed invention.

The examples of PCT Publication No. WO 2011/008990 (PCT/US2010/042182), filed July 15, 2010, and US 2012-0270745.

### Example 1. Functional Profiling Of Multiple Signal Pathway Proteins In Gastric Cancer Patients.

### ABSTRACT

Although gastrectomy is the only curative treatment in gastric cancer (GCA) patients, a high recurrence rate ranging from 40 ∼ 60% following curative surgery still accounts for poor overall survival. In order to further improve survival, there is an urgent need to identify reliable molecular prognostic markers for survival or recurrence following adjuvant chemoradiation therapy, which will evolve to the development of patient-tailored treatment strategies. Hence, we have developed a multiplexed immunoassay platform for functional profiling of the signal transduction pathway proteins using a multiplexed immuno-microarray platform. Here we report the functional profiling of HER1, HER2, HER3, cMET, PI3K, and IGF1R in GCA.

### INTRODUCTION

Metastatic gastric cancer remains a therapeutic challenge for medical oncologists due to poor prognosis. HER2 amplification and/or overexpression are present in many gastric cancer cases. In quest of a novel therapeutic target for gastric cancer, trastuzumab has shown survival benefit in patients with HER2(+) metastatic GCA. However, HER2 (+) patients often do not respond to trastuzumab due to either primary or acquired resistance. In our studies, we analyzed expression/activation levels of proteins in signal transduction pathway in order to survey the prevalence of targetable pathway proteins for rational selection of inhibitors or combinations of agents. Here we report the expression/activation-profiling of HER2, p95-HER2 and other pathway proteins with transactivational potential for HER2 and other parallel pathways in order to lay the groundwork for future clinical trials incorporating HER2 targeted agents in GCA.

### METHODS

**Collaborative Enzyme Enhanced Reactive-immunoassay (CEER™):** Target proteins present in tissue-lysates are bound to specific capture antibodies printed on nitrocellulose surface and unbound non-target proteins are removed from the slide. The enzymatic interaction between one of detector antibodies against alternate epitope on captured target-protein are conjugated with Glucose Oxidase (GO) and the other detector antibodies specific to phosphorylated sites on target-protein or another non-overlapping epitope conjugated with HRP results in signal generation/amplification *(see,* Figure 1).

**Slide Printing:** Capture antibodies for each specific target protein are printed in triplicates in serial dilution. Each slide contains cell line controls for standard curve generation for accurate quantitation of samples on each slide run.

**Clinical Samples:** The flash frozen gastric cancer tissues were from patients with localized, histologically confirmed GCA (Samsung Medical Center). The flash frozen tissue samples were lysed in 100 µL of lysis buffer, and the resulting lysates were stored at -80°C before subsequent analysis.

### RESULTS

While 7% of patients in this cohort expressed high levels of HER2 (31/447) by IHC, approximately 12% of patients showed significant level of expression of HER2 by CEER™ assay. Truncated HER2 (or p95HER2) was detected in 5.6% (25/447) of GCA patients as shown in Figure 2. Varying levels of HER3 expression were found in approximately 40% of patients.

Higher levels of HER1 were found in samples with lower HER2 expression. The HER3-P level showed high degree of correlation with HER3-T and PI3K activation. The expression of IGF1R in this cohort was relatively lower in most samples. The p95HER2 levels were prominent in samples with higher HER2 expression, however evidence of p95HER2 activation had a wider distribution. Co-activation of HER1 was often observed for patients with cMET activation (*see,* Figure 3).

### CONCLUSION

The treatment of recurrent or metastatic GCA may be optimized based on disease-specific functional profiling of biomarkers, leading to patient-tailored treatment strategies. A combined targeting HER1 and cMET in sub-GCA may benefit patients with a co-activation pattern. As CEER™ can be performed even with a limited amount of tissue, the incidence of alteration in transduction pathway proteins during the course of a therapeutic regimen may guide clinicians in devising better treatment strategies and support clinical development of evolving or combined/sequenced targeted therapies.

### Example 2. Comprehensive Profiling Of Pathway Proteins In A Panel Of Gastric Cancer Cell Lines.

### ABSTRACT

With the advent of molecularly targeted therapy in cancer care, profiling of pathway proteins for targeted drug is of utmost importance. However, one of the causes for difficulties in predicting response in a targeted regimen is likely a consequence of the limited knowledge of the abnormal changes in proteins in a signal transduction pathway of a tumor. A deep level of understanding of the mechanism by which abnormal activation of pathway proteins causes a neoplastic phenotype in tumor cells and how the targeted interruption leads to clinical response would be a common goal for clinicians and drug developers. Here we report the comprehensive mutation profile along with pathway protein expression and/or activation (HER1, HER2, p95HER2, HER3, cMET, IGF1T, cKIT, Shc, PI3K, AKT, ERK, Src, FAK, PTEN and other signal proteins) and their modulation in response to targeted drug treatments. The mechanism of resistance in sub-classes of GCAs to various inhibitors can be utilized for designing rational treatment options with targeted drug selection, combination, and/or sequencing.

### INTRODUCTION

Preclinical cellular response profiling of tumor samples has become a cornerstone in the development of novel cancer therapeutics. Hence, we applied the Collaborative Enzyme Enhanced Reactive-immunoassay (CEER™) multiplexed protein microarray system to functionally profile signal transduction proteins in 30 GCA cell lines well characterized for their genetic alternations. The combination of genetic and functional pathway characterization in these cell lines allowed diverse and distinct sub classification of GCAs and may provide a road map for clinical studies and drug development.

### METHODS

**CEER™:** Target proteins present in tissue-lysates are bound to specific capture antibodies printed on a nitrocellulose surface and unbound non-target proteins are removed from the slide. The enzymatic interaction between one of the detector antibodies against an alternate epitope on a captured target protein conjugated with Glucose Oxidase (GO) and the other detector antibodies specific for phosphorylated sites on a target protein or another non-overlapping epitope conjugated with HRP results in signal generation/amplification *(see,* Figure 1).

**Multiple Drug Effect Analysis:** ∼ 1 X10⁴ cells were plated in 24-well plate and grown in appropriate growth media. Lapatinib, cMET inhibitor or both agents (combo) were added at 1 µM concentration for 4 hrs and lysed for subsequent pathway profiling.

### RESULTS

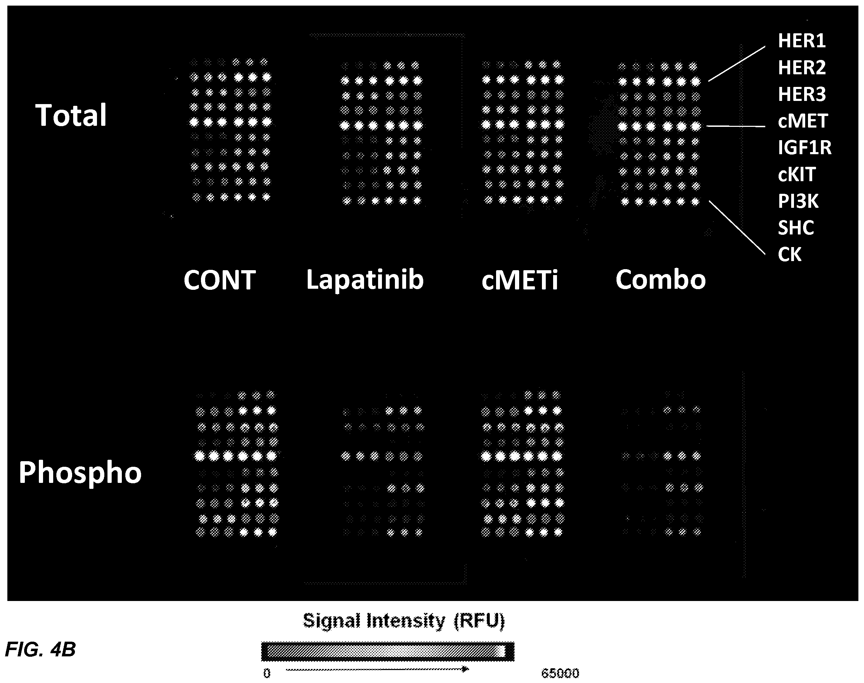

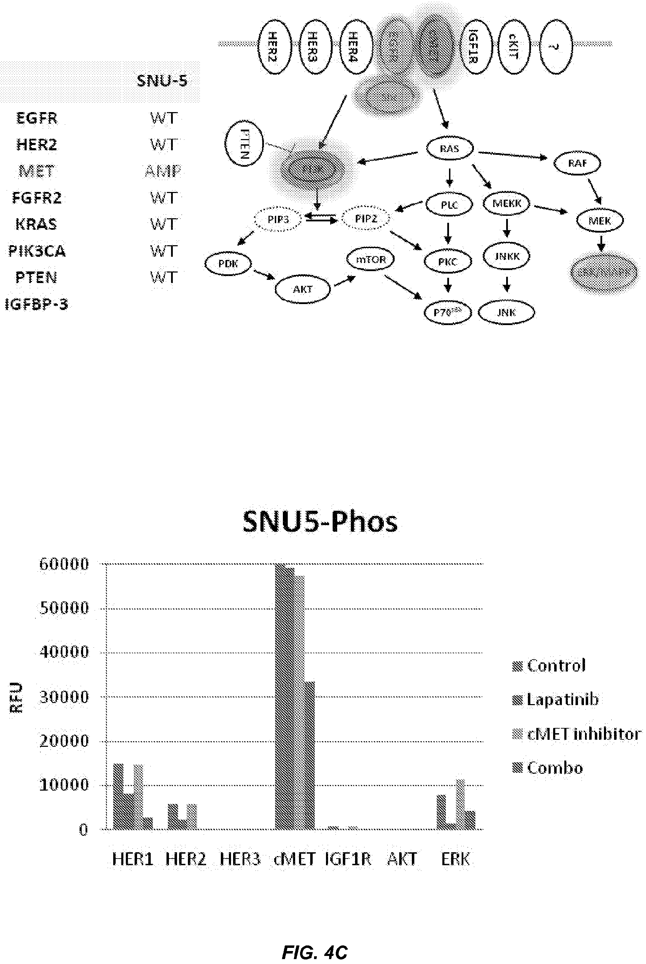

Figure 4A illustrates a comprehensive expression/activation profile of 30 GCA cell lines. Figure 4B provides exemplary immunoarray images for the SNU5 cell line pathway profile. Figures 4C-G provide a summary of baseline pathway profiles for cell lines SNU5 (C), SNU484 (D), SNU1 (E), MKN45 (F), and KATOIII (G) and their modulation post-drug treatment. Degree of activation is differentiated with highlights with different intensities.

### CONCLUSION

The treatment of recurrent or metastatic GCA may be optimized based on disease-specific functional profiling of biomarkers leading to patient-tailored treatment strategies. In addition, the incidence of alteration in transduction pathway proteins during treatment can be readily determined by the CEER™ platform which requires limited amounts of tumor samples *(e.g.,* FNA, CTCs, *etc.)* to better guide clinicians to combine or sequence appropriate agents.

### Example 3. Tyrosine Phosphorylation Profiling Identifies Concomitantly Activated Pathways in Gastric Cancer.

The study presented in this example illustrates that gastric cancers can be segregated based on the phosphorylation profiles of key growth factor receptor signaling pathways that are active in this cancer type.

### SUMMARY

Currently, trastuzumab is the only known targeted therapy beneficial in ∼20% of metastatic gastric cancers. An increased understanding of activated RTKs in these heterogeneous cancers can significantly advance targeted therapeutic development; however, it is impeded by our inability to interrogate phosphorylation networks in clinical specimens. Utilizing a novel immunoassay, CEER™, we demonstrate the presence of truncated HER2 variants in advanced gastric cancers for the first time. We also demonstrate the presence of concomitantly activated RTKs that influence disease-free survival times of recurrent gastric cancers. Hierarchical clustering of activated RTKs reveals co-clustering of samples expressing activated HER1:c-MET, HER2:HER3 and IGF1R-PI3K in gastric cancers. *In vitro* studies in cells and primary tumor cells demonstrate that differentially regulated RTK patterns can guide therapeutic targeting. Overall, our study has strong implications for drug development and therapeutic monitoring in gastric cancers.

### SIGNIFICANCE

Except for a subset of HER2(+) cancers, there is no globally accepted treatment consensus for the majority of advanced gastric cancers due to the lack of molecular markers for guiding efficacious treatment approaches. Herein, using a novel clinical diagnostics assay, we present evidence in 434 advanced stage gastric cancer samples, rare CTCs and ascites-derived tumor cells that concomitantly activated RTKs distinguish distinct subsets of gastric cancers. The differential RTK activation patterns correlate with postoperative disease-free survival. Ramifications of targeted phosphorylation analysis directly on clinical tissues are significant, including improving our understanding of unique molecular drivers of gastric cancer subtypes, aiding in the identification of novel prognostic markers, treatment paradigms, and longitudinal monitoring of therapeutic response or disease progression after drug administration.

### INTRODUCTION

With the advent of the molecularly targeted therapy era in the field of oncology, many oncologists have experienced unexpected and dramatic responses to molecular targeted agents in refractory, metastatic cancer patients who would otherwise have been on supportive care. However, patient selection criteria for targeted therapeutics is presently imperfect. Even patients selected based on their target profiles often do not respond to targeting agents or develop resistance after having a dramatic initial response. Major issues which hinder from enhancing therapeutic benefit with targeted agents are: (1) concomitant and redundant pathway activation in subgroups of patients; (2) development of resistance by activation of alternate signaling events which bypass the originally targeted protein(s) via pathway cross-talk; (3) alteration in molecular and pathologic features as cancer metastasizes and progresses over the course of anti-cancer treatment; (4) limited availability of re-biopsy during or after treatments; and (5) limitation of companion diagnostics for targeted drug to identify potential drug resistant mechanisms to address the "evolving" disease.

Approximately half of the tyrosine kinase complement of the human kinome is implicated in human cancers and provides targets for cancer treatment as well as biomarkers for patient selection (Blume-Jensen and Hunter, 2001; Hochgrafe et al., 2010). Moreover, previous studies have shown coactivation of receptor tyrosine kinases (RTKs) in subsets of several cancers such as glioblastoma multiforme (Stommel et al., 2007), breast cancer (Hochgrafe et al., 2010; Liu et al., 2011; Xu et al., 2011), lung cancer (Engelman and Janne, 2008; Engelman and Settleman, 2008), head and neck cancer (Xu et al., 2011) and gastric cancer (Liu et al., 2011). We have developed a proximity-based immunoassay which utilizes the formation of a unique immuno-complex requiring the co-localization of two detector enzyme-conjugated-antibodies once the target proteins are captured on the microarray, Collaborative Enzyme Enhanced Reactive-immunoassay (CEER™, as shown in Figure 5). This assay format enables us to profile RTKs and downstream pathway protein expressions and activations in a very sensitive manner to the single cell level (sensitivity of about 100 zeptomoles) and hence suitable for multiplexed analysis of redundant pathways with concomitant pathway activation (or activation potential).

Gastric cancer is the leading cause of cancer death worldwide with the incidence of 18.9/100,000 per year and the mortality rate of 14.7/100,000 per year (Cunningham et al., 2005) and is the most common malignancy in Korea (Bae and Park, 2002). Metastatic gastric cancer remains a therapeutic challenge for medical oncologists due to poor prognosis. Currently, trastuzumab is the only active targeted agent which has been proven to be efficacious for gastric cancer in a randomized phase III trial (Bang et al., 2010). Other known activated pathway proteins in gastric cancer include the human epidermal growth factor receptor (HER) family, mesenchymal epithelial transition factor or hepatocyte growth factor (HGF) receptor (cMET), phosphatidylinositol 3-kinase (PI3K) and insulin-like growth factor 1 receptor (IGF1R). Thus, we have used the multiplexed CEER™ platform to determine levels of activated RTKs in 434 fresh frozen gastric cancer tissues and attempted to categorize gastric cancer patients into potential subgroups (HER1, HER2, truncated variants of HER2, p95HER2, HER3, cMET, PI3K, and IGF1R). Based on their pathway protein activation patterns, we have observed multiple signal protein activation in a subgroup of tumors, and we hypothesized that redundant pathway activation inputs would lead to residual downstream signaling, thus limiting the anti-tumor efficacy of monotherapy targeted against single RTK. We conducted proof of principle experiments to investigate the anti-tumor effect of HER1/2 inhibitor + cMET inhibitor in cMET and HER co-activated gastric cancer cell lines. Lastly, we developed a potentially powerful tool which may enable the clinicians to monitor baseline and evolving alterations in RTK activation profile over treatment using circulating tumor cells and malignant cells isolated from peritoneal fluid (ascites). Taken together, our study provides evidence for concomitant RTK activation in gastric cancer human tissues and we established a clinical tool to monitor RTK activation as cancer evolves during treatment and progression.

### RESULTS

### Patient characteristics

Characteristics of 434 patients included in the analysis are provided in Table 1. All patients received gastrectomies with D2 lymph node dissection. Of 434 patients, 242 (55.8%) patients received subtotal gastrectomy with D2 lymph node dissection. According to AJCC 2002 staging system, 86 patients had pathologic stage I, 116 stage II, 126 stage III and 106 stage IV (35 patients with metastatic M1). At the time of analysis, 226 patients were dead and 237 patients had documented recurrence. For pathology, 70 patients had signet ring cell carcinoma. The 5-year overall survival rate was 52.4% and 5-year disease free survival rate was 50.0%. All primary gastric cancer tissues were procured at the time of surgery and immediately snap frozen for future molecular analysis.

**Table 1: Patient characteristics of the gastric cancer clinical sample set.**

| **Characteristics** | **N=434** |
|---|---|
| **Age (yr)** | |
| Median, range | 61,26 - 87 |

| **Sex** | |
|---|---|
| Male | 285(65.7%) |
| Female | 149(34.3%) |

| **Type of gastrectomy** | |
|---|---|
| Subtotal gastrectomy | 242(55.8%) |
| Total gastrectomy | 192(44.2%) |
| | |

| **Location of tumor** | |
|---|---|
| Cardia | 12(2.8%) |
| Body | 142(32.7%) |
| Antrum | 280(64.5%) |

| **Grade** | |
|---|---|
| Well to moderately differentiated tubular | 138(31.8%) |
| Poorly differentiated tubular | 186(42.9%) |
| Signet ring cell | 70(16.1%) |
| Mucinous | 32(7.4%) |
| Papillary | 2(0.5%) |
| Hepatoid | 2(0.5%) |
| Others. | 4(0.9%) |

| **Lauren type(N=397**) | |
|---|---|
| Intestinal | 154(38.8%) |
| Diffuse | 225(56.7%) |
| Mixed | 18(4.5%) |

| **Lymphovascular invasion** | |
|---|---|
| Present/identified | 256(59.0%) |
| Not present/Not identified | 178(41.0%) |

| **AJCC stage (2002)** | |
|---|---|
| I | 86(19.8%) |
| II | 116(26.7%) |
| III | 126(29.0%) |
| IV | 106(24.4%) |

### Gastric cancers express HER2 and its truncated variant, p95HER2

Superior efficacy of the HER2 targeting antibody, trastuzumab, in combination with chemotherapy has paved the way for molecularly targeted therapies in gastric cancers. However, not all HER2 expressing gastric cancer patients selected by immunohistochemistry and fluorescent *in situ* hybridization respond to this treatment, highlighting the need for more predictive biomarkers. We performed an in-depth analysis of the HER2 status including its activated state in the gastric cancer specimens obtained from 434 patients.

The HER2 status of our gastric cancer sample cohort was determined by standard IHC HercepTest followed by a FISH analysis for those tissues with IHC score of 2+. In our study, HER2 positive (HER2(+)) samples are defined as samples with an HER2-IHC score of 3+ or 2+ in addition to a *HER2* gene amplification as determined by FISH. HER2 negative (HER2(-)) samples include specimens either with a 2+ HER2-IHC score without a *HER2* gene amplification or samples with a 1+/0 HER2-IHC score. Based on these definitions that are specific for gastric cancers (Hofmann et al., 2008), 50 of 434 (11.5%) patients were confirmed to be HER2(+) by immunohistochemistry and/or FISH in our patient cohort (Table 2). In agreement with other reports (Grabsch et al., 2010; Hofmann et al., 2008; Tanner et al., 2005), HER2 expression varied with the Lauren histotype with a higher number of HER2(+) samples (36 out of 50 (72%)) observed in the intestinal cancers compared to the diffuse or mixed type gastric cancers. It is important to note that the majority of the 'HER2 negative' samples still express total HER2 albeit at distinctly lower levels than the HER2 positive patient population (Figure 6A). When HER2(+) gastric cancer samples were reanalyzed for HER2 expression using the IHC guideline specific for breast cancers HER2-IHC, 78% (39/50) remained as 3+ or 2+, but the rest 22% (11/50) were scored as 1+ (Table 2).

As immunohistochemistry and FISH technologies do not provide a read out for the activated status of HER2 and may miss low HER2 expression, we next analyzed the expression levels of total and phosphorylated HER2 protein using the immuno-microarray based CEER™ assay (Kim et al., 2011) (Figure 5) in this patient sample set. We had previously developed algorithms for converting the RFU values obtained in a CEER™ assay into Computed Units (CU), a standard functional unit based on cell line controls with known HER2 expression and activation (Kim et al., 2011). As predicted, substantially higher HER2 expression levels were observed by CEER™ in IHC/FISH HER2(+) tumors than the HER2(-) tumors as shown in Figure 6A (with a mean value of 77.9 CU vs. 4.3 CU, p-value 8.18E-10). Moreover, CEER™ revealed a significant level of heterogeneity in HER2 expression within the HER2(+) tumor population, a trait that has been frequently reported in gastric cancers due to the focal or "clonal" nature of HER2 cellular distribution pattern (Hofmann et al., 2008). This also results in only a moderate degree of concordance between the IHC and FISH readouts for HER2 (ToGA trial, 87.5%) leading to the complexity in developing HER2 diagnostics for gastric cancers. In our analysis, 64% (32/50) of IHC/FISH HER2(+) cancers also showed HER2 expression by CEER™. Interestingly, ∼20% of the HER2(-) cancers were also positive for HER2 expression by CEER™. Of note, we have previously compared HER2 expression via IHC and CEER™ in 293 breast cancer patients which initially showed 83% concordance (Figure 7), however upon adjusting the IHC readouts with a HER2 western blot (WB)-based expression, the comparison revealed a 96% concordance between CEER™ and WB-adjusted IHC (Kim et al., 2011).

We explored the presence of alternate HER2 variants in gastric cancers. One of the important mechanisms for trastuzumab resistance in 70-80% of HER2 overexpressing breast cancers is the accumulation of truncated forms of the HER2 receptor, p95HER2, which lack the extracellular trastuzumab-binding domain (Scaltriti et al., 2007). We developed a highly sensitive and specific CEER™-based assay to specifically analyze total p95HER2 in gastric cancers. This was accomplished by using a triple antibody mediated immuno-microassay platform in tumor tissue lysates enriched for truncated HER2 variants after immuno-magnetic depletion of full length HER2 (Figure 8). The total p95HER2 assay readout was relative to the signals generated from the standard curves generated using a control cell lysate from the BT474 breast cancer cell line. Using the CEER™-based p95HER2 assay platform, truncated forms of HER2 were specifically detected, in addition to full length HER2, in gastric cancers for the first time. p95HER2 expression was analyzed in a subset of tumor samples from the HER2(+) group (31 samples) and HER2(-) group (27 samples) that demonstrated a significant full length HER2 expression as detected by CEER™. One sample in the HER2(-) group could not be accurately analyzed due to technical issues. The incidence of p95HER2 in our gastric cancer patient cohort was approximately 77% (in 24 out of 31) in the HER2(+) specimens. Similar to full-length HER2 expression, the majority of truncated HER2 expression (79% of p95HER2 expressed in HER2(+)) was observed in intestinal-type gastric cancers. p95HER2 expression was also detected in a small percentage of tumors (2.6% or 10/384 overall) that were HER2(-) by IHC/FISH but which showed a significant CEER™-based HER2 expression. These comprised ∼37% (10/27) of a subset of HER2(-) samples that were analyzed for p95HER2. However, the average expression of p95HER2 in HER2(+) gastric cancer samples was significantly higher than that observed in HER2(-) samples (5083.6 CU in HER2(+) vs 437.0 CU in HER2(-), p-value=1.27e-05). A cut-off value of 500 CU was used to score for p95HER2 positivity (as shown in Figure 6B) per 20 µg of tissue analyzed. Taken together, our data indicate that a comprehensive analysis of both full-length and truncated HER2 expression provides value to the further development of HER2 targeting strategies in gastric cancers.

### Heterogeneity in pathway activation profiles in gastric cancers: coactivated signaling path ways

Having determined the presence of HER2 and its truncated isoforms in gastric cancer, we explored the presence of total and phosphorylated (activated) forms of several signaling molecules which are drug targets. These included the RTK members of the HER kinase axis (HER1, HER2 and HER3), c-MET, IGF1R and PI3K. Representative images of multiplexed CEER™ pathway-arrays are shown in Figure 9A. In our study panel, tumors from 202 patients (46.5%) had no detectable activation of the RTKs tested in our study panel. Conversely, the pathway activation patterns for the rest of the patients, as depicted in a pathway clustering analysis (Figure 9B), varied widely with some gastric cancers demonstrating concomitant activation of multiple RTKs such as HER2/HER3, HER1/2/3, HER1/3/c-MET or HER3/c-MET while others were phosphorylated only on a single protein. The tumor content of all the analyzed samples was at least 70% based on their histological examination. However, the pan-cytokeratin expression was distinctly variable, indicating heterogeneity in the epithelial content of gastric cancers. Based on this profiling, we categorized the 434 gastric cancer sample cohort according to their RTK activation signatures and HER2 status.

***HER axis in gastric*** ***cancers (Table 3**)**:*** Of all the RTKs that we analyzed, we found that at least one receptor member of the HER axis was activated in 41 % of the gastric cancer samples in our data set. Approximately 66% of the HER2(+) and ∼38% of the HER2(-) cancers, demonstrated an activation of a HER kinase receptor member. Furthermore, ∼36% of the samples with an activated HER kinase receptor did not display a concomitant activation of c-MET or IGF1R pathways. HER2 and HER3 were phosphorylated in higher percentages of HER2(+) gastric cancer patients (50% and 36%) as compared to the HER2(-) cancers where they were activated in ∼22% and ∼24% samples, respectively. Phosphorylated HER1 did not appear to have such a preference for HER2 positive cancers and was equivalently activated in both HER2 positive and negative gastric cancers (26% in HER2(+) and 25% in HER2(-)). Histologically, a majority of the HER2 positive, intestinal type gastric cancers expressed an activated HER2 (in 22/36 or ∼61%) followed by HER3 (in 13/36 or ∼36%) with an overall 36% of intestinal type gastric cancers expressing an activated HER2 pathway. In general, HER2 activation was more concentrated in intestinal type (35.7%) and mixed type cancers (33.3%) as compared to the diffuse-type cancers (19.1%). In contrast, activated HER1 was observed in mostly intestinal-type (24.7%) and diffuse type (25.8%) gastric cancers. Activated HER3 was expressed equivalently among all three gastric cancer histotypes.

As the signaling function of the HER kinase axis is dependent upon activated receptor dimerization, we looked at the various pairs of activated HER members. Co-activation of HER2 with HER3 was preferred in HER2 positive cancers (13/50 or 26%) with 7/13 HER2:HER3 activated samples without a HER1 co-activation. On the other hand, HER2 negative gastric cancers did not demonstrate a preference for any specific HER kinase dimer pair with all three possible activated dimer pairs (HER1 :HER2, HER1 :HER3 and HER2:HER3) expressed in ∼15% each of HER2(-) samples. Triple activation of HER1/2/3 receptors was observed in 11.5% of gastric cancers with similar distribution between HER2(+) and HER2(-) cancers.

We also looked at the status of the activated HER axis receptor members with respect to the p95HER2 expression in the samples. p95HER2(+), HER2(+) gastric cancers had a predilection for activated full-length HER2 (expressed in 16/24 or ∼67% of p95HER2(+), HER2(+)) as compared to activated HER1 (expressed in 5/24 or ∼21% of p95HER2(+),HER2(+)) or activated HER3 (expressed in 9/24 or ∼37% of p95HER2(+),HER2(+)). However, the p95HER2(+), HER2(-) gastric cancers had no such preference with 50% (5/10) of p95HER2(+), HER2(-) samples demonstrating an activation of all three HER kinase axis receptor members.

***c-MET activated gastric cancers (Table 4):*** Similar to HER1, phosphorylation of c-MET was equivalently distributed between HER2(+) (22%) and HER2(-) (25%) gastric cancers. Furthermore, activated c-MET distribution based on the Lauren histotype was similar in intestinal (31.2%) and diffuse-type (24.4%) gastric cancers. None of the mixed type gastric cancers demonstrated the presence of activated c-MET in our sample set.

The majority of c-MET activated gastric cancer samples (∼71% or 77/108) demonstrated a concomitant activation of HER kinase receptor members. Therefore, we further investigated the preferred HER axis receptors that cross-talk with the c-MET pathway in gastric cancers. Activated c-MET and p95HER2 expression co-existed in 5/24 and 1/10 p95HER2 expressing, HER2(+) and HER2(-) samples, respectively. Overall, c-MET was preferentially co-activated with HER1 (in 66/434 or 15.2%) as compared to HER2 (10.1%) or HER3 (9.7%). The same trend was observed in HER2(-) gastric cancers where 15.4% of cancers demonstrated a c-MET-HER1 co-activation. In fact, 13/66 gastric cancers with c-MET-HER1 co-activation did not demonstrate a co-activation of HER2 or HER3. These observations suggest a possible cross-talk between the c-MET and HER1 signaling pathways in gastric cancers. c-MET activation never co-existed with HER3 activation unless HER1 and/or HER2 were also phosphorylated in the same sample. Approximately 7% of gastric cancer samples demonstrated a co-activation of all three HER axis receptor members with c-MET.

***IGF1R activated gastric cancers (Table 5):*** Similar to HER3 activation, the signaling pathway driven by the IGF1 receptor was active in higher percentage of HER2(+) cancers (30%) as compared to the HER2(-) cancers (24.5%). Furthermore, as observed with phosphorylated HER3, activated IGF1R was equivalently distributed among the three histological gastric cancer subtypes; i.e., 26% of intestinal cancers, 24% of diffuse and 28% of mixed type gastric cancers. This distribution lead us to investigate if there was an IGF1R-HER3 co-activation in our sample set. IGF1R was indeed maximally co-activated with phospho-HER3 especially in HER2(+) cancers where 22% or 11/50 HER2(+) samples demonstrated an IGF1R-HER3 co-activation. However, in HER2(-) cancers, percentage of IGF1R co-activation with HER3 (in 51/384 samples or 13.8%) was similar to IGF1R co-activation with HER1 (in 53/384 samples or 13.3%). IGF1R:HER2 co-activation (in 45/384 samples or 11.7%) followed close behind. Overall, 34/434 gastric cancer samples demonstrated co-activation of all members of the HER kinase axis with IGF1R of which 28 samples also demonstrated a c-MET co-activation. However, c-MET was rarely co-activated with IGF1R in the absence of a HER kinase receptor member co-activation. Furthermore, c-MET:IGF1R co-activations were primarily observed in HER2(-) gastric cancers. Few p95HER2(+) samples demonstrated an IGF1R activation (7/24 HER2(+) samples and 3/10 HER2(-) samples).

The gastric cancer samples were clustered into a heatmap based on the decile-based activation profiles of the six evaluated markers in this study. The clustering analysis was performed to confirm the aforementioned RTK activation correlation patterns in gastric cancers (Figure 9B). The analysis demonstrated that c-MET:pHER1, pHER2:pHER3 and IGF1R:PI3K activation patterns are closely correlated in our sample set with the c-MET-pHER1 cluster forming a distinct subset. This was in agreement with the RTK co-activation patterns that we have previously observed. Further analysis of signaling pathways that are resident downstream of these analyzed RTKs such as PI3K revealed that in the unselected gastric cancer population, PI3K activity was maximally observed with IGF1R co-activation (77/108 samples or 71.3%) followed by HER3 co-activation (71/108 samples or 65.7%). This is in agreement with the clustering analysis and reported studies further strengthening the validity of the CEER™ assay.

In summary, our comprehensive analysis of phosphorylated HER kinase axis receptor members, c-MET and IGF1R revealed that gastric cancers are significantly heterogeneous with respect to the activity of the analyzed RTKs and can in fact be segregated into distinct subclasses based on their RTK activation profiles. Moreover, concomitant signaling pathways are highly prevalent in gastric cancers rather than any one signal. Such molecular segregations can help guide prognostic and therapeutic studies for gastric cancer subtypes.

### Sample segregation based on CEER™-based pathway activation profiles correlates with post-operative disease-free survival

We investigated the impact of CEER™-based phosphorylation profiling on clinical prognosis of gastric cancers. Disease-free patient survival post-curative surgery was analyzed after segregating the patients into groups based on the signaling pathway activation profiles of their tumor samples obtained at the time of surgery. Initially, we analyzed the disease-free survival differences between patients with HER2(+) tumors versus patients with HER2(-) tumors. HER2(+) tumors are mostly of an intestinal histotype that distinctly respond to anti-HER2 therapies. Therefore, we investigated if the recurrence times of HER2(+) tumors may differ from those of HER2(-) gastric cancers. However, no such differences were observed even after samples were adjusted for their tumor stage. As intuitively expected, median disease-free survival times of gastric cancer patients, both with HER2(+) and HER2(-) tumors, progressively decreased with an increasing tumor stage from stage I to stage IV.

Since only a subset of both HER2(+) and HER2(-) gastric cancers demonstrated an activation of the RTKs analyzed in our study, we reasoned that the activated RTKs may influence the progression of these tumors. Therefore, we analyzed the survival differences between patients with no RTK activation versus patients with ≥1 RTK activation as determined by the CEER™ assay. No significant differences in survival were observed between the two groups until the tumor stages were also taken into account. As GCA patients with stage I disease typically show extreme benefit from curative surgical resection while patients with stage IV disease are limited to palliative treatment options, we sought to investigate if degree of RTK activation had any impact on clinical outcome of patients with stages II and III disease. Once the samples were segregated based on their tumor stages, it was clear that stage II and III patients with ≥1 RTK activation demonstrated a worse progression free survival than those where none of the analyzed RTKs were activated. Median survival times of patients with tumor stages II+III and ≥1 RTK activation were significantly lower than those with 0 RTK activation (Figure 10A; Tumor stages II+III: 32.63 months for ≥1 RTK activation vs 76.53 months for 0 RTK activation (p=0.0318, hazard ratio=0.69)). Significant differences in median survival times were maintained in HER2(-) only samples as well (Figure 10B; Tumor stages II+III: 30.10 months for ≥1 RTK activation vs 68.13 months for 0 RTK activation (p=0.0190, hazard ratio=0.64)). These data clearly indicate that the RTKs analyzed in this study, in various combinations, influence disease-free survival in gastric cancers. An important observation from this analysis is that it is the activated status of the RTKs, and not simply the expression status, that contributes towards disease-free survival of patients post-curative surgery. As is clear from Figure 9A, a number of gastric cancer samples express various RTKs to high levels, however, in most samples only a subset or none of those RTKs are phosphorylated. For the survival analysis, only the samples with significant RTK activation were analyzed.

In an effort to explore the contribution of individual RTKs on progression-free survival, we concentrated on the HER1 and c-MET activated gastric cancer samples. The reasons for focusing on these RTKs were several. c-MET is gene amplified in a significant number of gastric cancers; however, single agent anti-MET inhibitors have failed to demonstrate a clinical benefit in these cancers (Jhawer et al., 2008). Others have reported a superior efficacy of c-MET and HER1 combination therapy in preclinical models of gastric cancers (Corso et al., 2010). In our own CEER™-based analysis of the gastric cancer clinical specimens, we have observed co-activation of c-MET and HER1 including co-clustering of such samples. This indicates that the overall signaling characteristics in p-HER1:p-cMET co-expressing gastric cancer samples may be distinctly different from samples that express p-cMET without an activated HER1. We compared the progression-free survival in p-HER1(-):p-cMET(+) and p-HER1(+):p-cMET(+) sample sets. There was a significant difference in median survival times of these cohorts in the overall gastric cancer sample set (Figure 11; 46.17 months for p-HER1(+):p-cMET(+) vs 82.80 months for p-HER1(-):p-cMET(+) (p=0.0184, hazard ratio=0.51). Similar results were observed in HER2(-) only samples (Figure 10C). Subsequently, the p-HER1(-):p-cMET(+) and p-HER1(+):p-cMET(+) cohorts were analyzed in tumor stage-adjusted HER2(-) sample sets. While stage I gastric cancers showed no significant difference in post-operative disease free survival for patients with different RTK activation status, p-cMET(+) stage I gastric cancer revealed significant differences with HER1 coactivated subtype. Co-activation of cMET and HER1 showed worse disease-free survival when compared to stage I patients with cMET activation alone (Figure 10D). Our data strongly indicate that the p-HER1(+):p-cMET(+) gastric cancer samples have a poorer prognosis and that there is value in determining signaling pathway characteristics in a stage-specific manner to determine therapeutic options for such patients. Gastric cancer patients with stage I disease need to be further characterized based on pathway activation status to identify candidates for more aggressive adjuvant treatment options post surgery.

### Therapeutic targeting can be guided based on the RTK profiles in gastric cancer model systems

Using the CEER™ assay, we categorized>16 gastric cancer cell lines based on their RTK activation profiles and the data were concordant with that observed in gastric cancer tissues (Hoe et al., 2011). Data are presented for a few such models. MET-amplified gastric cancer cells, SNU5 and SNU484, were identified to co-express appreciable levels of HER1 and c-MET proteins. However, phosphorylation profiling demonstrated the expression of pHER1 and pHER2 in addition to pMET in these cells as previously noted in a subset of gastric cancer clinical specimens. NCI-87 (N87) cells, that are well known as HER2 amplified gastric cancer cells, demonstrated expression of only pHER2 within the analyzed phospho-RTK panel. FGFR2-amplified KATO III cells showed low levels of pHER1, pHER2, pHER3 and pMET proteins as shown in Figure 12.

Based on the observation that heterogeneous RTK activation patterns are present in gastric cancer model systems as also seen in subsets of gastric cancer patients, we hypothesized that this information could be utilized to prospectively direct therapeutic decisions in gastric cancers. Therefore, we performed "proof of concept" experiments using the dual-HER1/2 tyrosine kinase inhibitor (TKI), lapatinib, and MET inhibitors, PHA-665,752 and foretinib. As shown in Figure 12, lapatinib abolished HER2 activation in NCI-87 cells without altering total HER2 levels but was unable to do so in MET-amplified SNU5 or SNU484 cells. On the other hand, MET inhibitor abrogated pMET in SNU5 and SNU484 cells. Interestingly, pHER1 and pHER2 were also partially blocked in MET-amplified cells with MET inhibition but not in NCI-87 cells which do not display any significant MET activity. This indicated that the MET and HER-kinase signaling pathways may be linked in MET-amplified gastric cancers. Furthermore, a combination treatment with lapatinib and foretinib for 4 hours completely blocked HER kinase and MET kinase activities including those of the downstream signaling effectors, PI3K and Shc, in MET-amplified SNU5 and SNU484 cells. This indicates that combinatorial therapeutic approaches may be more useful in such gastric cancers.

In order to examine the phenotypic effect of pathway inhibition on cancer cell growth inhibition, we performed cytotoxic assays using the respective therapeutic treatments. In concordance with the RTK profiling data, IC₅₀ for lapatinib in c-MET amplified SNU5 cells was > 1.0 µM/L whereas it was almost two orders of magnitude lower at 0.058 µM/L for PHA665,752. However, the IC₅₀ for lapatinib + PHA665,752 combination was even lower at 0.018 µM/L which coincided with maximal blockade of the HER kinase and MET signaling pathways with combination treatment (p= 0.01). These experiments indicate that a combined inhibition of HER and MET kinases may be more beneficial in this class of gastric cancers as opposed to single agent therapies. In contrast, KATO III cells, that showed incomplete inhibition of the signaling pathways in presence of the targeted therapeutics, demonstrated minimal cell growth inhibitions (IC₅₀ > 1.0 µM/L) with both single or combination drug treatments. Taken together, these studies in preclinical models clearly demonstrate that the CEER™ platform can simultaneously interrogate the status of multiple RTK activations in gastric cancers that can then be used to select and monitor therapeutic outcomes.

### Investigating the utility of CTCs and ATCs for studying RTK profiles in gastric cancer patients

As demonstrated in gastric cancer preclinical models, patterns of RTK activations as determined by the CEER™ technology can be utilized for guiding therapeutic decisions and monitoring treatment responses. However, follow-up tumor biopsy to monitor molecular alterations is not always available at the clinic. Therefore, we sought to set up an RTK evaluation platform using circulating tumor cells (CTCs) and ascites tumor cells (ATCs) that would be more readily available. The number of CTCs per patient varied widely as shown by varying levels of CK found by CEER™ assay and results from 4 such patients are shown in Figure 13A. Although only HER1 and HER2 expression and activation are shown, we observed heterogeneous patterns of other RTK activations in CTCs isolated from metastatic gastric cancer patients. In addition, we have successfully isolated malignant cells from ascites fluid, another source of noninvasive cancer cells in gastric cancer. In a drainage of 100 mL of ascites fluid, several magnitudes higher numbers of tumor cells were found (> 1 X 10³ to 1 X 10⁴) than typical yields of CTCs in 7.5 ml of blood. Heterogeneous RTK activation patterns as seen in gastric cancer CTCs were also found in ATCs isolated from gastric cancer patients.

Next, we determined if the signaling pathways in these tumor cells (CTCs and ATCs) would be responsive to ligand and/or drug perturbations. This could potentially provide valuable information regarding the functionality and drug responsiveness of the signaling pathways in CTCs/ATCs that may be indicative of the *in situ* cancers. Upon treatment with a combination of lapatinib and c-MET inhibitors, a dose dependent inhibition of phosphorylated HER1, HER2 and c-MET was seen in CTCs isolated from gastric cancer patients. A representative example is shown in Figure 13B. In this particular set of CTCs, we also observed a concomitant activation of IGF1R upon inhibitor treatment. The precise mechanism of this phenomenon and if this occurs in gastric cancers too is unknown at this time. Subsequently, EpCAM-positive ATCs were treated with cocktails of growth factors (EGF, HRG, HGF and IGF1) with or without lapatinib and PHA-665,752 cocktail at 37°C for 4 hrs. The platform was tested on several different ATC isolations. Representative examples of ATCs isolated from three different gastric cancer patients who demonstrated distinct activated pathways are shown (Figures 13C-E). Significant levels of p-HER1, p-HER2 as well as p-cMET were observed in all three sets of ATCs indicating that combination of lapatinib and PHA-665,752 may benefit these particular patients. Indeed this combination of targeted therapeutics was able to decrease the phosphorylations of these 3 targets albeit to varying degrees. Whether or not this drug combination would indeed translate into a clinical benefit for such patients remains to be seen. However, the profiling of other activated RTKs in ATCs indicated that HER3 and IGF1R were also activated to varying levels in some samples which could not be efficiently inhibited by the inhibitor cocktail. It is possible that these activated RTKs may influence the outcome of the tested therapeutics. In fact, one patient (005-116) demonstrated an increase in p-IGF1R with inhibitor treatment. Similarly, an analysis of the signaling molecules downstream to the activated RTKs (PI3K, SHC, Erk and AKT) revealed heterogeneity in terms of their activation and inhibition profiles upon ligand and drug treatments. Our data clearly demonstrates a non-invasive platform for the evaluation of signaling pathways before and after therapeutic treatments in gastric cancer patients.

### DISCUSSION

In this study, we provide evidence that gastric cancers can be segregated based on the phosphorylation profiles of key growth factor receptor signaling pathways that are active in this cancer type. Gastric cancer is an exceedingly heterogeneous disease with a wide spectrum of prognosis in terms of recurrence and response to chemotherapy. The heterogeneity is recognized at several levels including histopathologic (Lauren, 1965), anatomic (Blot et al., 1991; Crew and Neugut, 2006) and epidemiologic (Carneiro et al., 2004; Correa et al., 1990; Uemura et al., 2001; You et al., 1993) distinctions. Recent efforts are beginning to further classify gastric cancers based on their gene expression signatures (Ooi et al., 2009; Shah et al., 2011; Tay et al., 2003). Our study identifies yet another level of heterogeneity in gastric cancers based on signaling pathway activation profiles that directly affect the selection and outcome of targeted therapeutics in this cancer type.

Targeted phosphorylation analysis of clinical samples has been hampered due to the lack of readily usable clinical assays that are sensitive enough to function on limited quantities of clinical tissues and yet score and differentiate the activation patterns of multiple RTKs. In this study, we have used a highly sensitive and novel immunoassay, CEER™, directly on flash frozen gastric cancer clinical samples that can be multiplexed to specifically analyze total and phosphorylated forms of several proteins. We demonstrate that ∼53% of gastric cancer patients have at least one RTK activated among the HER1, HER2, HER3, c-MET and IGF1R molecules. Among the RTKs analyzed in this study, HER family members were the most frequently activated tyrosine kinases. Expression of HER family members has been previously correlated with tumor progression and poor prognosis in gastric cancers (Allgayer et al., 2000; Garcia et al., 2003; Hayashi et al., 2008); however, our study is the first demonstration of their activation profiles. Although several gastric cancers with activated HER kinase members did not demonstrate activation of other analyzed RTK family members, c-MET activated cancers almost always showed a co-activation of HER and/or IGF1R. Clustering analysis showed that HER2 activated gastric cancers grouped with those with activated HER3, c-MET with HER1 and IGF1R activated gastric cancers were closest to PI3K activated gastric cancers. The RTK phosphorylation profiles were segregated based on the Lauren histotype and the HER2 status of the tumors. Based on differences in gene expression profiles, recent studies (Shah et al., 2011) have classified gastric cancers as "proximal nondiffuse," "diffuse," and "distal non-diffuse" with a suggestion that these distinctions can have a significant impact on the current clinical management of gastric cancers. Besides providing fresh insights into the distinct biology of these gastric cancer subtypes based on their active kinase profiles, identification of specific RTK activation patterns concordant with the histopathology of gastric cancers can now allow hypothesis-driven therapeutic testing of the available drug candidates.

A striking observation from our analysis is that a significant number of gastric cancers (48% of HER2 positive and ∼32% of HER2 negative) do not have any single active RTK, rather they are driven by networks of concomitantly activated RTKs. This finding has strong implications for drug development as it suggests that gastric cancers might exhibit resistance to individual targeted treatments, and a combination of targeted agents may be more effective in such subsets of gastric cancers. Indeed, a phase II trial of foretinib (XL-880) monotherapy in unselected gastric cancer patients failed to demonstrate an objective response (Jhawer et al., 2008) and trastuzumab activity is only observed in a subset of HER2(+) gastric cancer patients. To our knowledge, our study is the first demonstration of multiple RTK pathway activations in gastric cancer clinical samples. Similar observations have been previously reported in glioblastoma (Stommel et al., 2007) and breast cancers (Hochgrafe et al., 2010) where combinatorial treatment as guided by their RTK activation profiles was superior to single agent treatments. We identified that cMET/HER1 phosphorylations are closely associated in HER2 negative gastric cancers and that such gastric cancer patients have significantly poorer survival. Cross talk between the c-MET and HER1 axis has been reported in gastric cancer and lung cancer cell lines (Engelman et al., 2007; Liu et al., 2011). Recent studies report that HER kinase signaling promotes resistance to cMET inhibition in gastric cancer and simultaneous blockade of the two signaling families may be more beneficial (Corso et al., 2010; Liu et al., 2011). Our results demonstrate that combinatorial therapeutic inhibition of HER1 and cMET is a more effective anti-tumor activity in HER1/MET coactivated cells as opposed to monotherapy against either targets.

Trastuzumab activity is seen in breast cancers that overexpress the HER2 protein. This finding has now been extended to gastric cancers and trastuzumab is FDA approved for the treatment of HER2(+) advanced gastric cancers (Bang et al., 2010). In fact, trastuzumab is the only targeted therapy in gastric cancers that demonstrates a survival benefit. However, it is clear from the recent ToGA trial results that trastuzumab does not benefit all HER2(+) gastric cancer patients as trastuzumab plus standard chemotherapy rendered an overall response rate of 47% in HER2(+) gastric cancers. Our current CEER™-based analysis provides at least four possible reasons for this non-response and indicates that scoring of multiple markers, including their active states may be more beneficial in predicting responses to targeted therapeutics. One possibility for trastuzumab non-response is the high degree of heterogeneity in HER2 expression in gastric cancers as demonstrated by the CEER™ assay that contributes to the complexity in developing efficient HER2 diagnostics. Although modified IHC/FISH-HER2 scoring criteria have been defined for gastric cancers which are distinct from the breast cancer guidelines, they still cannot efficiently capture the heterogeneous HER2 status in all gastric cancers and may not be sufficient to accurately predict the anti-HER2 responders. Secondly, only 50% of HER2(+) gastric cancers express phosphorylated HER2 as revealed by the CEER™ analysis. Absence of an active HER2 pathway suggests that HER2 signaling may not significantly contribute to tumor survival in all HER2(+) gastric cancers; therefore, inhibiting it with trastuzumab would not result in a measurable tumor inhibition. Thirdly, signaling pathways such as those driven by HER1, HER3, c-MET and IGF1R, which we demonstrate are active in 26%, 36%, 22% and 30% of HER2(+) gastric cancers respectively, may provide alternate tumor survival signals that promote resistance to trastuzumab. It is known that HER2:HER3 heterodimers cannot be efficiently blocked by trastuzumab in breast cancers (Agus et al., 2002; Cho et al., 2003; Ghosh et al., 2011) and that IGF1R signaling promotes trastuzumab resistance (Huang et al., 2010; Nahta et al., 2005). Moreover, recent preclinical evidence in gastric cancer xenograft models (Yamashita-Kashima et al., 2011) demonstrates that combination treatment of trastuzumab with pertuzumab is superior to trastuzumab alone in HER2(+) models, in part due to efficient inhibition of HER2:HER3 heterodimer signaling. Lastly, our studies using the proximity-based CEER™ immunoassay demonstrate the presence of p95HER2, truncated carboxy-terminal fragments of HER2, in 46% of HER2(+) gastric cancers which may also contribute to trastuzumab resistance in gastric cancers. p95HER2 in breast cancers promotes resistance to herceptin treatment due to the lack of a trastuzumab-recognizing extracellular epitope (Scaltriti et al., 2007) in addition to a worse prognosis (Saez et al., 2006) and a higher metastatic propensity (Molina et al., 2002). However, a large-scale clinical validation of p95HER2 expression is still lacking due to the technical difficulties associated with the immunohistochemical detection of p95HER2 in paraffin-embedded specimens. Although two groups have generated monoclonal antibodies against p95HER2, their clinical validation is still pending (Parra-Palau et al., 2010; Sperinde et al., 2010) as recent conflicting findings from the GeparQuattro studies failed to correlate levels of p95HER2 with trastuzumab resistance (Loibl et al., 2011). It should be noted that we have been successful in detecting p95HER2 in flash frozen gastric cancer clinical specimens using the CEER™ assay. The frequency of p95HER2 occurrence in gastric cancers (77% of HER2(+)) is greater than that reported in breast cancers (∼30% of HER2(+)). The efficacy of trastuzumab versus lapatinib in gastric cancer cells expressing either full-length HER2 or truncated p95HER2 can be tested, as several studies indicate the use of the anti-HER2 TKI, lapatinib, in p95HER2(+) cancers (Arribas et al., 2011; Saez et al., 2006). Additionally, p95HER2 expression can be validated in a phase II neoadjuvant lapatinib + chemotherapy trial in gastric cancer patients. HER2 negative gastric cancers form the larger proportion (∼88%) of the entire gastric cancer population analyzed in our study but there are no approved targeted therapies for this population. Based on the CEER™ assay, some of these cancers still express the HER2 protein but at significantly lower levels as compared to the HER2(+) population. In fact, we detected 84 samples with a significant level of phosphorylated HER2, indicating that these cancers may utilize active HER2 receptor signaling to promote tumor growth, and thus may be potential responders to various HER2 targeting agents. Trastuzumab has been found to be beneficial in a fraction of breast cancer patients with a HER2(-) status as determined by IHC (Paik et al., 2008). In addition to active HER2 signaling, we identified several subgroups of patients with activated HER1, HER3, c-MET and IGF1R in HER2(-) gastric cancers that may also contribute in driving HER2(-) tumor growth. It is noteworthy that the proportion of samples expressing pHER1, pHER2 and pHER3 found in our study resemble those reported in breast cancers (Frogne et al., 2009). HER3 protein overexpression has been correlated with poor prognosis in gastric cancers (Begnami et al., 2011; Hayashi et al., 2008) and suggested as a potential therapeutic target. In our analysis, approximately 24% of HER2(-) gastric cancers expressed active HER3 signaling with a significant proportion of these cancers co-expressing phosphorylated HER2 and HER1. This indicates that HER3 signaling may play a significant role in gastric cancers.

After years of experience with molecularly targeted agents, we now recognize that metastatic cancer is an evolving disease with emerging resistant pathways during treatment. Oncologists will encounter dilemma between the necessity of re-biopsy to plan subsequent treatments and the clinical feasibility of re-biopsy. Hence, we developed an RTK activation profiling platform using CTCs and malignant cells isolated from body fluid (*e.g*., ascites). We demonstrated that the CEER™ assay is sensitive enough to profile CTCs. With CTCs, we may longitudinally monitor the targeted-RTK status during treatment and at progression. Given the fact that not all metastatic cancer patients have CTCs available for analysis, we also isolated malignant cells from body fluid such as malignant ascites from gastric cancer patients and have successfully shown that RTK activation can be profiled in these cells. This will definitely expand the spectrum of applicability and clinical feasibility of the phospho-RTK assay in gastric cancer patients. We isolated cancer cells from malignant ascites and activated pathways using growth factors and then treated the tumor cells with TKIs *ex vivo* as a potential surrogate system to test effectiveness of agents targeting specific pathway proteins in a patient for applying evidence-based targeted therapeutics and developing strategies to overcome drug resistance.

We are now rapidly approaching an era of personalized cancer medicine and biomarker driven clinical research. Our findings demonstrating multiple RTK co-activation provide a reasonable explanation for low response rates of monotherapy in gastric cancers. In the future, we may consider an upfront combinatorial targeted treatment in subsets of gastric cancer patients with multiple signaling pathways. With a monitoring tool utilizing CTCs and/or body fluids, we may rapidly pinpoint the required follow-up treatments upon disease progression targeting alternate signaling pathways.

### EXPERIMENTAL PROCEDURES

### Patient Cohort and Tissue Specimen Procurement

All patients had undergone gastrectomy with radical lymph node dissection with curative intent. 447 fresh frozen tissues collected from surgically resected primary gastric tumor were available for final analysis. All fresh frozen tissues were collected (within <30 minutes) at the surgical field by surgeon and were immediately snap frozen in liquid nitrogen and were stored at -80°C freezer until later use.

Tumor specimens were confirmed for the presence of tumor area > 70% by pathologist. For the analysis, small pieces of frozen tissues (10 µm section X 3) were prepared using a prechilled razor blade which were then lysed in 100 µL of lysis buffer. The resulting lysates were stored at -80°C before subsequent analysis.

### Histopathology Review and HER2 status determination

All available H&E-stained slides were centrally reviewed by two pathologists. All tumor specimens were from surgically resected primary gastric tumors. HER2 status was determined by immunohistochemistry (IHC). IHC analysis was performed using the HercepTest™ (Dako, Glostrup, Denmark). HER2 protein expression levels were scored as 0 to 3+, according to the consensus panel recommendations on HER2 scoring for gastric cancer (Hofmann et al., 2008; Ruschoff et al., 2010). For FISH, PathVysion HER2 DNA probe kit (Abbott, Des Plaines, IL) was used. Ariol image analysis system was used to count the hybridization signals (Genetix, San Jose, USA). 40 invasive tumor cells were counted. All samples with ratios of HER2/CEP17 between 1.8 and 2.2 by image analysis were scored manually by counting more than 60 non-overlapping cells. A ratio of more than 2.2, 1.8 to 2.2, or less than 1.8 was classified as positive, equivocal, or negative for amplification, respectively. Chromosomal 17 polysomy was defined as a CEP17 signal more than six copies on average per cell. Of the 447 specimens, 434 specimens were included in the final analysis.

### Collaborative Enzyme Enhanced Reactive-immunoassay (CEER™)

Immuno-microarray slides were rinsed 2x with TBST (50 mM Tris/ 150 mM NaCl/ 0.1% Tween-20, pH 7.2-7.4), blocked with 80 µL Whatman Blocking Buffer 1hr at RT, then washed 2x with TBST. Serially diluted lysate controls in 80 µL dilution buffer (2% BSA/ 0.1% TritonX-100/ TBS, pH 7.2-7.4) and samples were added to designated sub-arrays on slides, then incubated 1 hour at RT. Slides were washed 4x (3 min. each), and detector Abs were added in 80 µL of reaction buffer and incubated for 2 hours at RT. After washing slides with TBST to remove unbound detector Abs, 80 µL of biotin-tyramide solution (5 µg/ml in 50 mM glucose/PBS) prepared from 400 µg/mL in ethanol solution (Perkin-Elmer Life Science) was added and incubated 15 min in darkness. Glucose-oxidase (GO)/HRP-mediated tyramide signal amplification process was terminated by washing with TBST 4x, 3 min each. Local deposition of biotin-tyramide was detected by incubation with streptavidin (SA)-Alexa647 (Invitrogen) at 0.5 µg/mL in 2% BSA/ 0.1% Triton/TBS for 40 min. Upon completion of incubation, slides were washed 4x with TBST, dried and kept in darkness until imaged via microarray scanner.

### Truncated HER2 Enrichment

Full-length p185-ERBB2 receptors were cleared from lysates using magnetic-bead coupled antibodies specific to extracellular domain (ECD) of HER2 as shown in Figure 8. Resulting p185-HER2 depleted lysates, which contained truncated HER2 (t-HER2) receptor proteins lacking the ECD, were used for subsequent quantification of t-HER2 expression and phosphorylation.

### CEER™ Data analysis

Slides were scanned at four photomultiplier (PMT) gain settings to increase the effective dynamic range. Background-corrected signal intensities were averaged for spots printed in triplicate. Several criteria were used to filter data for further analyses, including limits on spot footprints, coefficient of variation for spot replicates, and overall pad background. For each assay, a standard curve was generated from serially diluted control cell lysates prepared from cell lines with well-characterized signal transduction proteins. Data were fit to a five-parameter equation derived as a function of capture-antibody concentration and PMT. The standard curve for each marker was fit as a function of log signal intensity, measured as relative fluorescence unit (RFU) vs. log concentration of cell lysates and referenced to the standard cell lines. For example, a standard curve of serially diluted cell lysates prepared from BT474 was used to normalize HER2 expression and the degree of phosphorylation in each sample (Figure 14). Hence, a sample with 1 CU of HER2 expression has the RFU value equivalent to RFU value of 1 standard reference BT474 cell. As reference cells have 1∼2×10⁶ HER1 or HER2 receptors per cell with approximately 10% phosphorylated receptors, 1 CU represents the expression of 1∼2×10⁶ RTKs or 1∼2×10⁵ phosphorylated RTKs (Kim et al., 2011). The limit of detection (LOD) value by CU was determined to be less than 1 CU for both expression and activation of HER2. Individual predictions from each dilution and gain were averaged into a single, final prediction.

### CEER™ marker determination and heat map clustering

Among the statistical challenges in evaluating biomarker data for use in a clinical setting is determining optimum diagnostic cutoff levels. In the absence of reference range values, positivity for a given biomarker was defined as greater than the third quartile for an individual biomarker in the patient population of this study. Patients were ranked based on the CU value from the CEER™ assay for a given biomarker. Those patients greater than the third quartile cutoff were considered at highest risk to have elevated biomarker levels and therefore potentially activated signal pathways. Subsequent survival analysis was done to access the predicative quality of these cutoffs.

The biomarker profile of the tumor samples was represented by a heat map. Each cell was colorized based on the decile rank of the activation of that marker. Each marker was ranked by deciles, represented by a distinct shade, with the scale indicating the color. Both the row (patient) and column (marker) clustering was shown. The complete linkage algorithm was used to obtain a hierarchical cluster (or dendrogram) by sequentially grouping the most correlated observations using the hclust function in R, called by the heatmap.2 function available with the gplots library of the statistical environment R: A Language and Environment for Statistical Computing (http://www.r-project.org/). Subgroups of markers are defined by the clustering and allow comparisons of marker profiles for patients.

### Multiplexed-microarray printing

Capture antibodies were printed on nitrocellulose-coated glass slides (ONCYTE®, Grace Bio-Labs) using non-contact printers (Nanoplotter, GeSiM). The spot diameter was approximately 175 µm, slides were kept in a desiccated chamber at 4°C. Approximately 500 pL of capture Abs were printed in triplicate and serial dilution concentrations of 1 mg/ mL, 0.5mg/ mL, and 0.25mg/ mL. Purified mouse-IgGs served as negative controls. Immunoarray slide configurations are shown in Figure 5.

### Antibody conjugation and purification

Target specific-Abs (mouse monoclonal against different epitopes on human signal transduction proteins) and the corresponding detector enzymes glucose oxidase (GO) or horseradish peroxidase (HRP) were activated with bi-functional cross-linker, succinimidyl-4-(N-maleimidomethyl) cyclohexane-1-carboxylate (SMCC), and coupled making antibody-enzyme conjugates (Gibson et al., 1964; Klapper and Hackett, 1963). Conjugates were purified by HPLC. Ab activities in the purified conjugates were determined by competition ELISA and the post-conjugation enzyme activities were detected by functional assays specific for detector enzymes.

### Cell culture and reagents

Human gastric cancer cell lines were purchased from the Korea Cell Line Bank (KCLB, Seoul, Korea). All of the cell lines were grown in RPMI-1640 medium (PAA Laboratories GmbH, Austria) supplemented with 10% heat-inactivated fetal bovine serum, antibiotic/antimycotic. Cells were incubated at 37°C in 5% CO₂ and the medium changed twice a week. After confluence, cells were subdivided into new flasks until the end of the experiment.

CEER™ assay control cell lines, MDA-MB-468, T47D, HCC827 and BT474 cells with varying degrees of ErbB-RTK expression (Dragowska et al., 2004; Filmus et al., 1987; Imai et al., 1982) were obtained from ATCC and grown at 37°C in 5% CO₂ for MDA-MB-468 (Dulbecco's minimal essential medium, DMEM, 10% FBS), BT474 (DMEM, 10% FBS), and HCC827 and T47D (RPMI 1640, 10% FBS, 0.2 U/ml bovine insulin). Cells were counted and washed with 1× PBS before growth factor stimulation. MDA-MB-468 cells were stimulated with 100 nM epidermal growth factor (EGF) or transforming growth factor a

(TGFa), T47D cells were stimulated with 20 nM heregulin β (HRGβ) or 100 ng/mL Insulin-like Growth factor-1 (IGF1) in serum-free growth media for 5 or 15 min. Stimulated cells were washed with 1× PBS and then lysed (lysis buffer: 50 mM Tris, pH 7.4, 150 mM NaCl, 1% Trition X-100 and 2 mM Na₃VO₄) and kept on ice for 30 min before taking the supernatant for a subsequent assay or kept at -80°C.

### Drug treatment

Cells were seeded on 2x10⁵ cells/6-well plates and incubated for 24h at 37°C and treated with 1 µM/ml of lapatinib, foretinib or combination for 4h at 37°C. Cells were harvested after drug treatment and pelleted by centrifugation. For protein analysis, cell pellets were stored at -80°C prior to extraction of protein.

### Cell growth-inhibition assay

Cell growth inhibition was determined via CellTiter 96 Aqueous One Solution Assay (Promega) according to the manufacturer's protocol. Briefly, cells (3 X 10³ in 100 uL/well) were seeded on 96-well plates and incubated for 24 h at 37°C and treated with drugs (lapatinib or foretinib or combination) for 3 d at 37°C. After drug treatment, MTS solution was added to each well and incubation was continued for 4 h at 37°C. Absorbance value (OD) of each well was measured with a microplate reader set at 490 nm. All experiments were performed in triplicates.

### REFERENCES

Agus, D. B., Akita, R. W., Fox, W. D., Lewis, G. D., Higgins, B., Pisacane, P. I., Lofgren, J. A., Tindell, C., Evans, D. P., Maiese, K., et al. (2002). Targeting ligand-activated ErbB2 signaling inhibits breast and prostate tumor growth. Cancer Cell 2, 127-137.
Allgayer, H., Babic, R., Gruetzner, K. U., Tarabichi, A., Schildberg, F. W., and Heiss, M. M. (2000). c-erbB-2 is of independent prognostic relevance in gastric cancer and is associated with the expression of tumor-associated protease systems. J Clin Oncol 18, 2201-2209.
Arribas, J., Baselga, J., Pedersen, K., and Parra-Palau, J. L. (2011). p95HER2 and breast cancer. Cancer Res 71, 1515-1519.
Bae, J. M., and Park, J. G. (2002). Annual report of the Korea Central Cancer REgistry Program 2000: based on registered data from 131 hospitals. Cancer Res Treat 34, 77-83.
Bang, Y. J., Van Cutsem, E., Feyereislova, A., Chung, H. C., Shen, L., Sawaki, A., Lordick, F., Ohtsu, A., Omuro, Y., Satoh, T., et al. (2010). Trastuzumab in combination with chemotherapy versus chemotherapy alone for treatment of HER2-positive advanced gastric or gastro-oesophageal junction cancer (ToGA): a phase 3, open-label, randomised controlled trial. Lancet 376, 687-697.
Begnami, M. D., Fukuda, E., Fregnani, J. H., Nonogaki, S., Montagnini, A. L., da Costa, W. L., Jr., and Soares, F. A. (2011). Prognostic implications of altered human epidermal growth factor receptors (HERs) in gastric carcinomas: HER2 and HER3 are predictors of poor outcome. J Clin Oncol 29, 3030-3036.
Blot, W. J., Devesa, S. S., Kneller, R. W., and Fraumeni, J. F., Jr. (1991). Rising incidence of adenocarcinoma of the esophagus and gastric cardia. JAMA 265, 1287-1289.
Blume-Jensen, P., and Hunter, T. (2001). Oncogenic kinase signalling. Nature 411, 355-365.
Carneiro, F., Huntsman, D. G., Smyrk, T. C., Owen, D. A., Seruca, R., Pharoah, P., Caldas, C., and Sobrinho-Simoes, M. (2004). Model of the early development of diffuse gastric cancer in E-cadherin mutation carriers and its implications for patient screening. J Pathol 203, 681-687.
Cho, H. S., Mason, K., Ramyar, K. X., Stanley, A. M., Gabelli, S. B., Denney, D. W., Jr., and Leahy, D. J. (2003). Structure of the extracellular region of HER2 alone and in complex with the Herceptin Fab. Nature 421, 756-760.
Correa, P., Haenszel, W., Cuello, C., Zavala, D., Fontham, E., Zarama, G., Tannenbaum, S., Collazos, T., and Ruiz, B. (1990). Gastric precancerous process in a high risk population: cross-sectional studies. Cancer Res 50, 4731-4736.
Corso, S., Ghiso, E., Cepero, V., Sierra, J. R., Migliore, C., Bertotti, A., Trusolino, L., Comoglio, P. M., and Giordano, S. (2010). Activation of HER family members in gastric carcinoma cells mediates resistance to MET inhibition. Mol Cancer 9, 121*.*
Crew, K. D., and Neugut, A. I. (2006). Epidemiology of gastric cancer. World J Gastroenterol 12, 354-362.
Cunningham, D., Jost, L. M., Purkalne, G., Oliveira, J., and Force, E. G. T. (2005). ESMO Minimum Clinical Recommendations for diagnosis, treatment and follow-up of gastric cancer. Ann Oncol 16 Suppl 1, i22-23.
Dragowska, W. H., Warburton, C., Yapp, D. T., Minchinton, A. I., Hu, Y., Waterhouse, D. N., Gelmon, K., Skov, K., Woo, J., Masin, D., et al. (2004). HER-2/neu overexpression increases the viable hypoxic cell population within solid tumors without causing changes in tumor vascularization. Mol Cancer Res 2, 606-619.
Engelman, J. A., and Janne, P. A. (2008). Mechanisms of acquired resistance to epidermal growth factor receptor tyrosine kinase inhibitors in non-small cell lung cancer. Clin Cancer Res 14, 2895-2899.
Engelman, J. A., and Settleman, J. (2008). Acquired resistance to tyrosine kinase inhibitors during cancer therapy. Curr Opin Genet Dev 18, 73-79.
Engelman, J. A., Zejnullahu, K., Mitsudomi, T., Song, Y., Hyland, C., Park, J. O., Lindeman, N., Gale, C. M., Zhao, X., Christensen, J., et al. (2007). MET amplification leads to gefitinib resistance in lung cancer by activating ERBB3 signaling. Science 316, 1039-1043.
Filmus, J., Trent, J. M., Pollak, M. N., and Buick, R. N. (1987). Epidermal growth factor receptor gene-amplified MDA-468 breast cancer cell line and its nonamplified variants. Mol Cell Biol 7, 251-257.
Frogne, T., Laenkholm, A. V., Lyng, M. B., Henriksen, K. L., and Lykkesfeldt, A. E. (2009). Determination of HER2 phosphorylation at tyrosine 1221/1222 improves prediction of poor survival for breast cancer patients with hormone receptor-positive tumors. Breast Cancer Res 11, R11.
Garcia, I., Vizoso, F., Martin, A., Sanz, L., Abdel-Lah, O., Raigoso, P., and Garcia-Muniz, J. L. (2003). Clinical significance of the epidermal growth factor receptor and HER2 receptor in resectable gastric cancer. Ann Surg Oncol 10, 234-241.
Ghosh, R., Narasanna, A., Wang, S. E., Liu, S., Chakrabarty, A., Balko, J. M., Gonzalez-Angulo, A. M., Mills, G. B., Penuel, E., Winslow, J., et al. (2011). Trastuzumab has preferential activity against breast cancers driven by HER2 homodimers. Cancer Res 71, 1871-1882.
Gibson, Q. H., Swoboda, B. E., and Massey, V. (1964). Kinetics and Mechanism of Action of Glucose Oxidase. J Biol Chem 239, 3927-3934.
Grabsch, H., Sivakumar, S., Gray, S., Gabbert, H. E., and Muller, W. (2010). HER2 expression in gastric cancer: Rare, heterogeneous and of no prognostic value - conclusions from 924 cases of two independent series. Cell Oncol 32, 57-65.
Hayashi, M., Inokuchi, M., Takagi, Y., Yamada, H., Kojima, K., Kumagai, J., Kawano, T., and Sugihara, K. (2008). High expression of HER3 is associated with a decreased survival in gastric cancer. Clin Cancer Res 14, 7843-7849.
Hochgrafe, F., Zhang, L., O'Toole, S. A., Browne, B. C., Pinese, M., Porta Cubas, A., Lehrbach, G. M., Croucher, D. R., Rickwood, D., Boulghourjian, A., et al. (2010). Tyrosine phosphorylation profiling reveals the signaling network characteristics of Basal breast cancer cells. Cancer Res 70, 9391-9401.
Hoe, N., Park, J. O., Lee, J., Park, S. H., Park, Y. S., Lim, H. Y., Kuller, A., Kirkland, R., Zhou, J., Liu, X., et al. (2011). A comprehensive profiling of mutation, pathway proteins and their modulation in response to drug treatment in panel of 39 gastric cancer cell lines Proceedings of the 102nd Annual Meeting of the American Association for Cancer Research, 2011 Abstract nr 2897.
Hofmann, M., Stoss, O., Shi, D., Buttner, R., van de Vijver, M., Kim, W., Ochiai, A., Ruschoff, J., and Henkel, T. (2008). Assessment of a HER2 scoring system for gastric cancer: results from a validation study. Histopathology 52, 797-805.
Huang, X., Gao, L., Wang, S., McManaman, J. L., Thor, A. D., Yang, X., Esteva, F. J., and Liu, B. (2010). Heterotrimerization of the growth factor receptors erbB2, erbB3, and insulin-like growth factor-i receptor in breast cancer cells resistant to herceptin. Cancer Res 70, 1204-1214.
Imai, Y., Leung, C. K., Friesen, H. G., and Shiu, R. P. (1982). Epidermal growth factor receptors and effect of epidermal growth factor on growth of human breast cancer cells in long-term tissue culture. Cancer Res 42, 4394-4398.
Jhawer, M. P., Kindler, H. L., Wainberg, Z. A., Hecht, J. R., Kerr, R. O., Ford, J. M., Henderson, C., Mueller, T., Keer, H. N., and Shah, M. A. (2008). Preliminary activity of XL880, a dual MET/VEGFR2 inhibitor, in MET amplified poorly differentiated gastric cancer (PDGC): Interim results of a multicenter phase II study. J Clin Oncol 26, (May 20 suppl; ASCO abstr 4572).
Kim, P., Liu, X., Lee, T., Liu, L., Barham, R., Kirkland, R., Leesman, G., Kuller, A., Ybarrondo, B., Ng, S. C., and Singh, S. (2011). Highly sensitive proximity mediated immunoassay reveals HER2 status conversion in the circulating tumor cells of metastatic breast cancer patients. Proteome Sci 9, 75.
Klapper, M. H., and Hackett, D. P. (1963). The Oxidatic Activity of Horseradish Peroxidase. I. Oxidation of Hydro- and Naphthohydroquinones. J Biol Chem 238, 3736-3742.
Lauren, P. (1965). The Two Histological Main Types of Gastric Carcinoma: Diffuse and So-Called Intestinal-Type Carcinoma. An Attempt at a Histo-Clinical Classification. Acta Pathol Microbiol Scand 64, 31-49.
Liu, L., Shi, H., Liu, Y., Anderson, A., Peterson, J., Greger, J., Martin, A. M., and Gilmer, T. M. (2011). Synergistic effects of foretinib with HER-targeted agents in MET and HER1- or HER2-coactivated tumor cells. Mol Cancer Ther 10, 518-530.
Loibl, S., Bruey, J., Von Minckwitz, G., Huober, J. B., Press, M. F., Darb-Esfahani, S., Solbach, C., Denkert, C., Tesch, H., Holms, F., et al. (2011). Validation of p95 as a predictive marker for trastuzumab-based therapy in primary HER2-positive breast cancer: A translational investigation from the neoadjuvant GeparQuattro study. J Clin Oncol 29, (suppl; ASCO abstr 530).
Molina, M. A., Saez, R., Ramsey, E. E., Garcia-Barchino, M. J., Rojo, F., Evans, A. J., Albanell, J., Keenan, E. J., Lluch, A., Garcia-Conde, J., et al. (2002). NH(2)-terminal truncated HER-2 protein but not full-length receptor is associated with nodal metastasis in human breast cancer. Clin Cancer Res 8, 347-353.
Nahta, R., Yuan, L. X., Zhang, B., Kobayashi, R., and Esteva, F. J. (2005). Insulin-like growth factor-I receptor/human epidermal growth factor receptor 2 heterodimerization contributes to trastuzumab resistance of breast cancer cells. Cancer Res 65, 11118-11128.
Ooi, C. H., Ivanova, T., Wu, J., Lee, M., Tan, I. B., Tao, J., Ward, L., Koo, J. H., Gopalakrishnan, V., Zhu, Y., et al. (2009). Oncogenic pathway combinations predict clinical prognosis in gastric cancer. PLoS Genet 5, e1000676.
Paik, S., Kim, C., and Wolmark, N. (2008). HER2 status and benefit from adjuvant trastuzumab in breast cancer. N Engl J Med 358, 1409-1411.
Parra-Palau, J. L., Pedersen, K., Peg, V., Scaltriti, M., Angelini, P. D., Escorihuela, M., Mancilla, S., Sanchez Pla, A., Ramon, Y. C. S., Baselga, J., and Arribas, J. (2010). A major role of p95/611-CTF, a carboxy-terminal fragment of HER2, in the down-modulation of the estrogen receptor in HER2-positive breast cancers. Cancer Res 70, 8537-8546.
Ruschoff, J., Dietel, M., Baretton, G., Arbogast, S., Walch, A., Monges, G., Chenard, M. P., Penault-Llorca, F., Nagelmeier, I., Schlake, W., et al. (2010). HER2 diagnostics in gastric cancer-guideline validation and development of standardized immunohistochemical testing. Virchows Arch 457, 299-307.
Saez, R., Molina, M. A., Ramsey, E. E., Rojo, F., Keenan, E. J., Albanell, J., Lluch, A., Garcia-Conde, J., Baselga, J., and Clinton, G. M. (2006). p95HER-2 predicts worse outcome in patients with HER-2-positive breast cancer. Clin Cancer Res 12, 424-431.
Scaltriti, M., Rojo, F., Ocana, A., Anido, J., Guzman, M., Cortes, J., Di Cosimo, S., Matias-Guiu, X., Ramon y Cajal, S., Arribas, J., and Baselga, J. (2007). Expression of p95HER2, a truncated form of the HER2 receptor, and response to anti-HER2 therapies in breast cancer. J Natl Cancer Inst 99, 628-638.
Shah, M. A., Khanin, R., Tang, L., Janjigian, Y. Y., Klimstra, D. S., Gerdes, H., and Kelsen, D. P. (2011). Molecular classification of gastric cancer: a new paradigm. Clin Cancer Res 17, 2693-2701.
Sperinde, J., Jin, X., Banerjee, J., Penuel, E., Saha, A., Diedrich, G., Huang, W., Leitzel, K., Weidler, J., Ali, S. M., et al. (2010). Quantitation of p95HER2 in paraffin sections by using a p95-specific antibody and correlation with outcome in a cohort of trastuzumab-treated breast cancer patients. Clin Cancer Res 16, 4226-4235.
Stommel, J. M., Kimmelman, A. C., Ying, H., Nabioullin, R., Ponugoti, A. H., Wiedemeyer, R., Stegh, A. H., Bradner, J. E., Ligon, K. L., Brennan, C., et al. (2007). Coactivation of receptor tyrosine kinases affects the response of tumor cells to targeted therapies. Science 318, 287-290.
Tanner, M., Hollmen, M., Junttila, T. T., Kapanen, A. I., Tommola, S., Soini, Y., Helin, H., Salo, J., Joensuu, H., Sihvo, E., et al. (2005). Amplification of HER-2 in gastric carcinoma: association with Topoisomerase IIalpha gene amplification, intestinal type, poor prognosis and sensitivity to trastuzumab. Ann Oncol 16, 273-278.
Tay, S. T., Leong, S. H., Yu, K., Aggarwal, A., Tan, S. Y., Lee, C. H., Wong, K., Visvanathan, J., Lim, D., Wong, W. K., et al. (2003). A combined comparative genomic hybridization and expression microarray analysis of gastric cancer reveals novel molecular subtypes. Cancer Res 63, 3309-3316.
Uemura, N., Okamoto, S., Yamamoto, S., Matsumura, N., Yamaguchi, S., Yamakido, M., Taniyama, K., Sasaki, N., and Schlemper, R. J. (2001). Helicobacter pylori infection and the development of gastric cancer. N Engl J Med 345, 784-789.
Xu, H., Stabile, L. P., Gubish, C. T., Gooding, W. E., Grandis, J. R., and Siegfried, J. M. (2011). Dual blockade of EGFR and c-Met abrogates redundant signaling and proliferation in head and neck carcinoma cells. Clin Cancer Res 17, 4425-4438.
Yamashita-Kashima, Y., Iijima, S., Yorozu, K., Furugaki, K., Kurasawa, M., Ohta, M., and Fujimoto-Ouchi, K. (2011). Pertuzumab in combination with trastuzumab shows significantly enhanced antitumor activity in HER2-positive human gastric cancer xenograft models. Clin Cancer Res 17, 5060-5070.
You, W. C., Blot, W. J., Li, J. Y., Chang, Y. S., Jin, M. L., Kneller, R., Zhang, L., Han, Z. X., Zeng, X. R., Liu, W. D., and et al. (1993). Precancerous gastric lesions in a population at high risk of stomach cancer. Cancer Res 53, 1317-1321.

### Example 4. Personalized Mouse Model for Gastric Cancer Therapy.

This example describes a mouse model (an "avatar") to aid physicians in identifying the most effective anticancer drug or combination (*e.g*., cocktail) of anticancer drugs that can be administered to a patient to combat a particular tumor such as a gastric tumor.

An "avatar" includes a mouse or other animal onto which tissue from a human tumor is grafted to create a personalized model of one patient's cancer. In one example of this approach, a patient's gastric tumor (*e.g*., stage I or II gastric cancer such as diffuse-type gastric cancer) is analyzed for the presence or level of expression and/or activation of one or more analytes such as cMET and HER1, and optionally HER2 and/or HER3 as well as other signal transduction pathway components. A personalized mouse model is then created of that specific gastric cancer by xenografting a piece of the patient's tumor onto immunodeficient mice. The xenograft tumor's response to an anticancer drug or a combination (*e.g*., cocktail) of anticancer drugs can then be tested. For example, if both cMET and HER1 are activated in the patient's gastric tumor (*e.g*., as determined by the CEER™ technology), a combination of a cMET inhibitor and a HER1 inhibitor can be tested on the xenograft tumor present on the avatar to determine whether this is an effective treatment for that patient's cancer. If the xenograft tumor responds to the inhibitor cocktail (*e.g.,* the tumor shrinks), that particular combination therapy can be administered to the patient.

## Claims

1. An in vitro method for predicting the survival of a subject having stage I gastric cancer after tumor surgery, the method comprising:
(a) determining the presence of activation or the level of activation of at least two analytes comprising cMET and HER1 in a cancer cell obtained from the subject, wherein cMET and HER1 are coactivated in the cancer cell; and
(b) predicting the survival of the subject based upon the presence of activation or the level of activation of the at least two analytes determined in step (a), wherein the subject is predicted to have worse survival compared to stage I gastric cancer subjects with cMET activation in the absence of HER1 activation.

2. The method of claim 1, wherein the method predicts the disease-free survival or progression-free survival of the subject.

3. The method of claims 1 or 2, wherein the gastric cancer is a diffuse-type gastric cancer.

4. The method of any one of claims 1 to 3, wherein step (a) further comprises determining the presence or level of activation of at least one additional analyte comprising HER2 and/or HER3 in the cancer cell.

5. The method of claim 4, wherein HER2 is not activated in the cancer cell, wherein optionally the subject is predicted to have worse survival compared to stage I gastric cancer subjects with cMET activation in the absence of HER1 and/or HER2 activation.

6. The method of claim 4, wherein cMET, HER1, and HER3 are coactivated but HER2 is not activated in the cancer cell, wherein optionally the subject is predicted to have worse survival compared to stage I gastric cancer subjects with cMET activation in the absence of HER1, HER2, and/or HER3 activation.

7. The method of any one of claims 1 to 6, wherein step (a) comprises determining the presence or level of activation of the analytes with a Collaborative Enzyme Enhanced Reactive Immunoassay (CEER™).

8. A composition comprising a cMET inhibitor and a HER1 inhibitor for use in the treatment of stage I gastric cancer in a subject, wherein cMET and HER1 are coactivated in the stage I gastric cancer, and wherein the subject has worse survival and/or poorer prognosis compared to stage I gastric cancer subjects with cMet activation in the absence of HER1 activation.

9. The composition of claim 8 for use in the treatment of stage I gastric cancer in a subject, wherein the composition is to be administered to the subject as primary therapy.

10. The composition of claims 8 or 9 for use in the treatment of stage I gastric cancer in a subject, wherein the composition is to be administered as adjuvant composition after tumor surgery.

11. The composition of any one of claims 8-10 for use in the treatment of stage I gastric cancer in a subject, wherein HER2 is not activated in the stage I gastric cancer, wherein optionally the subject has worse survival and/or poorer prognosis compared to stage I gastric cancer subjects with cMet activation in the absence of HER1 and/or HER2 activation.

12. The composition of any one of claims 8-11 for use in the treatment of stage I gastric cancer in a subject, wherein HER3 is activated in the stage I gastric cancer, wherein optionally the subject has worse survival and/or poorer prognosis compared to stage I gastric cancer subjects with cMET activation in the absence of HER1, HER2, and/or HER3 activation.

13. The composition of any one of claims 8-12 for use in the treatment of stage I gastric cancer in a subject, wherein the cMET inhibitor is selected from the group consisting of AMG102, ARQ197, JNJ-38877605, PF- 2341066, PF-04217903, SGX523, GSK1363089/XL880, XL184, MGCD265, MK-2461, PHA-665752, and combinations thereof, or wherein the HER1 inhibitor is selected from the group consisting of erlotinib, lapatinib, gefitinib, BIBW-2992, and combinations thereof.

14. The composition of anyone of claims 8-13 for use in the treatment of stage I gastric cancer in a subject, wherein the presence of activation or the level of activation of cMET and HER1 is determined in a cancer cell obtained from the subject, wherein the cancer cell is selected from the group consisting of a primary tumor cell, a circulating tumor cell (CTC), an ascites tumor cell (ATC), and combinations thereof.

15. The composition of claim 14 for use in the treatment of stage I gastric cancer in a subject, wherein the presence of activation or the level of activation of cMET and HER1 is determined with a Collaborative Enzyme Enhanced Reactive Immunoassay (CEER™).

## Patentansprüche

1. In-vitro-Verfahren zum Vorhersagen des Überlebens eines Subjekts mit Magenkrebs Grad 1 nach Tumorchirurgie, das Verfahren umfassend:
(a) Bestimmen der Anwesenheit einer Aktivierung oder des Aktivierungsgrads von mindestens zwei Analyten umfassend cMET und HER1 in einer Krebszelle, die vom Subjekt erlangt wurde, wobei cMET und HER1 in der Krebszelle zusammen aktiviert sind; und
(b) Vorhersagen des Überlebens des Subjekts beruhend auf der Anwesenheit einer Aktivierung oder des Aktivierungsgrads von den mindestens zwei in Schritt (a) bestimmten Analyten, wobei dem Subjekt vorhergesagt wird, dass es ein schlechteres Überleben im Vergleich zu Subjekten mit Magenkrebs Grad 1 mit einer cMET-Aktivierung in der Abwesenheit einer HER1-Aktivierung hat.

2. Verfahren nach Anspruch 1, wobei das Verfahren das krankheitsfreie oder fortschrittsfreie Überleben des Subjekts vorhersagt.

3. Verfahren nach Anspruch 1 oder 2, wobei der Magenkrebs ein Magenkrebs vom diffusen Typ ist.

4. Verfahren nach einem der Ansprüche 1 bis 3, wobei Schritt (a) ferner ein Bestimmen der Anwesenheit oder des Aktivierungsgrads von mindestens einem zusätzlichen Analyt umfassend HER2 und/oder HER3 in der Krebszelle umfasst.

5. Verfahren nach Anspruch 4, wobei HER2 in der Krebszelle nicht aktiviert ist, wobei optional dem Subjekt vorhergesagt wird, dass es ein schlechteres Überleben im Vergleich zu Subjekten Magenkrebs Grad 1 mit einer cMET-Aktivierung in der Abwesenheit einer HER1- und/oder HER2-Aktivierung hat.

6. Verfahren nach Anspruch 4, wobei cMET, HER1, und HER3 zusammen aktiviert sind, aber HER2 nicht in der Krebszelle aktiviert ist, wobei optional dem Subjekt vorhergesagt wird, dass es ein schlechteres Überleben im Vergleich zu Subjekten Magenkrebs Grad 1 mit einer cMET-Aktivierung in der Abwesenheit einer HER1-, HER2- und/oder HER3-Aktivierung hat.

7. Verfahren nach einem der Ansprüche 1 bis 6, wobei Schritt (a) ein Bestimmen der Anwesenheit oder des Aktivierungsgrads der Analyte mit einem kollaborativen, enzymverstärkten, reaktiven Immuntest (Collaborative Enzyme Enhanced Reactive Immunoassay, CEER™) umfasst.

8. Zusammensetzung, umfassend einen cMET-Hemmer und einen HER1-Hemmer zur Verwendung in der Behandlung von Magenkrebs Grad 1 in einem Subjekt, wobei cMET und HER1 im Magenkrebs Grad 1 zusammen aktiviert sind, und wobei das Subjekt ein schlechteres Überleben und/oder eine schlechtere Prognose im Vergleich zu Subjekten mit Magenkrebs Grad 1 mit einer cMet-Aktivierung in der Abwesenheit einer HER1-Aktivierung hat.

9. Zusammensetzung nach Anspruch 8 zur Verwendung in der Behandlung von Magenkrebs Grad 1 in einem Subjekt, wobei die Zusammensetzung dem Subjekt als primäre Therapie verabreicht werden soll.

10. Zusammensetzung nach Anspruchs 8 oder 9 zur Verwendung in der Behandlung von Magenkrebs Grad 1 in einem Subjekt, wobei die Zusammensetzung als Adjuvans-Zusammensetzung nach Tumorchirurgie verabreicht werden soll.

11. Zusammensetzung nach einem der Ansprüche 8-10 zur Verwendung in der Behandlung von Magenkrebs Grad 1 in einem Subjekt, wobei HER2 im Magenkrebs Grad 1 nicht aktiviert ist, wobei optional das Subjekt ein schlechteres Überleben und/oder eine schlechtere Prognose im Vergleich zu Subjekten mit Magenkrebs Grad 1 mit einer cMet-Aktivierung in der Abwesenheit einer HER1- und/oder HER2-Aktivierung hat.

12. Zusammensetzung nach einem der Ansprüche 8-11 zur Verwendung in der Behandlung von Magenkrebs Grad 1 in einem Subjekt, wobei HER3 im Magenkrebs Grad 1 aktiviert ist, wobei optional das Subjekt ein schlechteres Überleben und/oder eine schlechtere Prognose im Vergleich zu Subjekten mit Magenkrebs Grad 1 mit einer cMet-Aktivierung in der Abwesenheit einer HER1-, HER2- und/oder HER3-Aktivierung hat.

13. Zusammensetzung nach einem der Ansprüche 8-12 zur Verwendung in der Behandlung von Magenkrebs Grad 1 in einem Subjekt, wobei der cMET-Hemmer ausgewählt ist aus der Gruppe bestehend aus AMG102, ARQ197, JNJ-38877605, PF-2341066, PF-04217903, SGX523, GSK1363089/XL880, XL184, MGCD265, MK-2461, PHA-665752, und Kombinationen davon, oder wobei der HER1-Hemmer ausgewählt ist aus der Gruppe bestehend aus Erlotinib, Lapatinib, Gefitinib, BIBW-2992 und Kombinationen davon.

14. Zusammensetzung nach einem der Ansprüche 8-13 zur Verwendung in der Behandlung von Magenkrebs Grad 1 in einem Subjekt, wobei die Anwesenheit einer Aktivierung oder des Aktivierungsgrads von cMET und HER1 in einer Krebszelle bestimmt wird, die von dem Subjekt erlangt wird, wobei die Krebszelle ausgewählt ist aus der Gruppe bestehend aus einer primären Tumorzelle, einer zirkulierenden Tumorzelle (circulating tumor cell, CTC), einer Aszites-Tumorzelle (ascites tumor cell, ATC) und Kombinationen davon.

15. Zusammensetzung nach Anspruch 14 zur Verwendung in der Behandlung von Magenkrebs Grad 1 in einem Subjekt, wobei die Anwesenheit einer Aktivierung oder der Aktivierungsgrad von cMET und HER1 mit einem kollaborativen, enzymverstärkten, reaktiven Immuntest (Collaborative Enzyme Enhanced Reactive Immunoassay, CEER™) bestimmt wird.

## Revendications

1. Méthode in-vitro de prédiction de la survie d'un sujet atteint de cancer gastrique de stade I après une chirurgie tumorale, la méthode comprenant :
(a) la détermination de la présence d'activation ou du niveau d'activation d'au moins deux analytes, dont cMET et HER1, dans une cellule cancéreuse provenant du sujet, cMET et HER1 étant coactivées dans la cellule cancéreuse ; et
(b) la prédiction de la survie du sujet en fonction de la présence d'activation ou du niveau d'activation des au moins deux analytes déterminés dans l'étape (a), dans laquelle on prédit que le sujet présente une chance de survie plus faible par rapport aux sujets atteint de cancer gastrique de stade I avec activation de cMET en l'absence d'activation d'HER1.

2. Méthode selon la revendication 1, la méthode prédisant la survie du sujet sans maladie ou sans progression de la maladie.

3. Méthode selon les revendications 1 et 2, dans laquelle le cancer gastrique est un cancer gastrique de type diffus.

4. Méthode selon l'une quelconque des revendications 1 à 3, dans laquelle l'étape (a) comprend en outre la détermination de la présence ou du niveau d'activation d'au moins un analyte supplémentaire, dont HER2 et/ou HER3, dans la cellule cancéreuse.

5. Méthode selon la revendication 4, dans laquelle HER2 n'est pas activée dans la cellule cancéreuse, la prédiction étant éventuellement que le sujet présente une chance de survie plus faible que les sujets atteints de cancer gastrique de stade I avec activation de cMET en l'absence d'activation d'HER1 et/ou HER2.

6. Méthode selon la revendication 4, dans laquelle cMET, HER1 et HER3 sont coactivées mais HER2 n'est pas activée dans la cellule cancéreuse, la prédiction étant éventuellement que le sujet présente une chance de survie plus faible que les sujets atteints de cancer gastrique de stade I avec activation de cMET en l'absence d'activation d'HER1, HER2 et/ou HER3.

7. Méthode selon l'une quelconque des revendications 1 à 6, dans laquelle l'étape (a) comprend la détermination de la présence ou du niveau d'activation des analytes par immunoessai CEER™ (Collaborative Enzyme Enhanced Reactive immunoessay).

8. Composition comprenant un inhibiteur de cMET et un inhibiteur d'HER1 en vue d'une utilisation dans le traitement du cancer gastrique de stade I chez un sujet, dans laquelle cMET et HER1 sont coactivées dans le cancer gastrique de stade I, et dans laquelle le sujet présente une chance de survie plus faible et/ou un pronostic plus mauvais par rapport aux sujets atteints de cancer gastrique de stade I avec activation de cMet en l'absence d'activation d'HER1.

9. Composition selon la revendication 8 en vue d'une utilisation dans le traitement du cancer gastrique de stade I chez un sujet, la composition devant être administrée au sujet en thérapie primaire.

10. Composition selon la revendication 8 ou 9 en vue d'une utilisation dans le traitement du cancer gastrique de stade I chez un sujet, la composition devant être administrée sous forme de composition adjuvante après une chirurgie tumorale.

11. Composition selon l'une quelconque des revendications 8 à 10 en vue d'une utilisation dans le traitement du cancer gastrique de stade I chez un sujet, dans laquelle HER2 n'est pas activée dans le cancer gastrique de stade I, le sujet présentant éventuellement une chance de survie plus faible et/ou un pronostic plus mauvais par rapport aux sujets atteints de cancer gastrique de stade I avec activation de cMet en l'absence d'activation d'HER1 et/ou HER2.

12. Composition selon l'une quelconque des revendications 8 à 11 en vue d'une utilisation dans le traitement du cancer gastrique de stade I chez un sujet, dans laquelle HER3 est activée dans le cancer gastrique de stade I, le sujet ayant éventuellement une chance de survie plus faible et/ou un pronostic plus mauvais par rapport aux sujets atteints de cancer gastrique de stade I avec activation de cMET en l'absence d'activation d'HER1, HER2 et/ou HER3.

13. Composition selon l'une quelconque des revendications 8 à 12 en vue d'une utilisation dans le traitement du cancer gastrique de stade I chez un sujet, dans laquelle l'inhibiteur de cMET est choisi dans le groupe constitué par AMG102, ARQ197, JNJ-38877605, PF-2341066, PF-04217903, SGX523, GSK1363089/XL880, XL184, MGCD265, MK-2461, PHA-665752 et des combinaisons de ceux-ci, ou dans laquelle l'inhibiteur d'HER1 est choisi dans le groupe constitué par erlotinib, lapatinib, grefitinib, BIBW-2992 et des combinaisons de ceux-ci.

14. Composition selon l'une quelconque des revendications 8 à 13 en vue d'une utilisation dans le traitement du cancer gastrique de stade I chez un sujet, dans laquelle la présence d'activation ou le niveau d'activation de cMET et HER1 est déterminé dans une cellule cancéreuse provenant du sujet, la cellule cancéreuse étant choisie dans le groupe constitué par une cellule tumorale primaire, une cellule tumorale circulante (CTC), une cellule tumorale d'ascite (ATC) et des combinaisons de celles-ci.

15. Composition selon la revendication 14 en vue d'une utilisation dans le traitement du cancer gastrique de stade I chez un sujet, dans laquelle la présence d'activation ou le niveau d'activation de cMET et de HER1 est déterminé par immunoessai CEER™ (Collaborative Enzyme Enhanced Reactive immunoessay).
